# EUROPÄISCHE PATENTSCHRIFT

(11) **EP 2 260 042 B1**
(45) Veröffentlichungstag und Bekanntmachung des Hinweises auf die Patenterteilung: **21.09.2011**
(21) Anmeldenummer: 09724314.1
(22) Anmeldetag: 25.03.2009
(51) Int. Cl.: C07D 491/147, C07D 471/04, C07D 491/052, A61K 31/407, A61P 25/04, A61P 25/30, A61P 25/28

(54) **SUBSTITUIERTE SPIROCYCLISCHE CYCLOHEXAN-DERIVATE**
SUBSTITUTED SPIROCYCLIC CYCLOHEXANE DERIVATIVES
DÉRIVÉS DE CYCLOHEXANE SPIROCYCLIQUES SUBSTITUÉS

(30) Priorität: 27.03.2008 EP 08005808
(43) Veröffentlichungstag der Anmeldung: 15.12.2010
(73) Patentinhaber: Grünenthal GmbH, 52078 Aachen (DE)
(72) Erfinder: ZEMOLKA, Saskia, 52066 Aachen (DE); NOLTE, Bert, 53902 Bad Münstereifel (DE); FRORMANN, Sven, 52066 Aachen (DE); HINZE, Claudia, 53175 Bonn (DE); LINZ, Klaus, 53343 Wachtberg (DE); SCHRÖDER, Wolfgang, 52074 Aachen (DE); ENGLBERGER, Werner, 52223 Stolberg (DE); SCHICK, Hans, 13086 Berlin (DE); SONNENSCHEIN, Helmut, 10245 Berlin (DE)
(74) Vertreter: Bülle, Jan
(86) Internationale Anmeldenummer: PCT/EP2009/002186
(87) Internationale Veröffentlichungsnummer: WO 2009/118173

(56) Entgegenhaltungen:
- WO-A-2005/066183
- WO-A-2008/009416

## Beschreibung

Die vorliegende Erfindung betrifft substituierte spirocyclische Cyclohexan-Derivate, welche eine Affinität an den µ-Opioid-Rezeptor und den ORL1-Rezeptor aufweisen, Verfahren zu deren Herstellung, Arzneimittel enthaltend diese Verbindungen und die Verwendung dieser Verbindungen zur Herstellung von Arzneimitteln.

Spirocyclische Cyclohexan-Derivate, welche eine Affinität an den µ-Opioid-Rezeptor und den ORL1-Rezeptor aufweisen, sind im Stand der Technik bekannt. In diesem Zusammenhang kann beispielsweise vollumfänglich verwiesen werden auf WO2004/043967, WO2005/ 063769, WO2005/066183, WO2006/018184, WO2006/108565, WO2007/12490 und WO2008/009416.

Die bekannten Verbindungen sind jedoch nicht in jeglicher Hinsicht zufrieden stellend und es besteht ein Bedarf an weiteren Verbindungen mit vergleichbaren oder besseren Eigenschaften.

So zeigen die bekannten Verbindungen in geeigneten Bindungsassays mitunter eine gewisse Affinität zum hERG-Ionenkanal, zum L-Typ Calcium-Ionenkanal (Phenylalkylamin-, Benzothiazepin-, Dihydropyridin-Bindungsstellen) bzw. zum Natrium-Kanal im BTX-Assay (Batrachotoxin), was jeweils als Anzeichen für kardiovaskuläre Nebenwirkungen gedeutet werden kann. Ferner zeigen zahlreiche der bekannten Verbindungen eine nur geringe Löslichkeit in wässrigen Medien, was sich u.a. negativ auf die Bioverfügbarkeit auswirken kann. Darüber hinaus ist die chemische Stabilität der bekannten Verbindungen oft nur unzureichend. So zeigen die Verbindungen mitunter keine ausreichende pH-, UV- bzw. Oxidationsstabilität, was sich u.a. negativ auf die Lagerstabilität und auch auf die orale Bioverfügbarkeit auswirken kann. Ferner weisen die bekannten Verbindungen teilweise ein ungünstiges PK/PD (Pharmakokinetik/Pharmakodynamik)-Profil auf, was sich z.B. in einer zu langen Wirkdauer zeigen kann.

Auch die metabolische Stabilität der bekannten Verbindungen scheint verbesserungsbedürftig. Eine verbesserte metabolische Stabilität kann auf eine erhöhte Bioverfügbarkeit hinweisen. Auch eine schwache oder nicht vorhandene Wechselwirkung mit Transportermolekülen, die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten. Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind.

Der Erfindung liegt die Aufgabe zugrunde, Verbindungen zur Verfügung zu stellen, welche sich zu pharmazeutischen Zwecken eignen und Vorteile gegenüber den Verbindungen des Standes der Technik aufweisen.

Diese Aufgabe wird durch den Gegenstand der Patentansprüche gelöst.

Es wurde überraschend gefunden, dass substituierte spirocyclische Cyclohexan-Derivate hergestellt werden können, welche eine Affinität an den µ-Opioid-Rezeptor und den ORL1-Rezeptor aufweisen.

Die Erfindung betrifft Verbindungen der allgemeinen Formel (1), 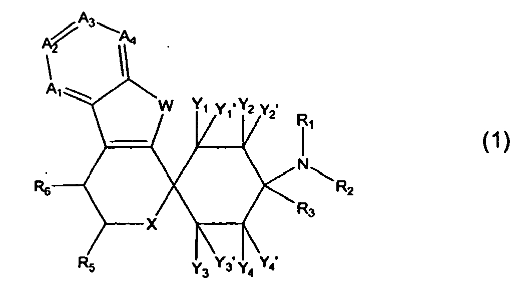 worin
A₁ für -N= oder -CR₇= steht,
A₂ für -N= oder -CR₈= steht,
A₃ für -N= oder -CR₉= steht,
A₄ für -N= oder -CR₁₀= steht;
mit der Maßgabe, dass höchstens zwei der Reste A₁, A₂, A₃ und A₄, vorzugsweise 0, 1 oder 2 der Reste A₁, A₂, A₃ und A₄, für -N= stehen;
W für -NR₄-, -O- oder -S- steht, vorzugsweise für -NR₄- oder -O-;
X für -NR₁₇-, -O-, -S(=O)₀₋₂- oder -CR₁₈R₁₉- steht, vorzugsweise für -NR₁₇- oder -O-;
Y₁, Y₁', Y₂, Y₂', Y₃, Y₃', Y₄ und Y₄' jeweils unabhängig voneinander ausgewählt sind aus der Gruppe bestehend aus -H, -F, -Cl, -Br, -I, -CN, -NO₂, -CHO, -R₀, -C(=O)R₀, -C(=O)-H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)-NHR₀, -NHC(=O)-N(R₀)₂; vorzugsweise jeweils unabhängig voneinander ausgewählt sind aus der Gruppe bestehend aus -H, -F, -Cl, -CN, -C₁₋₈-Aliphat, -C₁₋₈-Aliphat-NHC₁₋₈-Aliphat, -C₁₋₈-Aliphat-N(C₁₋₈-Aliphat)₂, -S-C₁₋₈-Aliphat, -S-Aryl, -Aryl, -C₁₋₈-Aliphat-Aryl; oder Y₁ und Y₁', oder Y₂ und Y₂', oder Y₃ und Y₃', oder Y₄ und Y₄ gemeinsam für =O stehen;
mit der Maßgabe, dass wenigstens einer der Reste Y₁, Y₁', Y₂, Y₂', Y₃, Y₃', Y₄ und Y₄', vorzugsweise einer oder zwei der Reste Y₁, Y₁', Y₂, Y₂', Y₃, Y₃', Y₄ und Y₄', nicht für -H steht;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
R₁ und R₂, unabhängig voneinander für -H oder -R₀ stehen; oder R₁ und R₂ zusammen für -CH₂CH₂OCH₂CH₂-, -CH₂CH₂NR₁₁CH₂CH₂- oder -(CH₂)₃₋₆- stehen;
R₃ für -R₀ steht;
R₄ für -H, -R₀, -COR₁₂ oder -S(=O)₂R₁₂ steht;
R₅, R₆, R₇, R₈, R₉, R₁₀, R₁₈ und R₁₉ jeweils unabhängig voneinander für -H, -F, -Cl, -Br, -I, -NO₂, -CF₃, -OR₁₃, -SR₁₃, -SO₂R₁₃, -S(=O)₂OR₁₃, -CN, -COOR₁₃, -CONR₁₃, -NR₁₄R₁₅, =O oder -R₀ stehen; oder R₅ und R₆ gemeinsam für -(CH₂)₂₋₆- stehen, wobei einzelne Wasserstoffatome auch durch -F, -Cl, -Br, -I, -NO₂, -CF₃, -OR₁₃, -CN oder -C₁₋₆-Aliphat ersetzt sein können;
R₁₁ jeweils unabhängig für -H, -R₀ oder -C(=O)R₀ steht;
R₁₂ jeweils unabhängig für -H, -R₀, -OR₁₃, oder -NR₁₄R₁₅ steht;
R₁₃ jeweils unabhängig für -H oder R₀ steht;
R₁₄ und R₁₅ unabhängig voneinander für -H oder R₀ stehen; oder R₁₄ und R₁₅ zusammen für -CH₂CH₂OCH₂CH₂-, -CH₂CH₂NR₁₆CH₂CH₂- oder -(CH₂)₃₋₆- stehen;
R₁₆ für -H oder -C₁₋₆-Aliphat steht;
R₁₇ für -H, -R₀, -COR₁₂ oder -S(=O)₂R₁₂ steht;
wobei
"Aliphat" jeweils ein verzweigter oder unverzweigter, gesättigter oder ein ein- oder mehrfach ungesättigter, unsubstituierter oder ein- oder mehrfach substituierter, aliphatischer Kohlenwasserstoffrest ist;
"Cycloaliphat" jeweils ein gesättigter oder ein ein- oder mehrfach ungesättigter, unsubstituierter oder ein- oder mehrfach substituierter, alicyclischer, mono- oder multicyclischer Kohlenwasserstoffrest ist, dessen Zahl der Ringkohlenstoffatome vorzugsweise im angegebenen Bereich liegt (d.h. "C₃₋₈-"Cycloaliphat weist vorzugsweise 3, 4, 5, 6, 7 oder 8 Ringkohlenstoffatome auf);
wobei in Bezug auf "Aliphat" und "Cycloaliphat" unter "ein- oder mehrfach substituiert" die ein- oder mehrfache Substitution eines oder mehrerer Wasserstoffatome, z.B. die einfache, zweifache, dreifache oder vollständige Substitution, durch Substituenten unabhängig voneinander ausgewählt aus der Gruppe bestehend aus -F, -Cl, -Br, -I, -CN, -NO₂, -CHO, =O -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NH-C(=O)N(R₀)₂, -Si(R₀)₃, -PO(OR₀)₂ verstanden wird;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)_{1- 2}NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃, -PO(OR₀)₂ verstanden wird;
wobei ggf. vorhandene N-Ringatome jeweils oxidiert sein können (N-Oxid);
in Form eines einzelnen Stereoisomers oder deren Mischung, der freien Verbindungen und/oder ihrer physiologisch verträglichen Salze und/oder Solvate.

Bei der Zusammenfassung verschiedener Reste, beispielsweise R₇, R₈, R₉ und R₁₀ sowie der Zusammenfassung von Resten an deren Substituenten, wie z. B. -OR₁₃, -SR₁₃, -SO₂R₁₃ oder -COOR₁₃, kann ein Substituent, z.B. R₁₃, für zwei oder mehrere Reste, beispielsweise R₇, R₈, R₉ und R₁₀, innerhalb einer Substanz unterschiedliche Bedeutungen annehmen.

Die erfindungsgemäßen Verbindungen zeigen gute Bindung an den ORL1-Rezeptor und den µ-Opioid-Rezeptor.

In einer bevorzugten Ausführungsform weisen die erfindungsgemäßen Verbindungen ein Verhältnis ORL1/µ-Affinität von wenigstens 0,1 auf. Das ORL1/µ-Verhältnis ist definiert als 1/[K_{i(ORL1)}/K_{i(µ)}]. Besonders bevorzugt beträgt das ORL1/µ-Verhältnis wenigstens 0,2 oder wenigstens 0,5, bevorzugter wenigstens 1,0 oder wenigstens 2,0, noch bevorzugter wenigstens 3,0 oder wenigstens 4,0, am bevorzugtesten wenigstens 5,0 oder wenigstens 7,5 und insbesondere wenigstens 10 oder wenigstens 15. In einer bevorzugten Ausführungsform liegt das ORL1/µ-Verhältnis im Bereich von 0,1 bis 30, bevorzugter 0,1 bis 25.

In einer anderen bevorzugten Ausführungsform weisen die erfindungsgemäßen Verbindungen ein Verhältnis ORL1/µ-Affinität von mehr als 30, bevorzugter wenigstens 50, noch bevorzugter wenigstens 100, am bevorzugtesten wenigstens 200 und insbesondere wenigstens 300 auf.

Die erfindungsgemäßen Verbindungen weisen bevorzugt einen Kᵢ-Wert am µ-Opioid-Rezeptor von höchstens 500 nM, bevorzugter höchstens 100 nM, noch bevorzugter 50 nM, am bevorzugtesten höchstens 10 nM und insbesondere höchstens 1,0 nM auf.

Methoden zur Bestimmung des Kᵢ-Werts am µ-Opioid-Rezeptor sind dem Fachmann bekannt. Bevorzugt erfolgt die Bestimmung wie im Zusammenhang mit den Beispielen beschrieben.

Die erfindungsgemäßen Verbindungen weisen bevorzugt einen Kᵢ-Wert am ORL1-Rezeptor von höchstens 500 nM, bevorzugter höchstens 100 nM, noch bevorzugter 50 nM, am bevorzugtesten höchstens 10 nM und insbesondere höchstens 1,0 nM auf.

Methoden zur Bestimmung des Kᵢ-Werts am ORL1-Rezeptor sind dem Fachmann bekannt. Bevorzugt erfolgt die Bestimmung wie im Zusammenhang mit den Beispielen beschrieben.

Überraschenderweise hat sich gezeigt, dass Verbindungen mit Affinität zum ORL1- und µ-Opioid-Rezeptor, bei denen das durch 1/[K_{i(ORL1)}/K_{i(µ)}] definierte Verhältnis von ORL1 zu µ im Bereich von 0,1 bis 30 liegt, vorzugsweise von 0,1 bis 25, ein pharmakologisches Profil aufweisen, das verglichen mit dem anderer Opioidrezeptorliganden deutliche Vorteile aufweist:
1. Die erfindungsgemäßen Verbindungen zeigen eine Wirksamkeit in Akutschmerzmodellen, die mitunter vergleichbar ist mit der gebräuchlicher Stufe-3 Opioide. Gleichzeitig zeichnen sie sich aber durch eine gegenüber klassischen µ-Opioiden deutlich bessere Verträglichkeit aus.
2. Im Gegensatz zu gebräuchlichen Stufe-3 Opioiden zeigen die erfindungsgemäßen Verbindungen eine deutlich höhere Wirksamkeit in Mono- und Polyneuropathieschmerzmodellen, was auf einen Synergismus von ORL1- und µ-Opioid Komponente zurückzuführen ist.
3. Im Gegensatz zu gebräuchlichen Stufe-3 Opioiden zeigen die erfindungsgemäßen Verbindungen in neuropathischen Tieren eine weitgehende, bevorzugt eine vollständige, Trennung von antiallodynischer bzw. antihyperalgetischer Wirkung und antinociceptivem Effekt.
4. Im Gegensatz zu gebräuchlichen Stufe-3 Opioiden zeigen die erfindungsgemäßen Verbindungen in Tiermodellen für chronischen Entzündungsschmerz (u.a. Carrageenan- oder CFA-induzierte Hyperalgesie, viszeraler Entzündungsschmerz) eine deutliche Wirkverstärkung gegenüber dem Akutschmerz.
5. Im Gegensatz zu gebräuchlichen Stufe-3 Opioiden sind µ-opioidtypische Nebenwirkungen (u.a. Atemdepression, opioidinduzierte Hyperalgesie, körperliche Abhängigkeit/Entzug, psychische Abhängigkeit/Sucht) bei den erfindungsgemäßen Verbindungen im therapeutisch wirksamen Dosisbereich deutlich reduziert, bzw. vorzugsweise nicht zu beobachten.

Aufgrund der reduzierten µ-opioiden Nebenwirkungen einerseits und der erhöhten Wirksamkeit bei chronischem, bevorzugt neuropathischem Schmerz andererseits zeichnen sich die gemischten ORL1/µ-Agonisten somit durch deutlich vergrößerte Sicherheitsabstände gegenüber reinen µ-Opioiden aus. Daraus resultiert ein deutlich vergrößertes "therapeutisches Fenster" bei der Behandlung von Schmerzzuständen, bevorzugt chronischen Schmerzen, noch bevorzugter neuropathischen Schmerzen.

Bevorzugte Ausführungsformen der erfindungsgemäßen Verbindungen der allgemeinen Formel (1) haben die allgemeine Formel (1.1), (1.2), (1.3), (1.4), (1.5), (1.6) oder (1.7): 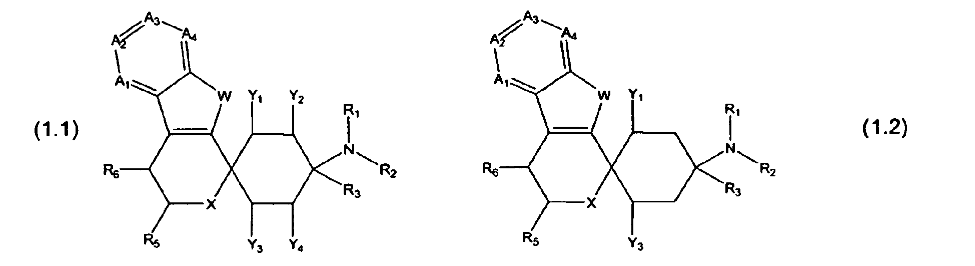 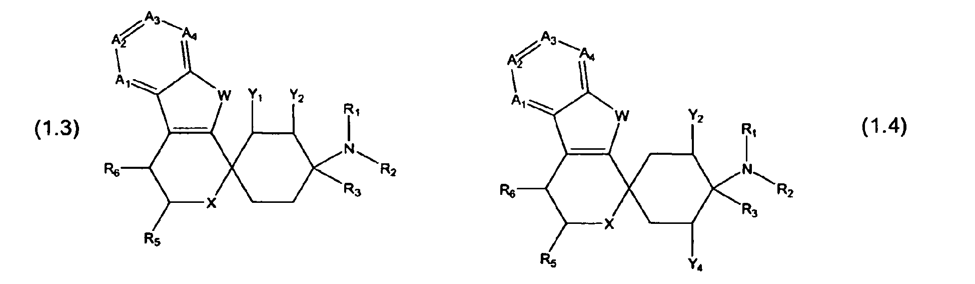 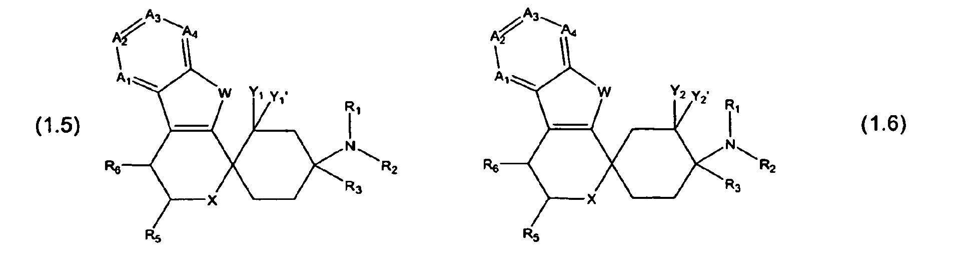 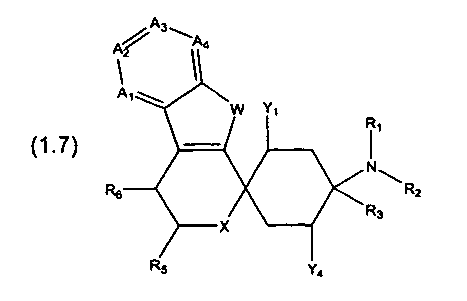 bei denen jeweils einige der Reste Y₁, Y₁', Y₂, Y₂', Y₃, Y₃', Y₄ und Y₄' jeweils -H bedeuten und nur diejenigen Reste, welche ggf. ungleich -H sind (aber auch -H sein können), abgebildet sind.

In einer bevorzugten Ausführungsform der erfindungsgemäßen Verbindungen (1), (1.1), (1.2), (1.3), (1.4), (1.5), (1.6) oder (1.7) sind A₁, A₂, A₃ und A₄ ungleich -N=. In einer anderen bevorzugten Ausführungsform der erfindungsgemäßen Verbindungen (1), (1.1), (1.2), (1.3), (1.4), (1.5), (1.6) oder (1.7) sind drei der Reste A₁, A₂, A₃ und A₄ ungleich -N= und der übrige der Reste ist gleich -N=. Vorzugsweise sind A₁, A₂ und A₃ ungleich -N=; oder A₁, A₂ und A₄ sind ungleich -N=; oder A₁, A₃ und A₄ sind ungleich -N=; oder A₂, A₃ und A₄ sind ungleich -N=. In einer anderen bevorzugten Ausführungsform der erfindungsgemäßen Verbindungen (1), (1.1), (1.2), (1.3), (1.4), (1.5), (1.6) oder (1.7) sind zwei der Reste A₁, A₂, A₃ und A₄ ungleich -N= und die anderen beiden Reste sind -N=. Vorzugsweise sind A₁ und A₂ -N= und A₃ und A₄ sind ungleich -N=; oder A₂ und A₃ sind -N= und A₁ und A₄ sind ungleich -N=; oder A₃ und A₄ sind -N= und A₁ und A₂ sind ungleich -N=; oder A₁ und A₃ sind -N= und A₂ und A₄ sind ungleich -N=; oder A₁ und A₄ sind -N= und A₂ und A₃ sind ungleich -N=; oder A₂ und A₄ sind -N= und A₁ und A₃ sind ungleich -N=.

Eine bevorzugte Ausführungsform der Erfindung betrifft Verbindungen der allgemeinen Formel (1.1.1), (1.1.2), (1.1.3) oder (1.1.4)

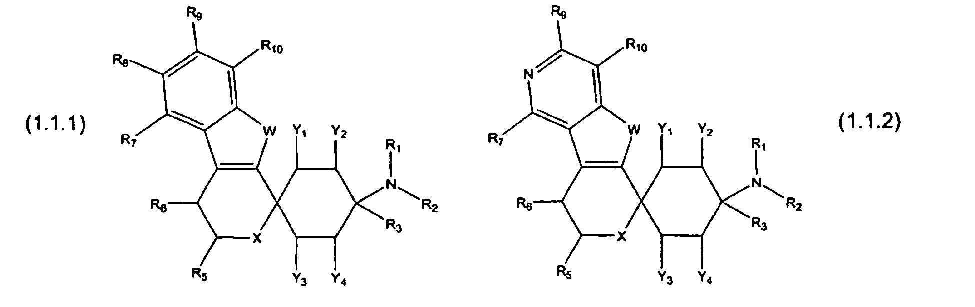

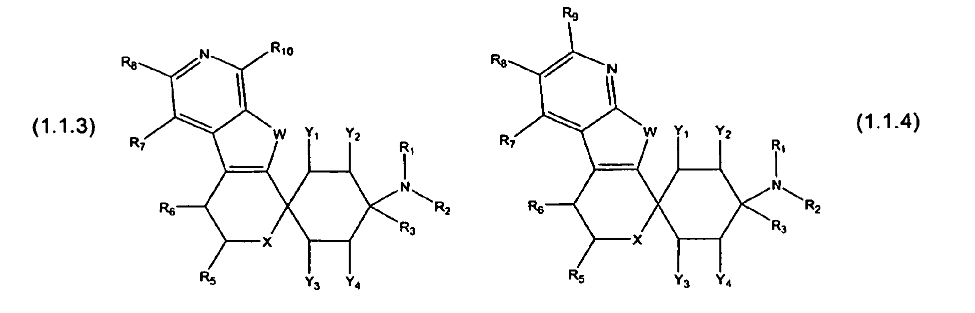

Eine andere bevorzugte Ausführungsform der Erfindung betrifft Verbindungen der allgemeinen Formel (1.2.1), (1.2.2), (1.2.3) oder (1.2.4)

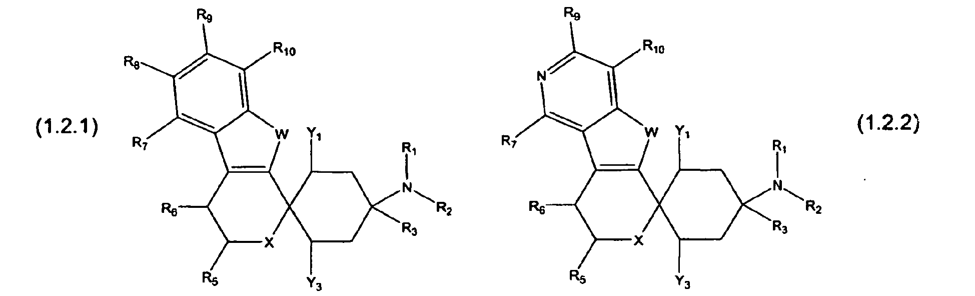

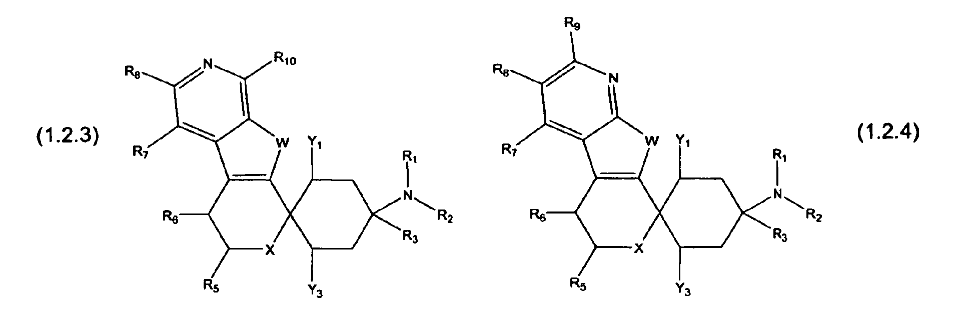

Eine andere bevorzugte Ausführungsform der Erfindung betrifft Verbindungen der allgemeinen Formel (1.3.1), (1.3.2), (1.3.3) oder (1.3.4)

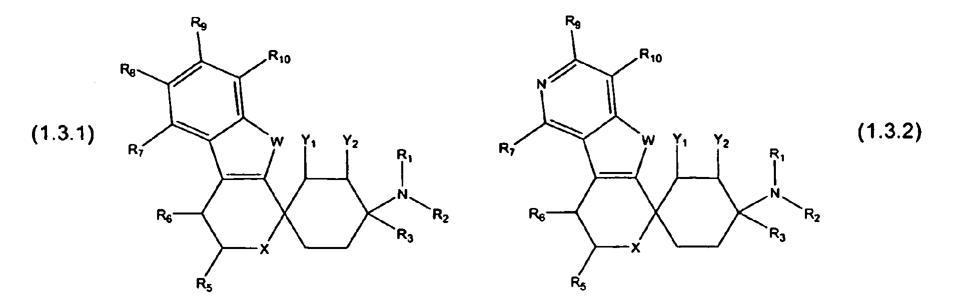

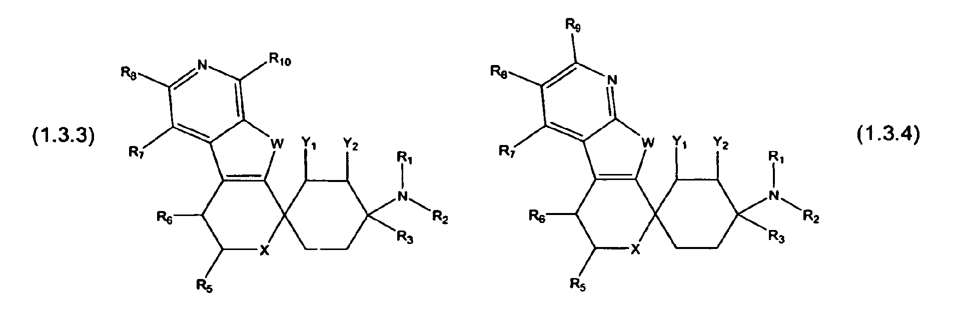

Eine weitere bevorzugte Ausführungsform der Erfindung betrifft Verbindungen der allgemeinen Formel (1.4.1), (1.4.2), (1.4.3) oder (1.4.4)

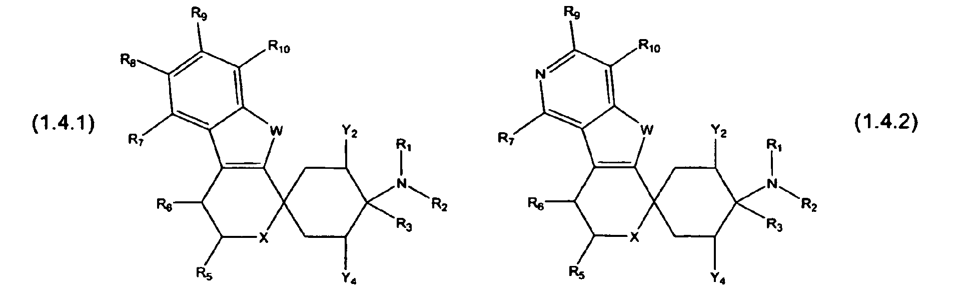

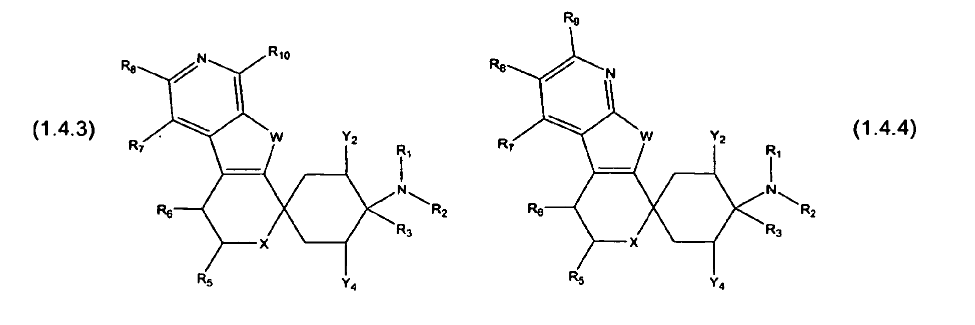

Eine weitere bevorzugte Ausführungsform der Erfindung betrifft Verbindungen der allgemeinen Formel (1.5.1), (1.5.2), (1.5.3) oder (1.5.4)

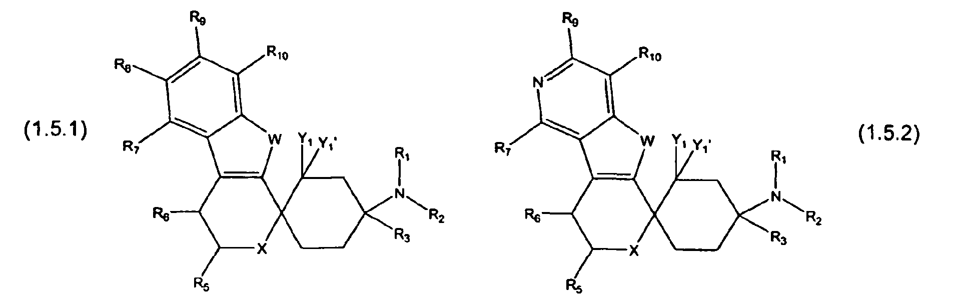

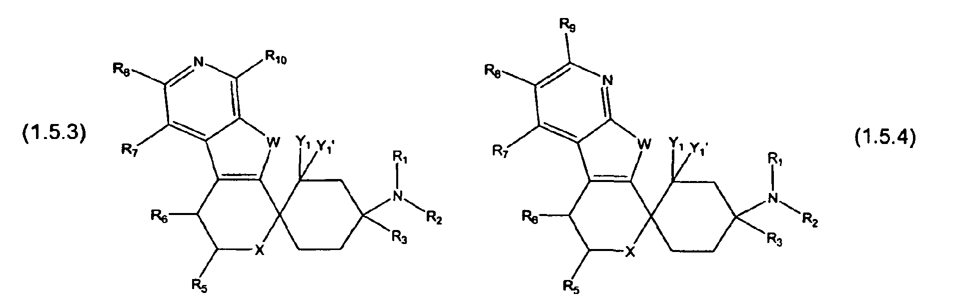

Eine weitere bevorzugte Ausführungsform der Erfindung betrifft Verbindungen der allgemeinen Formel (1.6.1), (1.6.2), (1.6.3) oder (1.6.4)

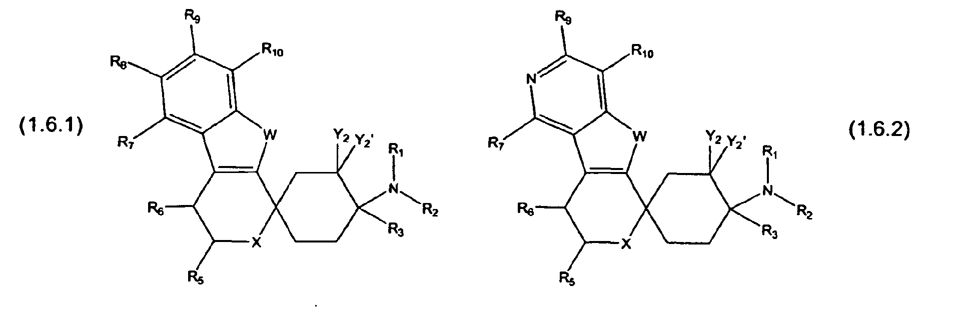

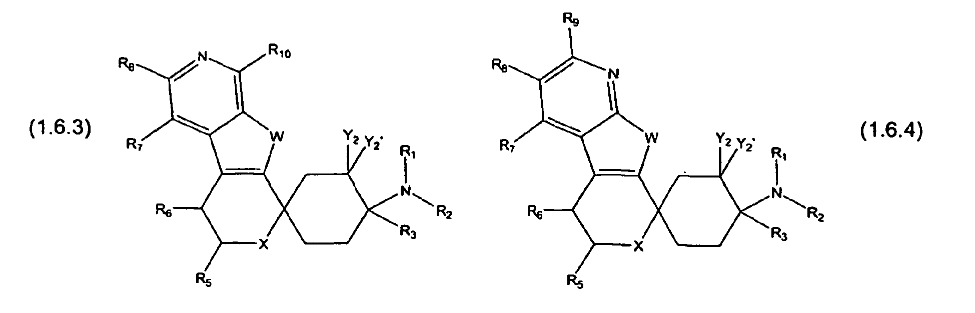

Eine weitere bevorzugte Ausführungsform der Erfindung betrifft Verbindungen der allgemeinen Formel (1.7.1), (1.7.2), (1.7.3) oder (1.7.4)

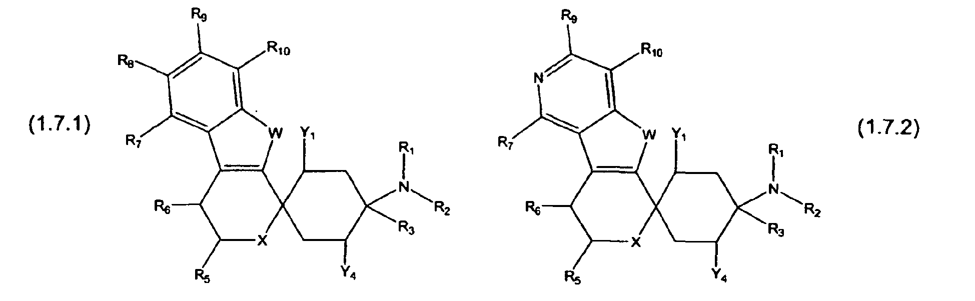

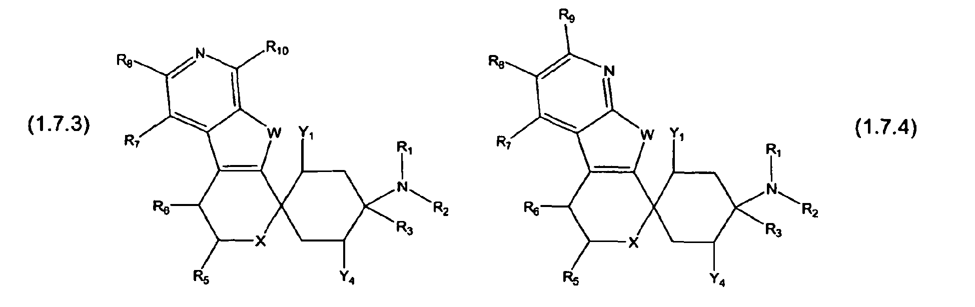

Weitere bevorzugte Ausführungsformen der erfindungsgemäßen Verbindungen der allgemeinen Formel (1) haben die allgemeine Formel (2): 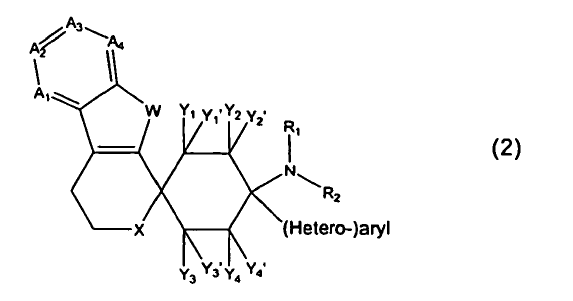 worin (Hetero-)aryl für -Aryl oder -Heteroaryl steht, jeweils unsubstituiert oder ein- oder mehrfach substituiert.

Bevorzugte Ausführungsformen der Verbindungen der allgemeinen Formel (2) haben die allgemeine Formel (2.1), (2.2), (2.3) oder (2.4): 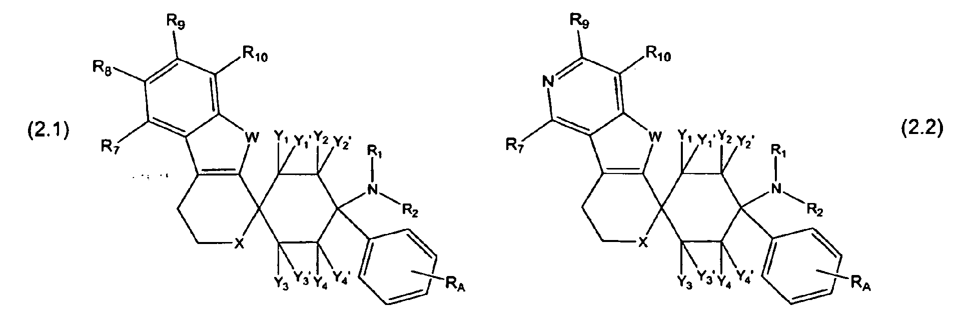 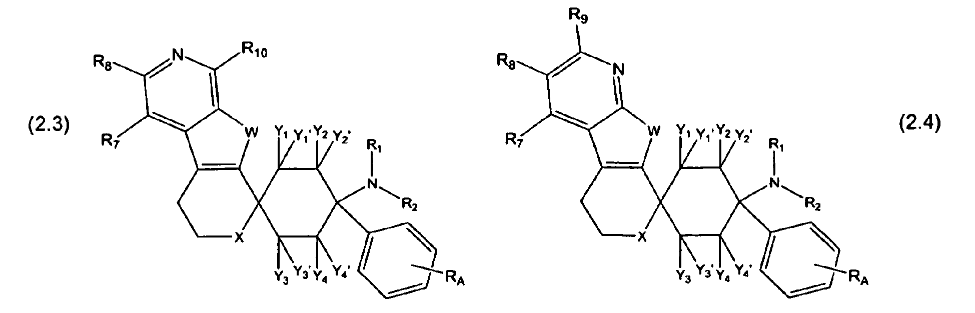 worin R_{A} für -H, -F, -Cl, -CN oder -CH₃ steht, bevorzugt für -H.

Bei den Verbindungen der allgemeinen Formeln (2), (2.1), (2.2), (2.3) und (2.4) ist W bevorzugt -NH-.

Besonders bevorzugte Ausführungsformen der Verbindungen der allgemeinen Formel (2.1) haben die allgemeine Formel (2.1.1) und besonders bevorzugte Ausführungsformen der Verbindungen der allgemeinen Formel (2.4) haben die allgemeine Formel (2.4.1):

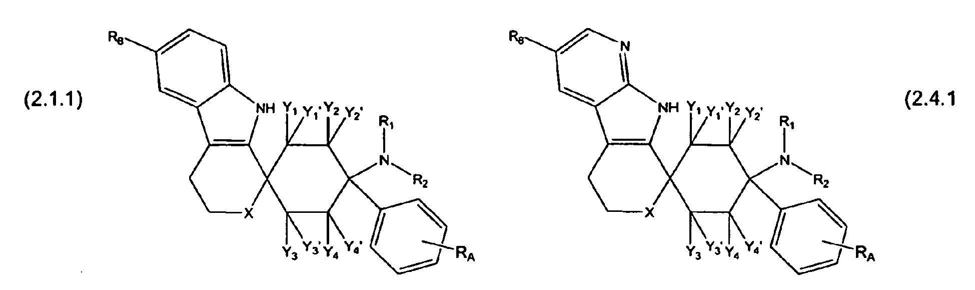

Besonders bevorzugt sind Verbindungen der allgemeinen Formel (2.1.1) oder (2.1.4), worin X für -O- oder -NR₁₇- steht, bevorzugt für -O- oder -NH-;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloatiphat, -C₁₋₈-Aliphat-Aryl, -C₁₋₈-Aliphat-Heteroaryl, -C₃₋₈-Cycloaliphat-C₁₋₈-Aliphat, - C₃₋₈-Cycloaliphat-Aryl oder -C₃₋₈-Cycloaliphat-Heteroaryl steht;
R₁ für -CH₃ steht;
R₂ für -H oder -CH₃ steht;
R₈ für -H oder -F steht;
R₁₂ jeweils unabhängig für -H, R₀, -OR₁₃, oder -NR₁₄R₁₅ steht;
R₁₃ jeweils unabhängig für -H oder R₀ steht;
R₁₄ und R₁₅ unabhängig voneinander für -H oder R₀ stehen; oder R₁₄ und R₁₅ zusammen für - CH₂CH₂OCH₂CH₂-, -CH₂CH₂NR₁₆CH₂CH₂- oder -(CH₂)₃₋₆- stehen;
R₁₆ für -H oder -C₁₋₆-Aliphat steht;
R₁₇ für -H, -R₀, -COR₁₂ oder -S(=O)₂R₁₂ steht; und
Y₁, Y₁', Y₂, Y₂', Y₃, Y₃', Y₄ und Y₄ jeweils unabhängig voneinander ausgewählt sind aus der Gruppe bestehend aus -H, -F, -Cl, -CN, -C₁₋₈-Aliphat, -C₁₋₈-Aliphat-NHC₁₋₈-Aliphat, -C₁₋₈-Aliphat-N(C₁₋₈-Aliphat)₂, -S-C₁₋₈-Aliphat, -S-Aryl, -Aryl und -C₁₋₈-Aliphat-Aryl; mit der Maßgabe, dass wenigstens einer der Reste Y₁, Y₁', Y₂, Y₂', Y₃, Y₃', Y₄ und Y₄' ungleich -H ist; und
R_{A} für -H, -F, -Cl, -CN oder -CH₃ steht.

In einer bevorzugten Ausführungsform der erfindungsgemäßen Verbindungen ist W -NR₄- und X ist -NR₁₇-, -O-, -S-, -S(=O)₁₋₂- (d.h. -S(=O)- oder -S(=O)₂-), oder -CR₁₈R₁₉-.

In einer anderen bevorzugten Ausführungsform der erfindungsgemäßen Verbindungen ist W -O- und X ist -NR₁₇-, -O-, -S-, -S(=O)₁₋₂-, oder -CR₁₈R₁₉-.

In einer anderen bevorzugten Ausführungsform der erfindungsgemäßen Verbindungen ist W -S- und X ist -NR₁₇-, -O-, -S-, -S(=O)₁₋₂-, oder -CR₁₈R₁₉-.

Bevorzugt sind Y₁, Y₁', Y₂, Y₂', Y₃, Y₃', Y₄ und Y₄' jeweils unabhängig voneinander ausgewählt aus der Gruppe bestehend aus -H, -F, -Cl, -Br, -I, -CN, -NH₂, -NH-C₁₋₆-Aliphat, -NH-C₃₋₈-Cycloaliphat, -NH-C₁₋₆-Aliphat-OH, -N(C₁₋₆-Aliphat)₂, -N(C₃₋₈-Cycloaliphat)₂, -N(C₁₋₈-Aliphat-OH)₂, -NO₂, -NH-C₁₋₆-Aliphat-C₃₋₈-Cycloaliphat, -NH-C₁₋₆-Aliphat-Aryl, -NH-C₁₋₆-Aliphat-Heteroaryl, -NH-Aryl, -NH-Heteroaryl, -SH, -S-C₁₋₆-Aliphat, -S-C₃₋₈-Cycloaliphat, -S-C₁₋₆-Aliphat-C₃₋₈-Cycloaliphat, -S-C₁₋₆-Aliphat-Aryl, -S-C₁₋₈-Aliphat-Heteroaryl, -S-Aryl, -S-Heteroaryl, -OH, -O-C₁₋₆-Aliphat, -O-C₃₋₈-Cycloaliphat, -O-C₁₋₆-Aliphat-OH, -O-C₁₋₆-Aliphat-C₃₋₈-Cycloaliphat, -O-C₁₋₆-Aliphat-Aryl, -O-C₁₋₆-Aliphat-Heretoaryl, -O-Aryl, -O-Heteroaryl, -O-C(=O)C₁₋₆-Aliphat, -O-C(=O)C₃₋₈-Cycloaliphat, -O-C(=O)C₁₋₆-Aliphat-OH, -O-C(=O)C₁₋₈-Aliphat-C₃₋₈-Cycloaliphat, -O-C(=O)C₁₋₆-Aliphat-Ary, -O-C(=O)C₁₋₈-Aliphat-Heretoaryl, -O-C(=O)Aryl, -O-C(=O)Heteroaryl, -C₁₋₆-Aliphat, -C₁₋₈-Aliphat-NHC₁₋₈-Aliphat, -C₁₋₈-Aliphat-N(C₁₋₈-A)iphat)₂, -C₃₋₈-Cycloaliphat, -C₁₋₆-Aliphat-C₃₋₈-Cycloaliphat, -C₁₋₆-Aliphat-Aryl, -C₁₋₈-Aliphat-Heteroaryl, -Aryl, -Heteroaryl, -C(=O)C₁₋₈-Aliphat, -C(=O)C₃₋₈-Cycloaliphat, -C(=O)C₁₋₆-Aliphat-C₃₋₈-Cycloaliphat, -C(=O)C₁₋₆-Aliphat-Aryl, -C(=O)C₁₋₆-Aliphat-Heteroaryl, -C(=O)Aryl, -C(=O)Heteroaryl, -CO₂H, -CO₂-C₁₋₈-Aliphat, -CO₂-C₃₋₈-Cycloaliphat, -CO₂-C₁₋₆-Aliphat-C₃₋₈-Cycloaliphat, -CO₂-C₁₋₆-Aliphat-Aryl, -CO₂-C₁₋₆-Aliphat-Heteroaryl, -CO₂-Aryl, -CO₂-Heteroaryl; oder Y₁ und Y₁', oder Y₂ und Y₂', oder Y₃ und Y₃', oder Y₄ und Y₄' stehen gemeinsam für =O; mit der Maßgabe, dass wenigstens einer der Reste Y₁, Y₁', Y₂, Y_{2'}, Y₃, Y₃', Y₄ und Y₄' nicht für -H steht.

Bevorzugter sind Y₁, Y₁', Y₂, Y₂', Y₃, Y₃', Y₄ und Y₄' jeweils unabhängig voneinander ausgewählt aus der Gruppe bestehend aus -H, -F, -Cl, -Br, -I, -CN, -C₁₋₆-Aliphat, -C₁₋₈-Aliphat-NHC₁₋₈-Aliphat, -C₁₋₆-Aliphat-N(C₁₋₈-Aliphat)₂, -C₃₋₈-Cycloaliphat, -C₁₋₈-Aliphat-C₃₋₈-Cycloaliphat, -C₁₋₈-Aliphat-Aryl, -C₁₋₈-Aliphat-Heteroaryl, -S-C₁₋₈-Aliphat, -S-Aryl, -Aryl oder -Heteroaryl.

Besonders bevorzugt sind Y₁, Y₁', Y₂, Y₂', Y₃, Y₃', Y₄ und Y₄' jeweils unabhängig voneinander ausgewählt aus der Gruppe bestehend aus -H, -F, -Cl, -C₁₋₆-Alkyl, -C₂₋₆-Alkenyl, -C₁₋₆-Alkyl-NH-C₁₋₈-Alkyl, -C₁₋₆-Alkyl-N(C₁₋₆-Alkyl)₂, -Aryl, -C₁₋₈-Alkyl-Aryl, -S-C₁₋₆-Alkyl und -S-Aryl.

In einer besonders bevorzugten Ausführungsform steht einer der Reste Y₁, Y₁', Y₂, Y₂', Y₃, Y₃', Y₄ und Y₄', vorzugsweise Y₁, Y₁', Y₃ oder Y₃', oder stehen zwei der Reste Y₁, Y₁', Y₂, Y₂', Y₃, Y₃', Y₄ und Y₄' jeweils für -C₁₋₈-Aliphat, vorzugsweise für -CH₃, -CH₂CH₃, -CH₂CH₂CH₃ oder -CH₂CH=CH₂, und die übrigen Reste stehen für -H.

Bevorzugte Vertreter sind die Verbindungen E-1 bis E-9, bei denen X jeweils -O- oder -NHist:

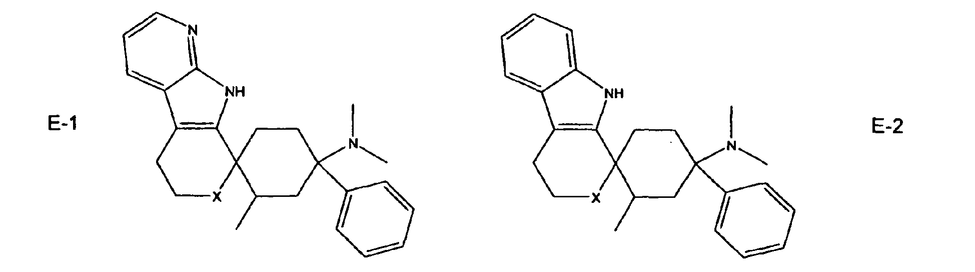

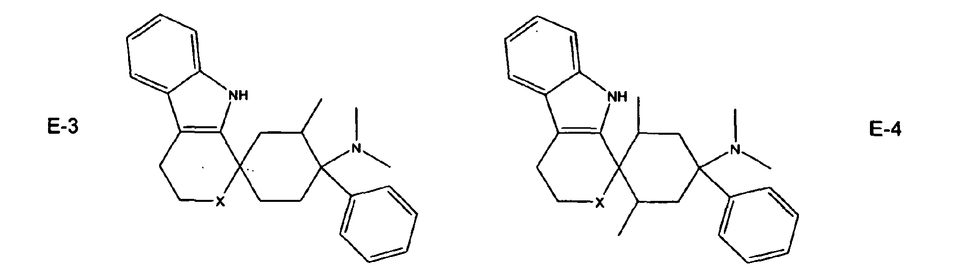

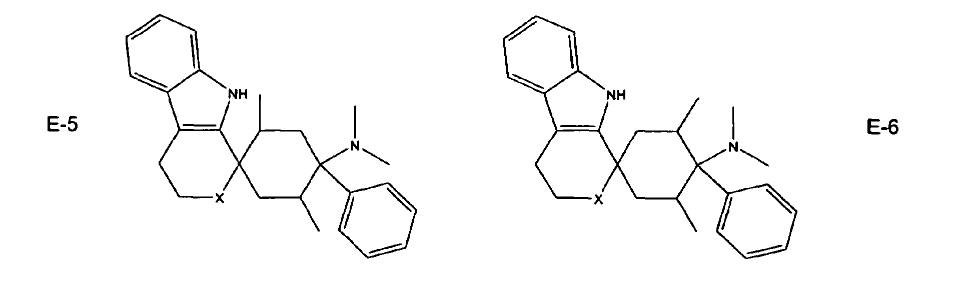

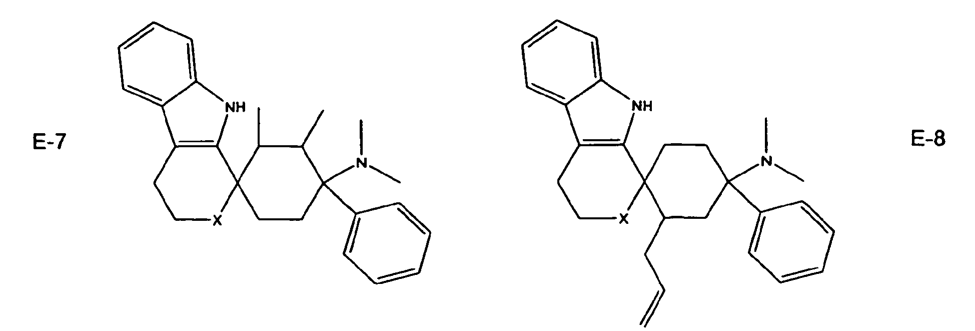

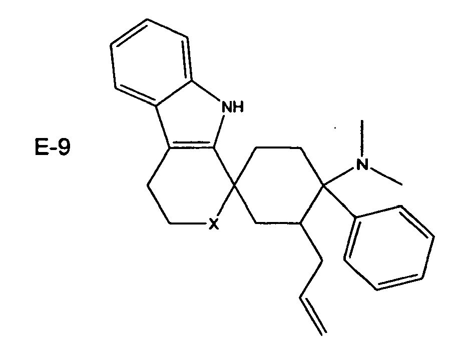

In einer anderen besonders bevorzugten Ausführungsform steht einer der Reste Y₁, Y₁', Y₂, Y₂', Y₃, Y₃', Y₄ und Y₄', vorzugsweise Y₁, Y₁', Y₃ oder Y₃', für -Aryl (vorzugsweise -Phenyl oder 4-Fluor-Phenyl) oder -C₁₋₆-Aliphat-Aryl (vorzugsweise -Benzyl oder 4-Fluor-benzyl) und die übrigen Reste stehen für -H.

Bevorzugte Vertreter sind die Verbindungen E-10 bis E-13, bei denen X jeweils -O- oder -NH- ist:

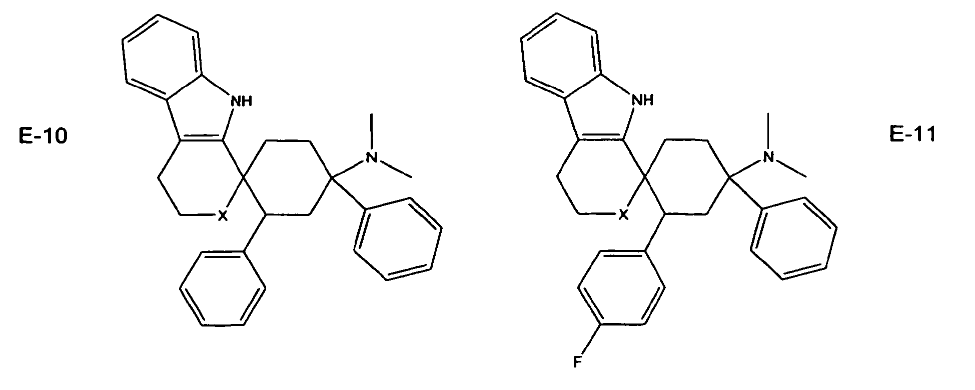

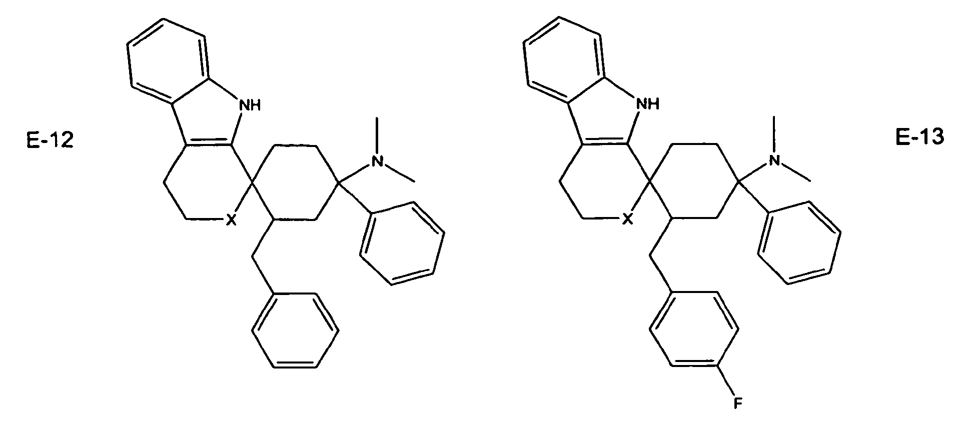

In einer anderen besonders bevorzugten Ausführungsform steht einer der Reste Y₁, Y₁', Y₂, Y₂', Y₃, Y₃', Y₄ und Y₄', vorzugsweise Y₁, Y₁', Y₃ oder Y₃', oder stehen zwei der Reste Y₁, Y₁', Y₂, Y₂', Y₃, Y₃', Y₄ und Y₄' jeweils für -F und die übrigen Reste stehen für -H.

Bevorzugte Vertreter sind die Verbindungen E-14 bis E-17, bei denen X jeweils -O- oder - NH- ist:

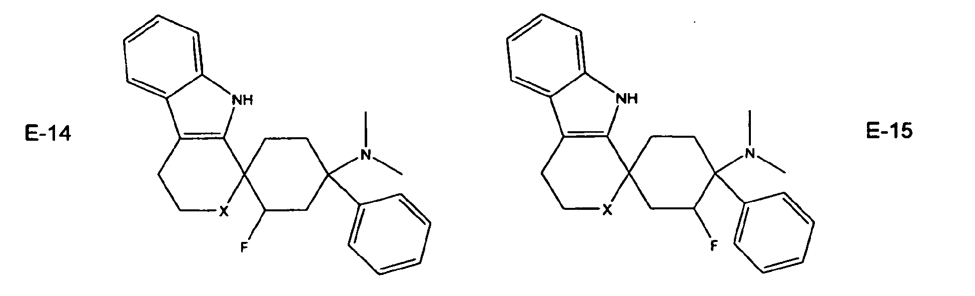

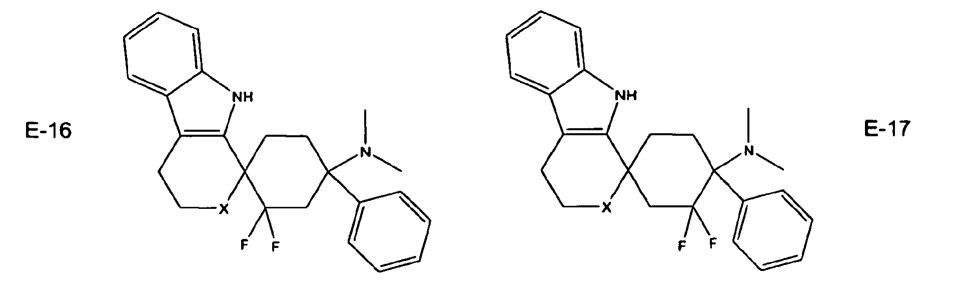

In einer anderen besonders bevorzugten Ausführungsform steht einer der Reste Y₁, Y₁', Y₂, Y₂', Y₃, Y₃', Y₄ und Y₄', vorzugsweise Y₁, Y₁', Y₃ oder Y₃', für -S-C₁₋₆-Aliphat (vorzugsweise - S-C₁₋₆-Alkyl), -S-Aryl (vorzugsweise -S-Phenyl) oder -C₁₋₆-Aliphat-N(C₁₋₆-Aliphat)₂ (vorzugsweise -C₁₋₆-Alkyl-N(C₁₋₆-Alkyl)₂) und die übrigen Reste stehen für -H.

Bevorzugte Vertreter sind die Verbindungen E-18 bis E-20, bei denen X jeweils -O- oder -NH- ist:

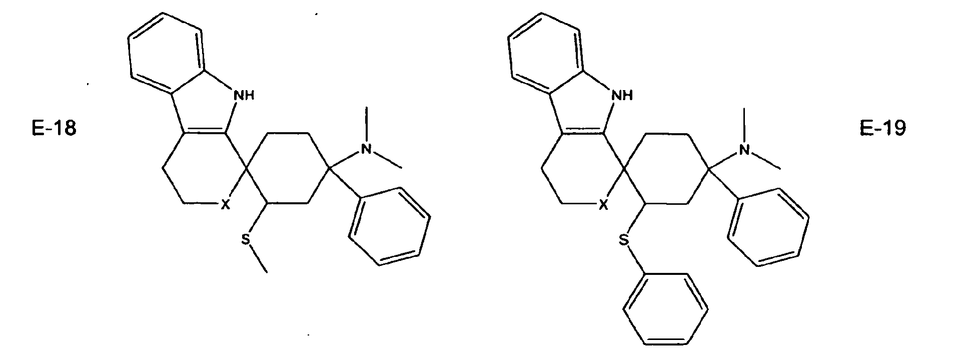

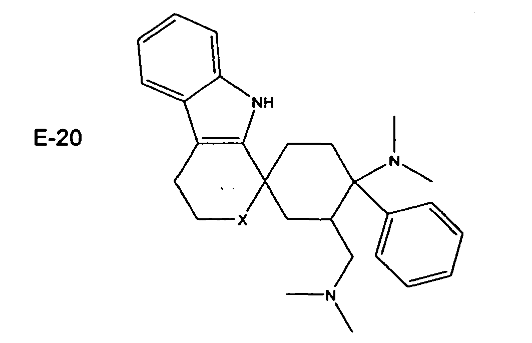

R₀ steht bevorzugt jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl oder -C₁₋₈-Aliphat-Heteroaryl. Dabei bedeuten -C₁₋₈-Aliphat-C₃₋₁₂-Cycloaliphat, -C₁₋₈-Aliphat-Aryl oder -C₁₋₈-Aliphat-Heteroaryl, dass die Reste -C₃₋₁₂-Cycloaliphat, -Aryl oder -Heteroaryl jeweils über eine zweibindige Brücke -C₁₋₈-Aliphat- gebunden sind. Bevorzugte Beispiele für -C₁₋₈-Aliphat-Aryl sind -CH₂-C₆H₅, -CH₂CH₂-C₆H₅, und -CH=CH-C₆H₅.

Bevorzugt stehen R₁ und R₂, unabhängig voneinander für -H; -C₁₋₈-Aliphat; -C₃₋₈-Cycloaliphat, -C₁₋₈-Aliphat-Aryl, -C₁₋₈-Aliphat-C₃₋₈-Cycloaliphat oder -C₁₋₈-Aliphat-Heteroaryl; oder die Reste R₁ und R₂ bilden zusammen einen Ring und bedeuten -CH₂CH₂OCH₂CH₂-, -CH₂CH₂NR₁₁CH₂CH₂- oder -(CH₂)₃₋₆-.

Bevorzugter stehen R₁ und R₂ unabhängig voneinander für -H; -C₁₋₅-Aliphat; oder die Reste R₁ und R₂ bilden zusammen einen Ring und bedeuten -CH₂CH₂OCH₂CH₂-, -CH₂CH₂NR₁₁-CH₂CH₂- oder -(CH₂)₃₋₆-, wobei R₁₁ bevorzugt -H oder -C₁₋₅-Aliphat bedeutet.

Besonders bevorzugt sind Verbindungen, worin R₁ und R₂ unabhängig voneinander für -CH₃ oder -H stehen, wobei R₁ und R₂ nicht gleichzeitig -H bedeuten; oder R₁ und R₂ bilden einen Ring und bedeuten -(CH₂)₃₋₄-.

Ganz besonders bevorzugt sind Verbindungen, worin R₁ und R₂ für -CH₃ stehen.

Bevorzugt steht R₃ für -C₁₋₈-Aliphat, -C₃₋₈-Cycloaliphat, -Aryl, -Heteroaryl; oder für jeweils über eine -C₁₋₃-Aliphat-Gruppe gebundenes -Aryl, -Heteroaryl oder -C₃₋₈-Cycloaliphat.

Besonders bevorzugt steht R₃ für -Ethyl, -Propyl, -Butyl, -Pentyl, -Hexyl, -Heptyl, -Cyclopentyl, -Cyclohexyl, -Phenyl, -Benzyl, -Naphthyl, -Anthracenyl, -Thiophenyl, -Benzothiophenyl, -Furyl, -Benzofuranyl, -Benzodioxolanyl, -Indolyl, -Indanyl, -Benzodioxanyl, -Pyrrolyl, -Pyridyl, -Pyrimidyl oder -Pyrazinyl, jeweils unsubstituiert oder ein- oder mehrfach substituiert; über eine gesättigte, unverzweigte -C₁₋₃-Aliphat-Gruppe gebundenes -C₅₋₆-Cycloaliphat, -Phenyl, -Naphthyl, -Anthracenyl, -Thiophenyl, -Benzothiophenyl, -Pyridyl, -Furyl, -Benzofuranyl, -Benzodioxolanyl, -Indolyl, -Indanyl, -Benzodioxanyl, -Pyrrolyl, -Pyrimidyl, -Triazolyl oder -Pyrazinyl, jeweils unsubstituiert oder ein- oder mehrfach substituiert.

Bevorzugter steht R₃ für -Propyl, -Butyl, -Pentyl, -Hexyl, -Phenyl, -Furyl, -Thiophenyl, -Naphthyl, -Benzyl, -Benzofuranyl, -Indolyl, -Indanyl, -Benzodioxanyl, -Benzodioxolanyl, -Pyridyl, -Pyrimidyl, -Pyrazinyl, -Triazolyl oder -Benzothiophenyl, jeweils unsubstituiert oder ein- oder mehrfach substituiert; über eine gesättigte, unverzweigte -C₁₋₃-Aliphat-Gruppe gebundenes -Phenyl, -Furyl oder -Thiophenyl, jeweils unsubstituiert oder ein- oder mehrfach substituiert.

Noch bevorzugter steht R₃ für -Propyl, -Butyl, -Pentyl, -Hexyl, -Phenyl, -Phenethyl, -Thiophenyl, -Pyridyl, -Triazolyl, -Benzothiophenyl oder -Benzyl, jeweils substituiert oder unsubstituiert, besonders bevorzugt für -Propyl, -3-Methoxypropyl, -Butyl, -Pentyl, -Hexyl, -Phenyl, -3-Methylphenyl, -3-Fluorphenyl, -Benzo[1,3]-dioxolyl, -Thienyl, -Benzothiophenyl, -4-Chlorbenzyl, -Benzyl, -3-Chlorbenzyl, -4-Methylbenzyl, -2-Chlorbenzyl, -4-Fluorbenzyl, -3-Methylbenzyl, -2-Methylbenzyl, -3-Fluorbenzyl, -2-Fluorbenzyl, -1-Methyl-1,2,4-triazolyl oder -Phenethyl.

Ganz besonders bevorzugt steht R₃ für -Butyl, -Ethyl, -3-Methoxypropyl, -Benzothiophenyl, -Phenyl, -3-Methylphenyl, -3-Fluorphenyl, -Benzo[1,3]-dioxolyl, -Benzyl, -1-Methyl-1,2,4-triazolyl, -Thienyl oder -Phenethyl.

Am bevorzugtesten steht R₃ für -Phenyl, -Benzyl oder -Phenethyl, jeweils unsubstituiert oder am Ring ein- oder mehrfach substituiert; -C₁₋₅-Aliphat, -C₄₋₈-Cycloaliphat, -Pyridyl, -Thienyl, -Thiazolyl, -Imidazolyl, -1,2,4 Triazolyl oder -Benzimidazolyl, unsubstituiert oder ein- oder mehrfach substituiert.

Insbesondere bevorzugt steht R₃ für -Phenyl, -Benzyl, -Phenethyl, -Thienyl, -Pyridyl, -Thiazolyl, -Imidazolyl, -1,2,4 Triazolyl, -Benzimidazolyl oder -Benzyl, unsubstituiert oder ein- oder mehrfach substituiert mit -F, -Cl, -Br, -CN, -CH₃, -C₂H₅, -NH₂, -NO₂, -SH, -CF₃, -OH, -OCH₃, -OC₂H₅ oder -N(CH₃)₂; -Ethyl, -n-Propyl, -2-Propyl, -Allyl, -n-Butyl, -iso-Butyl, -sec.-Butyl, -tert.-Butyl, -n-Pentyl, -iso-Pentyl, -neo-Pentyl, -n-Hexyl, -Cyclopentyl oder -Cyclohexyl, jeweils unsubstituiert oder ein- oder mehrfach substituiert mit -OH, -OCH₃ oder -OC₂H₂, wobei vorzugsweise -Thienyl, -Pyridyl, -Thiazolyl, -Imidazolyl, -1,2,4 Triazolyl und -Benzimidazolyl unsubstituiert sind.

Insbesondere bevorzugt steht R₃ für -Phenyl, unsubstituiert oder einfach substituiert mit -F, -Cl, -CN, -CH₃; -Thienyl; -Ethyl, -n-Propyl oder -n-Butyl, unsubstituiert oder ein- oder mehrfach substituiert mit -OCH₃, -OH oder -OC₂H₅, insbesondere mit -OCH₃.

Bevorzugt steht R₄ für -H; -C₁₋₆-Aliphat, -Aryl, -Heteroaryl, -C₁₋₆-Aliphat-Aryl, -C₁₋₈-Aliphat-Heteroaryl oder -C₁₋₈-Aliphat-Cycloaliphat, -COR₁₂ oder -SO₂R₁₂.

Besonders bevorzugt steht R₄ für -H.

Bevorzugt steht R₅ für =O; -H; -COOR₁₃, -CONR₁₃, -OR₁₃, -C₁₋₆-Aliphat, C₃₋₈-Cycloaliphat, Aryl-, oder Heteroaryl, oder jeweils über C₁₋₃-Aliphat gebundenes Aryl, C₃₋₈-Cycloaliphat oder Heteroaryl.

Bevorzugt steht R₆ für -H, -F, -Cl, -NO₂, -CF₃, -OR₁₃, -SR₁₃, -SO₂R₁₃, -SO₂OR₁₃, -CN, -COOR₁₃, -NR₁₄R₁₅, -C₁₋₅-Aliphat, -C₃₋₈-Cycloaliphat, -Aryl- oder Heteroaryl; oder jeweils über C₁₋₃-Aliphat gebundenes -Aryl, -C₃₋₈-Cycloaliphat oder -Heteroaryl; oder R₅ und R₆ bedeuten bevorzugt gemeinsam -(CH₂)ₙ- mit n = 2, 3, 4, 5 oder 6, wobei einzelne Wasserstoffatome auch durch -F, -Cl, -Br, -I, -NO₂, -CF₃, -OR₁₃, -CN oder -C₁₋₅-Aliphat ersetzt sein können.

Außerdem bevorzugt steht R₅ für -H, -C₁₋₅-Aliphat oder -COOR₁₃. Besonders bevorzugt steht R₅ für -CH₃, -CH₂OH, -COOH oder -COOCH₃. Ganz besonders bevorzugt steht R₅ für -H.

Bevorzugt sind auch Verbindungen, bei denen R₆ für -H, -C₁₋₅-Aliphat, -Aryl oder über eine -C₁₋₃-Aliphat-Gruppe (Brücke) verknüpftes -Aryl steht. Besonders bevorzugt steht R₆ für -H, -CH₃, -Phenyl oder -Benzyl. Ganz besonders bevorzugt steht R₆ für -H.

Bevorzugt stehen R₇, R₈, R₉ und R₁₀ jeweils unabhängig voneinander für -H, -F, -Cl, -Br, -I, -NO₂, -CF₃, -OR₁₃, -SR₁₃, -SO₂R₁₃, -SO₂OR₁₃, -CN, -COOR₁₃, -NR₁₄R₁₅; -C₁₋₅-Aliphat, -C₃₋₈-Cycloaliphat; -Aryl oder -Heteroaryl; oder jeweils über -C₁₋₃-Aliphat gebundenes -Aryl, -C₃₋₈-Cycloaliphat oder -Heteroaryl.

Bevorzugter stehen R₇, R₈, R₉ und R₁₀ jeweils unabhängig voneinander für -H, -Methyl, -Ethyl, -Propyl, -Butyl, -Pyridyl, -O-Benzyl, -F, -Cl, -Br, -I, -CF₃, -OH, -OCH₃, -NH₂, -COOH, -CO-OCH₃, -NHCH₃, -N(CH₃)² oder -NO₂.

Besonders bevorzugt stehen R₇, R₈, R₉ und R₁₀ jeweils unabhängig voneinander für -H, -F, -OH, -CH₃, -Cl, -OCH₃, -Br oder -NO₂.

In einer bevorzugten Ausführungsform stehen R₇, R₈, R₉ und R₁₀ für -H.

In einer anderen bevorzugten Ausführungsform stehen drei der Reste R₇, R₈, R₉ und R₁₀ für -H und der übrige Rest, vorzugsweise R₈ oder R₉, ist ungleich -H, vorzugsweise -F, -Cl, -OH oder -OCH₃.

In einer anderen bevorzugten Ausführungsform stehen zwei der Reste R₇, R₈, R₉ und R₁₀ für -H und die übrigen beiden Reste sind ungleich -H.

Bevorzugt steht R₁₁ für -H, -C₁₋₅-Aliphat, -C₃₋₈-Cycloaliphat, -Aryl, -Heteroaryl, -C₁₋₈-Aliphat-Aryl, -C₁₋₈-Aliphat-C₃₋₈-Cycloaliphat, -C₁₋₆-Aliphat-Heteroaryl, -C(=O)Aryl, -C(=O)Heteroaryl, oder -C(=O)C₁₋₆-Aliphat.

Bevorzugt steht R₁₂ für -H, -C₁₋₅-Aliphat, -C₃₋₈-Cycloaliphat, -Aryl- oder -Heteroaryl, oder jeweils über -C₁₋₃-Aliphat gebundenes -Aryl, -C₃₋₈-Cycloaliphat oder -Heteroaryl, oder für -OR₁₃ oder -NR₁₄R₁₅. Besonders bevorzugt ist R₁₂ über -C₂-Aliphat, vorzugsweise über -CH₂CH₂- oder -CH=CH-, gebundenes -Aryl, vorzugsweise Phenyl.

Bevorzugt steht R₁₃ für -H, -C₁₋₅-Aliphat, -C₃₋₈-Cycloaliphat, -Aryl oder -Heteroaryl; oder jeweils über -C₁₋₃-Aliphat gebundenes -Aryl, -C₃₋₈-Cycloaliphat oder -Heteroaryl.

Bevorzugt stehen R₁₄ und R₁₅ unabhängig voneinander für -H, -C₁₋₅-Aliphat, -C₃₋₈-Cycloaliphat, -Aryl oder -Heteroaryl; oder jeweils über -C₁₋₃-Aliphat gebundenes -Aryl, -C₃₋₈-Cycloaliphat oder -Heteroaryl; oder R₁₄ und R₁₅ bilden zusammen -CH₂CH₂OCH₂CH₂-, -CH₂CH₂N-R₁₆CH₂CH₂- oder -(CH₂)₃₋₆-.

Bevorzugt steht R₁₆ für -H oder -C₁₋₅-Aliphat.

Bevorzugt steht R₁₇ für -H, -R₀, (bevorzugt -C₁₋₈-Aliphat), -COR₁₂ oder -SO₂R₁₂. In einer bevorzugten Ausführungsform steht R₁₇ für -H oder -COR₀, bevorzugter für -H oder -CO-C₁₋₈-Aliphat-Aryl, besonders bevorzugt für -H oder -CO-CH=CH-Aryl.

Bevorzugt stehen R₁₈ und R₁₉ jeweils unabhängig voneinander für -H, -C₁₋₅-Aliphat, -C₃₋₈-Cycloaliphat, -Aryl oder -Heteroaryl; oder jeweils über -C₁₋₃-Aliphat gebundenes -Aryl, -C₃₋₈-Cycloaliphat oder -Heteroaryl. Besonders bevorzugt sind R₁₈ und R₁₉ -H.

Zum Zwecke der Beschreibung werden Kohlenwasserstoffreste unterteilt in aliphatische Kohlenwasserstoffreste einerseits und aromatische Kohlenwasserstoffreste andererseits.

Aliphatische Kohlenwasserstoffreste werden ihrerseits unterteilt in nicht-cyclische aliphatische Kohlenwasserstoffreste einerseits (= "Aliphat") und cyclische aliphatische Kohlenwasserstoffreste, d.h. alicyclische Kohlenwasserstoffreste, andererseits (= "Cycloaliphat"). Cycloaliphaten können monocyclisch oder multicyclisch sein. Alicyclische Kohlenwasserstoffreste ("Cycloaliphat") umfassen sowohl reine aliphatische Carbocyclen als auch aliphatische Heterocyclen, d.h. - sofern nicht ausdrücklich spezifiziert - umfasst "Cycloaliphat" reine aliphatische Carbocyclen (z.B. Cyclohexyl), reine aliphatische Heterocyclen (z.B. Piperidyl oder Piperazyl) sowie nicht-aromatische, multicyclische, ggf. gemischte Systeme (z.B. Decalinyl, Decahydrochinolinyl).

Aromatische Kohlenwasserstoffe werden ihrerseits unterteilt in carbocyclische aromatische Kohlenwasserstoffe einerseits (= "Aryl") und heterocyclische aromatische Kohlenwasserstoffe andererseits (= "Heteroaryl").

Die Zuordnung von multicyclischen, wenigstens teilaromatischen Systemen richtet sich vorzugsweise danach, ob wenigstens ein aromatischer Ring des multicyclischen Systems wenigstens ein Heteroatom (üblicherweise N, O oder S) im Ring aufweist. Ist wenigstens ein solches Heteroatom in diesem Ring vorhanden, handelt es sich bevorzugt um ein "Heteroaryl" (selbst wenn ggf. als zusätzlich vorhandener Cyclus des multicyclischen Systems ein weiterer carbocyclischer aromatischer oder nicht-aromatischer Ring mit oder ohne Heteroatom vorhanden ist); ist in keinem der ggf. mehreren aromatischen Ringe des multicyclischen Systems ein solches Heteroatom vorhanden, handelt es sich bevorzugt um "Aryl" (selbst wenn in einem ggf. zusätzlich vorhandenen, nicht-aromatischen Cyclus des multicyclischen Systems ein Ringheteroatom vorhanden ist).

Innerhalb der cyclischen Substituenten gilt demnach bevorzugt folgende Priorität bei der Zuordnung: Heteroaryl > Aryl > Cycloaliphat.

Zum Zwecke der Beschreibung werden einbindige und mehrbindige, z.B. zweibindige Kohlenwasserstoffreste nicht begrifflich unterschieden, d.h. "C₁₋₃-Aliphat" umfasst, je nach Sinnzusammenhang, z.B. sowohl -C₁₋₃-Alkyl, -C₁₋₃-Alkenyl und -C₁₋₃-Alkinyl, als auch z.B. -C₁₋₃-Alkylen-, -C₁₋₃-Alkenylen- und C₁₋₃-Alkinylen.

Bevorzugt ist Aliphat jeweils ein verzweigter oder unverzweigter, gesättigter oder ein ein- oder mehrfach ungesättigter, unsubstituierter oder ein- oder mehrfach substituierter, aliphatischer Kohlenwasserstoffrest. Soweit Aliphat ein- oder mehrfach substituiert ist, sind die Substituenten unabhängig voneinander ausgewählt aus der Gruppe bestehend ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=0)N(R₀)₂, -SH, -SR₀, -SO₃H, -S(=O)₁₋₂-R₀. -S(=O)₁₋₂NH₂, -NH₂, -NHR₀, -N(R₀)₂, -N⁺(R₀)₃, -N⁺(R₀)₂O . -NHC(=O)R₀, -NHC(=O)OR₀. -NHC(=O)NH₂, -NHC(=O)NHR₀, -NHC(=O)N(R₀)₂, -Si(R₀)₃, -PO(OR₀)₂. So umfasst "Aliphat" acyclische gesättigte oder ungesättigte Kohlenwasserstoffreste, die verzweigt oder geradkettig sein können, d.h. Alkanyle, Alkenyle und Alkinyle. Dabei weisen Alkenyle mindestens eine C=C-Doppelbindung und Alkinyle mindestens eine C≡C-Dreifachbindung auf. Bevorzugte unsubstituierte einbindige Aliphaten umfassen -CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂-CH₂CH₃ und -CH₂CH₂CH₂CH₂CH₂CH₃; aber auch -CH=CH₂, -C≡CH, -CH₂CH=CH₂, -CH=CHCH₃, -CH₂C≡CH, -C≡CCH₃ und -CH=CHCH=CH₂. Bevorzugte unsubstituierte zweibindige Aliphaten umfassen -CH₂-, -CH₂CH₂-, -CH₂CH(CH₃)-, -CH(CH₃)-CH₂-, -CH₂CH₂CH₂-, -CH(CH₃)CH₂CH₂-, -CH₂CH(CH₃)-CH₂-, -CH₂CH₂CH(CH₃)-, -CH-(CH₂CH₃)CH₂- und -CH₂CH₂-CH₂CH₂-; aber auch -CH=CH-, -C≡C-, -CH₂CH=CH-, -CH=CHCH₂-, -CH₂C≡C- und -C≡CCH₂-. Bevorzugte substituierte einbindige Aliphaten umfassen -CH₂F, -CHF₂, -CF₃, -CH₂CF₃, -CF₂CF₃, -CH₂OH, -CH₂CH₂OH, -CH₂CHOHCH₃, -CH₂OCH₃, -CH₂CH₂OCH₃ und -CH₂N(CH₃)₂. Bevorzugte substituierte zweibindige Aliphaten umfassen -CF₂-, -CF₂CF₂-, -CH₂CHOH-, -CHOHCH₂- und -CH₂CHOHCH₂-, Besonders bevorzugt sind -Methyl-, -Ethyl-, -n-Propyl- und -n-Butyl-.

Bevorzugt ist Cycloaliphat jeweils ein gesättigter oder ein ein- oder mehrfach ungesättigter, unsubstituierter oder ein- oder mehrfach substituierter, aliphatischer (d.h. nicht aromatischer), mono- oder multicyclischer Kohlenwasserstoffrest. Die Zahl der Ringkohlenstoffatome liegt vorzugsweise im angegebenen Bereich (d.h. ein "C₃₋₈-"Cycloaliphat weist vorzugsweise 3, 4, 5, 6, 7 oder 8 Ringkohlenstoffatome auf). Zum Zwecke der Beschreibung ist "C₃₋₈-Cycloaliphat" bevorzugt ein cyclischer Kohlenwasserstoff mit 3, 4, 5, 6, 7 oder 8 Ringkohlenstoffatomen, gesättigt oder ungesättigt, aber nicht aromatisch, wobei ggf. ein oder zwei Kohlenstoffatome unabhängig voneinander durch ein Heteroatom S, N oder O ersetzt sind. Soweit Cycloalkyl ein- oder mehrfach substituiert ist, sind die Substituenten unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)N(R₀)_{2,} -OH, -OR₀, -OC(=O)H, -OC(=O)R₀, -OC(=O)OR₀, -OC(=O)NHR₀ -OCX=OFN(R₀)₂, -SH, -SR₀, -SO₃H, -S(=O)₁₋₂-R₀-S(=O)₁-₂NH₂, -NH₂, -NHR₀, -N(R₀)₂ -N⁺(R₀)₃ -N⁺(R₀)₂O⁻, -NHC(=O)R₀, -NHC(=O)OR₀, -NHC(=O)NH₂, -NHC(=O)NHR₀, -NHC(=O)N(R₀)₂, -Si(R₀)₃, -PO(OR₀)₂. Vorteilhaft ist C₃₋₈-Cycloaliphat aus der Gruppe ausgewählt bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl.

Bevorzugt wird im Zusammenhang mit "Aliphat" bzw. "Cycloaliphat" unter "ein- oder mehrfach substituiert" die ein- oder mehrfache Substitution, z.B. einfache, zweifache, dreifache oder vierfache Substitution, eines oder mehrerer Wasserstoffatome durch -F, -Cl, -Br, -I, -OH, -OC₁₋₆-Alky). -OC(=O)C₁₋₆-Alkyl, -SH, -NH₂, -NHC₁₋₆-Alkyl, -N(C₁₋₆-Alkyl)₂, -C(=O)OC₁₋₆-Alkyl oder -C(=O)OH verstanden. Besonders bevorzugte Substituenten sind -F, -Cl, -OH, -SH, -NH₂ und -C(=O)OH.

Unter mehrfach substituierten Resten sind solche Reste zu verstehen, die entweder an verschiedenen oder an gleichen Atomen mehrfach, z. B. zwei- oder dreifach, substituiert sind, beispielsweise dreifach am gleichen C-Atom, wie im Falle von -CF₃ oder -CH₂CF₃, oder an verschiedenen Stellen, wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfasst -OAliphat u.a. auch -OCH₂CH₂O-CH₂CH₂OH. Bevorzugt ist es, wenn Aliphat oder Cycloaliphat substituiert sind mit -F, -Cl, -Br, -I, -CN, -CH₃, -C₂H₅, -NH₂, -NO₂, -SH, -CF₃, -OH, -OCH₃, -OC₂H₅ oder -N(CH₃)₂. Ganz besonders bevorzugt ist es, wenn Aliphat oder Cycloaliphat substituiert sind mit -OH, -OCH₃ oder -OC₂H₅.

Bevorzugt steht Aryl jeweils unabhängig für ein carbocyclisches Ringsystem mit mindestens einem aromatischen Ring, aber ohne Heteroatome in diesem Ring, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können. Bevorzugte Aryle sind Phenyl, Naphthyl, Anthracenyl, Phenanthrenyl, Fluoranthenyl, Fluorenyl, Indanyl und Tetralinyl. Besonders bevorzugt sind Phenyl und Naphthyl. Soweit Aryl ein- oder mehrfach substituiert ist, können die Aryl-Substituenten gleich oder verschieden und in jeder beliebigen und möglichen Position des Aryls sein, und sind unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)NH₂, -C(=O)NHR_{O}, -C(=O)N(R₀)₂, -OH, -O(CH₂)₁₋₂O-, -OR₀, -OC(=O)H, -OC(=O)R₀ , -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=ONH₂, -NHC(=O)NHR₀, -NHC(=O)N(R₀)₂, -Si(R₀)₃, -PO(OR₀)₂. Bevorzugte substituierte Aryle sind 2-Fluor-Phenyl, 3-Fluor-Phenyl, 4-Fluor-Phenyl, 2,3-Difluor-Phenyl, 2,4-Difluor-Phenyl, 3,4-Difluor-Phenyl, 2-Chlor-Phenyl, 3-Chlor-Phenyl, 4-Chlor-Phenyl, 2,3-Dichlor-Phenyl, 2,4-Dichlor-Phenyl, 3,4-Dichlor-Phenyl, 2-Methoxy-Phenyl, 3-Methoxy-Phenyl, 4-Methoxy-Phenyl, 2,3-Dimethoxy-Phenyl, 2,4-Dimethoxy-Phenyl, 3,4-Dimethoxy-Phenyl, 2-Methyl-Phenyl, 3-Methyl-Phenyl, 4-Methyl-Phenyl, 2,3-Dimethyl-Phenyl, 2,4-Dimethyl-Phenyl und 3,4-Dimethyl-Phenyl.

Bevorzugt steht Heteroaryl für einen 5-, 6- oder 7-gliedrigen cyclischen aromatischen Rest, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann. Bevorzugt ist "Heteroaryl" ausgewählt aus der Gruppe bestehend aus Pyrrolyl, Indolyl, Furyl (Furanyl), Benzofuranyl, Thienyl (Thiophenyl), Benzothienyl, Benzothiadiazolyl, Benzooxadiazolyl, Benzothiazolyl, Benzooxazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl, wobei die Bindung über jedes beliebige und mögliche Ringglied des Heteroaryl-Restes erfolgen kann. Soweit Heteroaryl ein- oder mehrfach substituiert ist, können die Heteroaryl-Substituenten gleich oder verschieden und in jeder beliebigen und möglichen Position des Heteroaryls sein, und sind unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)-NH₂, -C(=O)NHR₀, -C(=O)N(R₀)₂, -OH, -0(CH₂)₁-₂0-, -OR₀, -OC(=O)H, -OC(=O)R₀, -OC(=0)ORo, -OC(=O)NHR₀, -OC(=O)N(R_{O})₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NH-C(=O)R₀, -NHC(=O)OR₀, -NHC(=O)NH₂, -NHC(=O)NHR₀, -NHC(=O)N(R₀)₂, -Si(R₀)₃, -PO(OR₀)₂: wobei ggf. vorhandene N-Ringatome jeweils oxidiert sein können (N-Oxid).

In Bezug auf "Aryl" oder "Heteroaryl" versteht man unter "ein- oder mehrfach substituiert" die ein- oder mehrfache, z.B. zwei-, drei- vier- oder fünffache, Substitution eines oder mehrerer Wasserstoffatome des Ringsystems.

Besonders bevorzugt sind die (Hetero-)Aryl-Substituenten unabhängig voneinander ausgewählt aus -F, -Cl, -Br, -I, -CN, -CHO, -CO₂H, -NH₂, -NO₂, -NHR₀, -N(R₀)₂, -N⁺(R₀)₃, -N⁺(R₀)₂O⁻, -SH, -SR₀, -OH, -OR₀, -C(=O)R₀, -CO₂R₀, -C(=O)NH₂, -C(=O)NHR₀, -C(=O)N(R₀)₂, -S(=O)₁₋₂R₀, -S(=O)₂NH₂, -SO₃H, =O oder -R₀. Bevorzugte Substituenten sind -F, -Cl, -Br, -I, -OH, -OC₁₋₆-Alkyl, -O-C(=O)-C₁₋₆-Alkyl, -SH, -NH₂, -NHC₁₋₈-Alkyl, -N(C₁₋₆-Alkyl)₂. -C(=O)OC₁₋₆-Alkyl oder -C(=O)OH. Besonders bevorzugte Substituenten sind -F, -Cl, -OH, -SH, -NH₂ und -C(=O)OH.

Die erfindungsgemäßen Verbindungen können in Form eines einzelnen Stereoisomers oder deren Mischung, der freien Verbindungen und/oder ihrer physiologisch verträglichen Salze und/oder Solvate vorliegen.

Die erfindungsgemäßen Verbindungen können je nach Substitutionsmuster chiral oder achiral sein.

Bei den erfindungsgemäßen Verbindungen handelt es sich in Bezug auf das spiro-Ringsystem um Isomere, bei denen das Substitutionsmuster am spiro-Cyclohexanringsystem auch mit cis/trans, Z/E oder syn/anti bezeichnet werden kann. "Cis-trans-Isomere" sind eine Untergruppe der Stereoisomere (Konfigurationsisomere).

In einer bevorzugten Ausführungsform beträgt der Diastereomerenüberschuss des cis-Isomers wenigstens 50 %de, bevorzugter wenigstens 75 %de, noch bevorzugter wenigstens 90%de, am bevorzugtesten wenigstens 95%de und insbesondere wenigstens 99%de. In einer anderen bevorzugten Ausführungsform beträgt der Diastereomerenüberschuss des trans-Isomers wenigstens 50 %de, bevorzugter wenigstens 75 %de, noch bevorzugter wenigstens 90%de, am bevorzugtesten wenigstens 95%de und insbesondere wenigstens 99%de.

Geeignete Methoden zur Trennung der Isomere (Diastereomere) sind dem Fachmann bekannt. Als Beispiele können Säulenchromatographie, präparative HPLC und Kristallisationsverfahren genannt werden.

Sind die erfindungsgemäßen Verbindungen chiral, so liegen sie vorzugsweise als Racemat oder in angereicherter Form eines Enantiomers vor. In einer bevorzugten Ausführungsform beträgt der Enantiomerenüberschuss (ee) des S-Enantiomers wenigstens 50%ee, bevorzugter wenigstens 75%ee, noch bevorzugter wenigstens 90%ee, am bevorzugtesten wenigstens 95%ee und insbesondere wenigstens 99%ee. In einer anderen bevorzugten Ausführungsform beträgt der Enantiomerenüberschuss (ee) des R-Enantiomers wenigstens 50%ee, bevorzugter wenigstens 75%ee, noch bevorzugter wenigstens 90%ee, am bevorzugtesten wenigstens 95%ee und insbesondere wenigstens 99%ee.

Geeignete Methoden zur Trennung der Enantiomere sind dem Fachmann bekannt. Als Beispiele können präparative HPLC an chiralen stationären Phasen und Überführung in diastereomere Intermediate genannt werden. Die Überführung in diastereomere Intermediate kann beispielsweise als Salzbildung mit Hilfe chiraler, enantiomerenreiner Säuren erfolgen. Nach der Trennung der so gebildeten Diastereomere kann das Salz dann wieder in die freie Base oder ein anderes Salz überführt werden.

Sofern nicht ausdrücklich spezifiziert umfasst jeder Verweis auf die erfindungsgemäßen Verbindungen alle Isomere (z.B. Stereoisomere, Diastereomere, Enantiomere) in beliebigem Mischungsverhältnis.

Sofern nicht ausdrücklich spezifiziert umfasst jeder Verweis auf die erfindungsgemäßen Verbindungen die freien Verbindungen (d.h. die Formen, welche nicht als Salz vorliegen) und alle physiologisch verträglichen Salze.

Zum Zwecke der Beschreibung liegen physiologisch verträgliche Salze der erfindungsgemäßen Verbindungen vor als Salze mit Anionen oder Säuren der jeweiligen Verbindung mit anorganischen bzw. organischen Säuren, die physiologisch - insbesondere bei Anwendung im Menschen und/oder Säugetier - verträglich sind.

Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, α-Liponsäure, Acetylglycin, Acetylsalicylsäure, Hippursäure und/oder Asparaginsäure. Besonders bevorzugt sind das Hydrochlorid, das Citrat und das Hemicitrat.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calcium-Salze.

Die erfindungsgemäßen Verbindungen sind durch Substituenten definiert, beispielsweise durch R₁, R₂ und R₃ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Generation). Ist beispielsweise Y₁ = -R₀ wobei R₀ = -C₁-₈-Aliphat (Substituent der 1. Generation), so kann -C₁₋₈-Aliphat seinerseits substituiert sein, z.B. mit -OR₀ wobei R₀ = -Aryl (Substituent der 2. Generation). Es ergibt sich daraus die funktionelle Gruppe -C₁₋₈-Aliphat-OAryl. -Aryl kann dann seinerseits erneut substituiert sein, z.B. mit -Cl (Substituent der 3. Generation). Es ergibt sich daraus dann insgesamt die funktionelle Gruppe -C₁₋₈-Aliphat-OAryl-Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform die funktionellen Gruppen für R₀ bis R₁₉ jeweils ggf. substituiert sein, die jeweiligen Substituenten können dann ihrerseits jedoch nicht erneut substituiert sein.

In einer anderen bevorzugten Ausführungsform können bereits die Substituenten der 1. Generation nicht erneut substituiert sein, d.h. es gibt dann weder Substituenten der 2. noch Substituenten der 3. Generation. Mit anderen Worten können in dieser Ausführungsform die funktionellen Gruppen für R₀ bis R₁₉ jeweils nicht substituiert sein.

Bevorzugt sind Verbindungen, wobei "Aliphat substituiert" oder Cycloaliphat, substituiert" Aliphat oder Cycloaliphat substituiert mit -F, -Cl, -Br, -I, -CN, -CH₃, -C₂H₅, -NH₂, -NO₂, -SH, -CF₃, -OH, -OCH₃, -OC₂H₅oder -N(CH₃)₂ bedeutet; und "Aryl substituiert" oder "Heteroaryl substituiert" Aryl oder Heteroaryl substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet, in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Für eine bevorzugte Ausführungsform der erfindungsgemäßen Verbindungen gilt, dass R₁ und R₂ gemeinsam einen Ring bilden und für -CH₂CH₂CH₂- oder -CH₂CH₂CH₂CH₂- stehen. Besonders bevorzugt sind Verbindungen, worin R₁ und R₂ einen Ring bilden und gemeinsam -CH₂CH₂CH₂- bedeuten.

Weiterhin bevorzugt sind auch Verbindungen, worin R₃ für Phenyl, Benzyl oder Phenethyl, jeweils unsubstituiert oder am Ring ein- oder mehrfach substituiert; -C₁₋₅-Alkyl, unsubstituiert oder ein- oder mehrfach substituiert; -C₄₋₈-Cycloalkyl, unsubstituiert oder ein- oder mehrfach substituiert; -Pyridyl, -Thienyl, -Thiazolyl, -Imidazolyl, -1,2,4 Triazolyl oder -Benzimidazolyl, unsubstituiert oder ein- oder mehrfach substituiert, steht.

Besonders bevorzugt sind Verbindungen, worin R₃ -Phenyl, -Benzyl, -Phenethyl, -Thienyl, -Pyridyl, -Thiazolyl, -Imidazolyl, -1,2,4 Triazolyl, -Benzimidazolyl oder -Benzyl, unsubstituiert oder ein- oder mehrfach substituiert mit -F, -Cl, -Br, -CN, -CH₃, -C₂H₅, -NH₂,-NO₂, -SH, -CF₃, -OH, -OCH₃, -OC₂H₅ oder -N(CH₃)₂; -Ethyl, -n-Propyl, -2-Propyl, -Allyl, -n-Butyl, -iso-Butyl, -sec.-Butyl, -tert.-Butyl, -n-Pentyl, -iso-Pentyl, -neo-Pentyl, -n-Hexyl, -Cyclopentyl oder -Cyclohexyl steht, jeweils unsubstituiert oder ein- oder mehrfach substituiert mit -OH, -OCH₃ oder -OC₂H₅, wobei -Thienyl, -Pyridyl, -Thiazolyl, -Imidazolyl, -1,2,4 Triazolyl und -Benzimidazolyl vorzugsweise unsubstituiert sind; insbesondere -Phenyl, unsubstituiert oder einfach substituiert mit -F, -CI, -CN, -CH₃; -Thienyl; -Ethyl, -n-Propyl oder -n-Butyl, unsubstituiert oder ein- oder mehrfach substituiert mit -OCH₃, -OH oder -OC₂H₅, insbesondere mit -OCH₃.

Für eine bevorzugte Ausführungsform der erfindungsgemäßen Verbindungen gilt, dass R₅ für -H, -CH₃, -COOH, -COOCH₃, -CH₂O-Phenyl, wobei der Phenylrest mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ substituiert sein kann, oder -CH₂OH steht. Besonders bevorzugt sind Verbindungen, worin R₅ für H steht.

Bevorzugt sind auch Verbindungen, worin R₆ -H; -Methyl, -Ethyl, -CF₃, -Benzyl oder -Phenyl bedeuten kann, wobei der Benzyl- oder Phenylrest mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ substituiert sein kann. Besonders bevorzugt sind spirocyclische Cyclohexanderivate, worin R₆ H bedeutet.

Bevorzugt sind weiterhin Verbindungen, worin R₇, R₈, R₉ und R₁₀ unabhängig voneinander -H; -C₁₋₅-Alkyl, verzweigt oder unverzweigt, unsubstituiert oder ein- oder mehrfach substituiert; -F, -Cl, -Br, -I, -CF₃, -OH, -OCH₃, -NH₂, -COOH, -COOCH₃, -NHCH₃, -Thienyl, -Pyrimidinyl, -Pyridyl, -N(CH₃)₂ oder -NO₂ bedeuten; vorzugsweise einer der Reste R₇, R₈, R₉ und R₁₀ für -H; -C₁₋₅-Alkyl, verzweigt oder unverzweigt, unsubstituiert oder ein- oder mehrfach substituiert; -F, -Cl, -Br, -I, -OH, -OCH₃, -COOH, -COOCH₃, -NH₂, -NHCH₃ oder -N(CH₃)₂ oder -NO₂ steht, während die übrigen Reste -H sind; oder zwei der Reste R₇, R₈, R₉ und R₁₀ unabhängig voneinander für -H; -C₁₋₅-Alkyl, verzweigt oder unverzweigt, unsubstituiert oder ein- oder mehrfach substituiert; -F, -Cl, -Br, -I, -OH, -OCH₃, -COOH, -COOCH₃, -NH₂, -NHCH₃ oder -N(CH₃)₂ oder -NO₂ stehen, während die übrigen Reste -H sind. Besonders bevorzugt sind spirocyclische Cyclohexanderivate, worin R₇, R₈, R₉ und R₁₀ unabhängig voneinander für -H, -F, -OH, -Cl oder -OCH₃ stehen.

Verbindungen, bei denen X für -O steht, sind besonders bevorzugt. Weiterhin besonders bevorzugt sind Verbindungen, bei denen X für -NR₁₇- steht.

Bevorzugte Ausführungsformen der erfindungsgemäßen Verbindungen der allgemeinen Formeln (1.2.1), (1.2.4), (1.3.1), (1.3.4), (1.4.1), (1.4.4), (1.5.1), (1.5.4), (1.6.1), (1.6.4), (1.7.1) und (1.7.4) haben die allgemeine Formel (1.2.1.1), (1.2.1.2), (1.2.4.1), (1.2.4.2), (1.3.1.1), (1.3.1.2), (1.3.4.1), (1.3.4.2), (1.4.1.1), (1.4.1.2), (1.4.4.1), (1.4.4.2), (1.5.1.1), (1.5.1.2), (1.5.4.1), (1.5.4.2), (1.6.1.1), (1.6.1.2), (1.6.4.1), (1.6.4.2), (1.7.1.1), (1.7.1.2), (1.7.4.1) und (1.7.4.2): 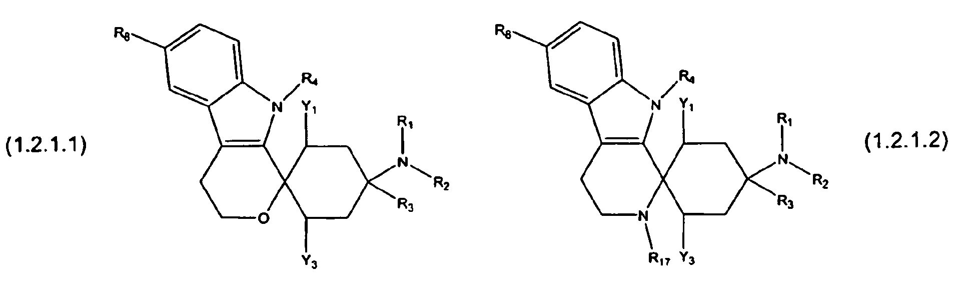 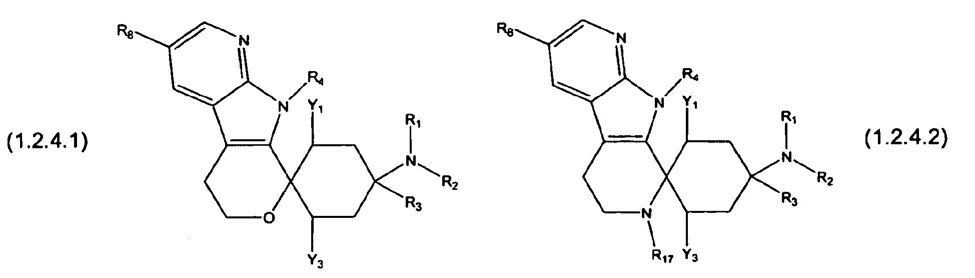 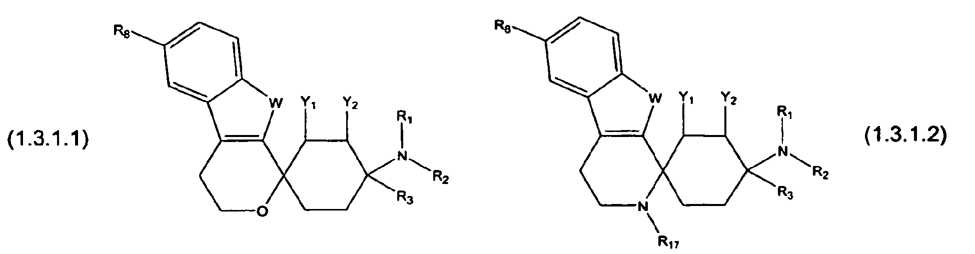 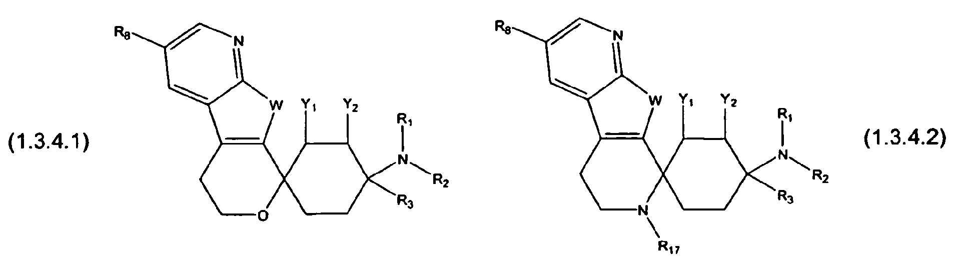 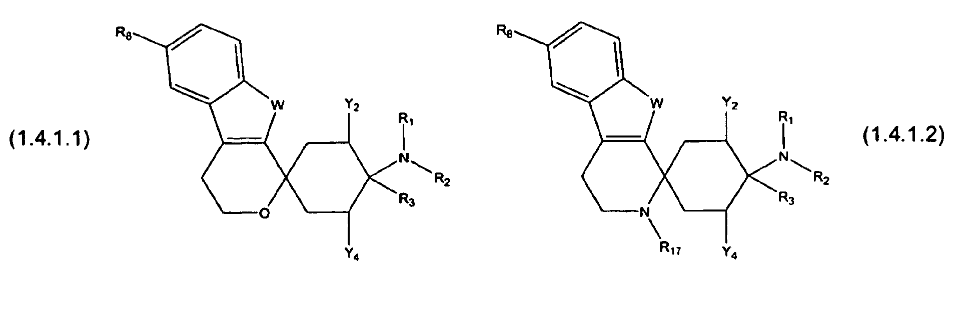 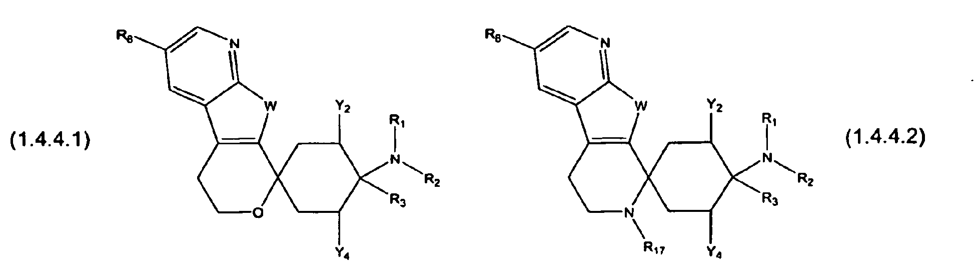 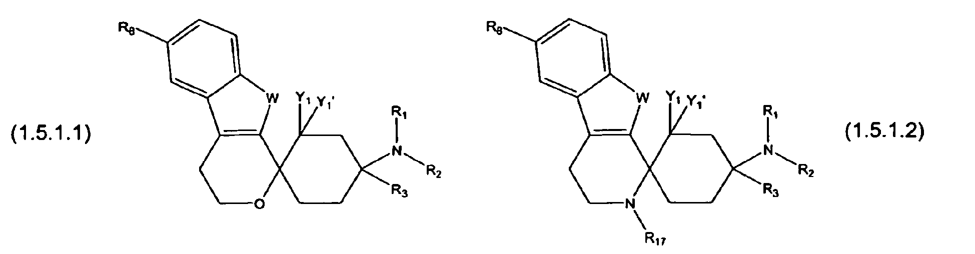 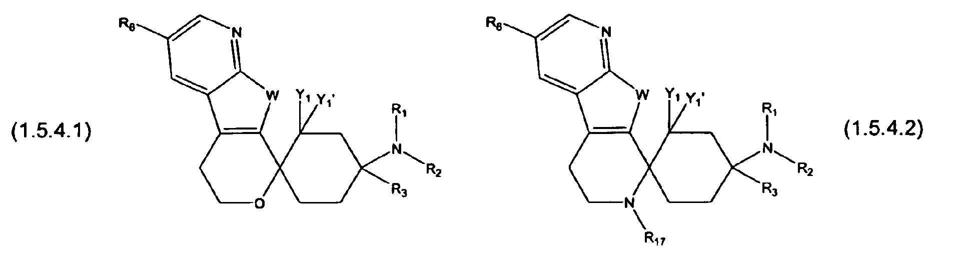 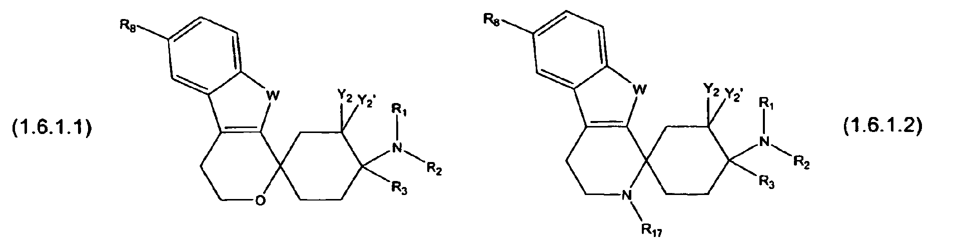 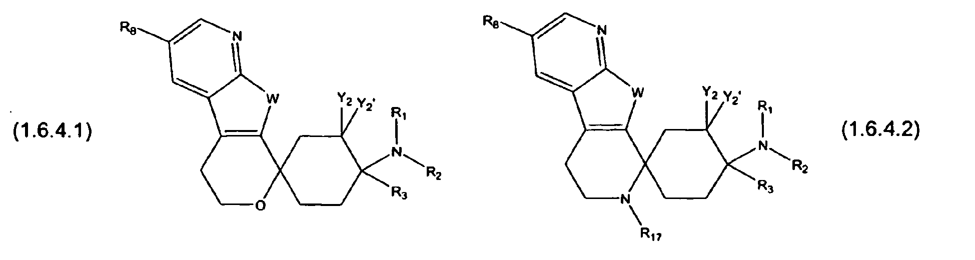 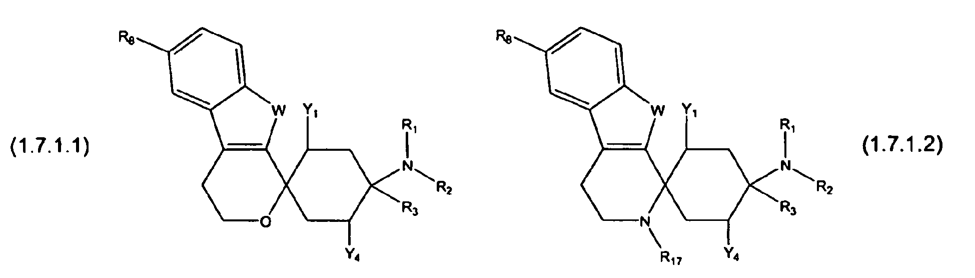 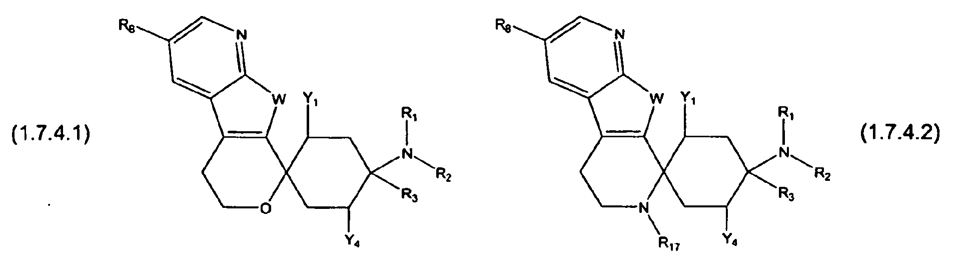 worin vorzugsweise
R₁ für -CH₃ steht;
R₂ für -H oder -CH₃ steht;
R₃ für -Phenyl steht, unsubstituiert oder ein- oder mehrfach substituiert mit -F, -Cl, -CN oder -CH₃;
R₄ für -H oder -COR₁₂ steht;
R₈ für -H oder -F steht;
R₁₇ für -H oder -COR₁₂ steht; und
Y₁, Y₁', Y₂, Y₂', Y₃ und Y₄, soweit vorhanden, jeweils unabhängig voneinander ausgewählt sind aus der Gruppe bestehend aus -H, -F, -Cl, -Br, -I, -CN, -C₁₋₈-Aliphat, -C₁₋₈-Aliphat-NHC₁₋₆-Aliphat, -C₁₋₈-Aliphat-N(C₁₋₈-Aliphath, -C₃₋₈-Cycloaliphat, -C₁₋₆-Aliphat-C₃₋₈-Cycloaliphat, -C₁₋₆-Aliphat-Aryl, -C₁₋₆-Aliphat-Heteroaryl, -S-C₁₋₈-Aliphat, -S-Aryl, -Aryl und -Heteroaryl; mit der Maßgabe, dass wenigstens einer der Reste Y₁, Y₁', Y₂, Y₂', Y₃ und Y₄ ungleich -H ist.

Ganz besonders bevorzugt sind Verbindungen aus der Gruppe:
- (±)-N,N,2-Trimethyl-4-phenyl-4',9'-dihydro-3'H-spiro[cyclohexan-1,1'-pyrano[3,4-blindol)-4-amin; 2-hydroxypropan-1,2,3-tricarboxylat;
- (±)-N,N,2-Trimethyl-4-phenyl-4',9'-dihydro-3'H-spiro[cyclohexan-1,1'-pyrano[3.4-b]indo)]-4-amin; 2-hydroxypropan-1,2,3-tricarboxylat;
- (±)-2-Methyl-4-(dimethylamino)1-4-phenyl-spiro[cyclohexan-1,8'-(5,6,8,9-tetrahydro-pyrano[3,4-b]-7-aza-indol)]; 2-hydroxypropan-1,2,3-tricarboxylat;
- (±)-2-Benzyl-N,N-dimethyl-4-phenyl-4',9'-dihydro-3'H-spiro[cyclohexane-1,1'-pyrano[3,4-b]indol]-4-amin;
- (±)-2-Benzyl-N,N-dimethyl-4-phenyl-2',3',4',9'-tetrahydrospiro[cyclohexan-1,1'-pyrido[3,4-b]indol]-4-amin; 2-hydroxypropan-1,2,3-tricarboxylat;
- (±)-2-(3-Fluorbenzyl)-N,N-dimethy)-4-pheny)-4',9'-dihydro-3'H-spiro[cyclohexan-1,1'-pyrano[3,4-b]indol]-4-amin;
- N,N,3,5-Tetramethyl-4-phenyl-4',9'-dihydro-3'H-spiro[cyclohexan-1,1'-pyrano[3,4-b]indol]-4-amin;
- N,N,2,6-Tetramethyl-4-phenyl-4',9'-dihydro-3'H-spiro[cyclohexan-1,1'-pyrano[3,4-b]indol]-4-amin;
- N,N,2,5-Tetramethyl-4-phenyl-4',9'-dihydro-3'H-spiro[cyclohexan-1,1'-pyrano[3,4-b]indol]-4-amin;
- 2-Benzyl-N,N-dimethyl-4-phenyl-4',9'-dihydro-3'H-spiro[cyclohexan-1,1'-pyrano[3,4-b]indol]-4-amin;
- 2-(4-Fluorobenzyl)-N,N-dimethyl-4-phenyl-4',9'-dihydro-3'H-spiro[cyclohexan-1,1'-pyrano[3,4-b]indol]-4-amin;
- N,N,2,3-Tetramethyl-4-phenyl-4',9'-dihydro-3'H-spiro[cyclohexan-1,1'-pyrano[3,4-b]indol]-4-amin;
- N,N-Dimethyt-3,4-diphenyt-4',9'-dihydro-3'H-spiro[cyc)ohexan-1,1'-pyrano[3,4-b]indol]-4-amin;
- 2-(4-Fluorophenyl)-N,N-dimethyl-4-phenyl-4',9'-dihydro-3'H-spiro[cyclohexan-1,1'-pyrano[3,4-b]indol]-4-amin;
- 3-((Dimethylamino)methyl)-N,N-dimethyl-4-phenyl-4',9'-dihydro-3'H-spiro[cyclohexan-1,1'-pyrano[3,4-b]indol]-4-amin;
- N,N,3-Trimethyl-4-phenyl-4',9'-dihydro-3'H-spiro[cyclohexan-1,1'-pyrano[3,4-b]indol]-4-amin;
- 3-Fluoro-N,N-dimethyl-4-phenyl-4',9'-dihydro-3'H-spiro[cyclohexan-1,1'-pyrano[3,4-b]indol]-4-amin
- 2-Fluoro-N,N-dimethyl-4-phenyl-4',9'-dihydro-3'H-spiro[cyclohexan-1,1'-pyrano[3,4-b]indol]-4-amin;
- N,N-Dimethyl-2-(methylthio)-4-phenyl-4',9'-dihydro-3'H-spiro[cyclohexan-1,1'-pyrano[3,4-b]indol]-4-amin;
- N,N-Dimethyl-4-phenyl-2-(phenylthio)-4',9'-dihydro-3'H-spiro[cyclohexan-1,1'-pyrano[3,4-b]indol]-4-amin;
- 3,3-Difluoro-N,N-dimethyl-4-phenyl-4',9'-dihydro-3'H-spiro[cyclohexan-1,1'-pyrano[3,4-b]indol]-4-amin;
- 2,2-Difluoro-N,N-dimethyl-4-phenyl-4',9'-dihydro-3'H-spiro[cyclohexan-1,1'-pyrano[3,4-b]indol]-4-amin;
- 2-Allyl-N,N-dimethyl-4-phenyl-4',9'-dihydro-3'H-spiro[cyclohexan-1,1'-pyrano[3,4-b]indol]-4-amin;
- N,N,3,5-Tetramethyl-4-phenyl-2',3',4',9'-tetrahydrospiro[cyclohexan-1,1'-pyrido[3,4-b]indol]-4-amin;
- N,N,2,6-Tetramethyl-4-phenyl-2',3',4',9'-tetrahydrospiro[cyclohexan-1,1'-pyrido[3,4-b]indol]-4-amin;
- N,N,2,5-Tetramethyl-4-phenyl-2',3',4',9'-tetrahydrospiro[cyclohexan-1,1'-pyrido[3,4-b]indol]-4-amin;
- 2-Benzyl-N,N-dimethyl-4-phenyl-2',3',4',9'-tetrahydrospiro[cyclohexan-1,1'-pyrido[3,4-b]indol]-4-amin;
- 2-(4-Fluorobenzyl)-N,N-dimethyl-4-phenyl-2',3',4',9'-tetrahydrospiro[cydohexan-1,1'-pyrido(3,4-b]indol]-4-amin;
- N,N,2,3-Tetramethyl-4-phenyl-2',3',4',9'-tetrahydrospiro[cyclohexan-1,1'-pyrido[3,4-b]indol]-4-amin;
- N,N-Dimethyl-3,4-diphenyl-2',3',4',9'-tetrahydrospiro[cyclohexan-1,1'-pyrido[3,4-b)indol]-4-amin;
- 2-(4-Fluorophenyl)-N,N-dimethyl-4-phenyl-2',3',4',9'-tetrahydrospiro[cyclohexan-1,1'-pyrido[3,4-b]indol]-4-amin;
- 3-((Dimethylamino)methyl)-N,N-dimethyl-4-phenyl-2',3',4',9'-tetrahydrospiro[cyclohexan-1,1'-pyrido[3,4-b]indol]-4-amin;
- N,N,3-Trimethyl-4-phenyl-2',3',4',9'-tetrahydrospiro[cyclohexan-1,1'-pyrido[3,4-b]indol]-4-amin;
- 3-Fluoro-N,N-dimethyl-4-phenyl-2',3',4',9'-tetrahydrospiro[cyclohexan-1,1'-pyrido[3,4-b]indol]-4-amin;
- 2-Fluoro-N,N-dimethyl-4-phenyl-2',3',4',9'-tetrahydrospiro[cyclohexan-1,1'-pyrido[3,4-b]indol]-4-amin;
- N,N-Dimethyl-2-(methylthio)-4-phenyl-2',3',4',9'-tetrahydrospiro[cyclohexan-1,1'-pyrido[3,4-b]indol]-4-amin;
- N,N-Dimethyl-4-phenyl-2-(phenylthio)-2',3',4',9'-tetrahydrospiro[cyclohexan-1,1'-pyrido[3,4-b]indol)-4-amin;
- 3,3-Difluoro-N,N-dimethyl-4-phenyl-2',3',4',9'-tetrahydrospiro[cyclohexan-1,1'-pyrido[3,4-b]indol]-4-amin;
- 2,2-Difluoro-N,N-dimethyl-4-phenyl-2',3',4',9'-tetrahydrospiro[cyclohexan-1,1'-pyrido[3,4-b]indol]-4-amin;
- 2-Allyl-N,N-dimethyl-4-phenyl-2',3',4',9'-tetrahydrospiro[cyclohexan-1,1'-pyrido[3,4-b]indol]-4-amin;
- 2-Benzyl-6'-fluor-N,N-dimethyl-4-phenyl-4',9'-dihydro-3'H-spiro[cyclohexan-1,1'-pyrano[3,4-b]indol]-4-amin;
- 6'-Fluor-2-(3-fluorbenzyl)-N,N-dimethyl-4-phenyl-4',9'-dihydro-3'H-spiro[cyclohexan-1,1'-pyrano[3,4-b]indof]-4-amin;
- 2-(3-Fluorbenzyl]-N,N-dimethyl-4-phenyl-2',3',4',9'-tetrahydrospiro[cyclohexan-1,1'-pyrido[3,4-b]indol]-4-amin;
- 3,6'-Difluor-N,N-dimethyl-4-phenyl-4',9'-dihydro-3'H-spiro[cyclohexan-1,1'-pyrano[3,4-b]indol]-4-amin;
- 2,6'-Difluor-N,N-dimethyl-4-phenyl-4',9'-dihydro-3'H-spiro[cyclohexan-1,1'-pyrano[3,4-b)indoll-4-amin;
- 3,3,6'-Trifluoro-N,N-dimethyl-4-phenyl-4',9'-dihydro-3'H-spiro[cyclohexan-1,1'-pyrano[3,4-b]indol]-4-amin;
- 2,2,6'-Trifluoro-N,N-dimethyl-4-phenyl-4',9'-dihydro-3'H-spiro[cyclohexan-1,1'-pyrano[3,4-b]indol]-4-amin;
- 2,6,6'-Trifluoro-N,N-dimethyl-4-phenyl-4',9'-dihydro-3'H-spiro[cyclohexan-1,1'-pyrano[3,4-b]indol]-4-amin;
- 2,2,6'-Trifluoro-N-methyl-4-phenyl-4',9'-dihydro-3'H-spiro[cyclohexan-1,1'-pyrano[3,4-b]indol]-4-amin;
- 2,6'-Dinuoro-N-methyl-4-phenyl-4',9'-dihydro-3'H-spiro[cyclohexan-1,1'-pyrano[3,4-b]indol]-4-amin;
- 2,6,6'-Trifluoro-N-methyl-4-phenyl-4',9'-dihydro-3'H-spiro[cyclohexan-1,1'-pyrano[3,4-b]indol]-4-amin;
- 3,3,6'-Trifluoro-N,N-dimethyl-4-(thiophen-2-yl)-4',9'-dihydro-3'H-spiro[cyclohexan-1,1'-pyrano[3,4-b]indol]-4-amin;
- 4-Butyl-3,3,6'-trifluoro-N,N-dimethyl-4',9'-dihydro-3'H-spiro[cyclohexan-1,1'-pyrano[3,4-b]indol]-4-amin;
- 2,2,6'-Trifluoro-N,N-dimethyl-4-(thiophen-2-yl)-4',9'-dihydro-3'H-spiro[cyclohexan-1,1'-pyrano[3,4-b]indol]-4-amin;
- 4-Butyl-2,2,6'-trifluoro-N,N-dimethyl-4',9'-dihydro-3'H-spiro[cyclohexan-1,1'-pyrano[3,4-b]indol]-4-amin;
- 2,6,6'-Trifluoro-N,N-dimethyl-4-(thiophen-2-yl)-4',9'-dihydro-3'H-spiro[cyclohexan-1,1'-pyrano[3,4-b]indol]-4-amin;
- 4-Butyl-2,6,6'-trifluoro-N,N-dimethyl-4',9'-dihydro-3'H-spiro[cyclohexan-1,1'-pyrano[3,4-b]indol)-4-amin,
- 3,6'-Difluoro-N,N-dimethyl-4-(thiophen-2-yl)-4',9'-dihydro-3'H-spiro[cyclohexan-1,1'-pyrano[3,4-b]indol]-4-amin;
- 4-Butyl-3,6'-difluoro-N,N-dimethyl-4',9'-dihydro-3'H-spiro[cyclohexan-1,1'-pyrano[3,4-b]indol]-4-amin;
- 2,6'-Difluoro-N,N-dimethyl-4-(thiophen-2-yl)-4',9'-dihydro-3'H-spiro[cyclohexan-1,1'-pyrano[3,4-b]indol]-4-amin;
- 4-Butyl-2,6'-difluoro-N,N-dimethyl-4',9'-dihydro-3'H-spiro[cyclohexan-1,1'-pyrano[3,4-b]indol]-4-amin;
- 3,3,6'-Trifluoro-4-(3-fluorophenyl)-N,N-dimethyl-2',3',4',9'-tetrahydrospiro[cyclohexan-1,1'-pyrido[3,4-b]indol]-4-amin;
- 2,2,6'-Trifluoro-4-(3-fluorophenyl)-N,N-dimethyl-2',3',4',9'-tetrahydrospiro[cyclohexan-1,1'-pyrido[3,4-b]indol]-4-amin;
- 3,6'-Difluoro-4-(3-fluorophenyl)-N,N-dimethyl-2',3',4',9'-tetrahydrospiro[cyclohexan-1,1'-pyrido[3,4-b]indol]-4-amin;
- 2,6'-Difluoro-4-(3-fluorophenyl)-N,N-dimethyl-2',3',4',9'-tetrahydrospiro[cyclohexan-1,1'-pyrido[3,4-b]indol]-4-amin;
- 2,6,6'-Trifluoro-4-(3-fluorophenyf)-N,N-dimethyl-2',3',4',9'-tetrahydrospiro[cyclohexan-1,1'-pyrido[3,4-b]indol]-4-amin;
- 3,3-Difluoro-N,N-dimethyl-4-phenyl-2',3',4',9'-tetrahydrospiro[cyclohexan-1,1'-pyrido[3,4-b]indol]-4-amin;
- 2,2-Difluoro-4-(3-fluorophenyl)-N,N-dimethyt-2',3',4',9'-tetrahydrospiro[cyclohexan-1,1'-pyrido[3,4-b]indol]-4-amin;
- 3-Fluoro-4-(3-ftuorophenyl)-N.N-dimethy)-2',3',4',9'-tetrahydrospirb[cyclohexan-1,1'-pyrido[3,4-b]indol]-4-amin;
- 2-Fluoro-4-(3-fluorophenyl)-N,N-dimethyl-2',3',4',9'-tetrahydrospiro[cyclohexan-1,1'-pyrido[3,4-b]indol]-4-amin;
- 2,6-Difluoro-4-(3-fluorophenyl)-N,N-dimethyl-2',3',4',9'-tetrahydrospiro[cyclohexan-1,1'-pyrido[3,4-b]indol]-4-amin;
- (E)-1-(4-(Dimethylamino)-3-fluoro-4-(3-fluorophenyl)-3',4'-dihydrospiro[cyclohexan-1,1'-pyrido[3,4-b]indo)]-2'(9'H)-yl)-3-pheny)prop-2-en-1-on;
- (E)-1-(4-(Dimethylamlno)-2-f)uoro-4-(3-fluorophenyl)-3'.4'-dihydrospiro[cyclohexan-1,1'-pyrido[3,4-b]indol]-2'(9'H)-yl)-3-phenylprop-2-en-1-on;
- (E)-1-(4-(Dimethylamino)-3,3-difluoro-4-(3-fluorophenyl)-3',4'-dihydrospiro[cyclohexan-1,1'-pyrido[3,4-b]indol]-2'(9'H)-yl)-3-phenylprop-2-en-1-on;
- (E)-1-(4-(Dimethylamino)-2,2-difluoro-4-(3-fluorophenyl)-3',4'-dihydrospiro[cyclohexan-1,1'-pyrido[3,4-b]indol]-2'(9'H)-yl)-3-phenylprop-2-en-1-on;
- (E)-1-(4-(Dimethy)amino)-2,6-difluoro-4-(3-fluorophenyl)-3',4'-dihydrospiro[cyclohexan-1,1'-pyrido[3,4-b]indo)]-2'(9'H)-yl)-3-pheny)prop-2-en-1-on;
- (E)-1-(4-(Dimethylamino)-3,6'-difluoro-4-(3-fluorophenyl)-3',4'-dihydrospiro[cyclohexan-1,1'-pyrido[3,4-b]indoll-2'(9'H)-yl)-3-phenylprop-2-en-1-on,
- (E)-1-(4-(Dimethyfamino)-2,6'-difluoro-4-(3-fluorophenyl)-3',4'-dihydrospiro(cyclohexan-1,1'-pyrido[3,4-b]indol]-2'(9'H)-yl)-3-phenylprop-2-en-1-on;
- (E)-1-(4-(Dimethylamino)-3,3,6'-trifluoro-4-(3-fluorophenyl)-3',4'-dihydrospiro[cyclohexan-1,1'-pyrldo[3,4-b]indol]-2'(9'H)-yl)-3-phenylprop-2-en-1-on;
- (E)-1-(4-(Dimethylamino)-2,2,6'-trifluoro-4-(3-fluorophenyl)-3',4'-dihydrospiro[cyclohexan-1,1'-pyrido[3,4-b]indol]-2'(9'H)-yl)-3-phenylprop-2-en-1-on;
- (E)-1-(4-(Dimethylamino)2,6,6'-trifluoro-4-(3-fluorophenyl)-3',4'-dihydrospiro[cyclohexan-1,1'-pyrido[3,4-b]indol]-2'(9'H)-yl)-3-phenylprop-2-en-1-on;
- 2-Methyl-4-(dimethylamino)1-4-phenyl-spiro[cyclohexan-1,8'-(5,6,7,8,9-Pentahydro-pyrido[3,4-b]-7-aza-indol)]:
- N,N,2-Trimethyl-4-phenyl-2',3',4',9'-tetrahydrospiro[cyclohexan-1,1'-pyrido[3,4-b]indol]-4-amin;
- 2-Benzyl-N,N-dimethyl-4-phenyl-2',3',4',9'-tetrahydrospiro[cyclohexan-1,1'-pyrido[3,4-b]indol]-4-amin; und
- 2-Benzyl-N,N-dimethyl-4-phenyl-2',3',4',9'-tetrahydrospiro[cyclohexan-1,1'-pyrido[3,4-b]indol]-4-amin
- 6'-Fluor-2-(3-fluorbenzyl)-N,N-dimethyl-4-phenyl-4',9'-dihydrospiro(cyclo-hexan-1,1'-pyrano-[3,4-b]indol]-4-amin
- 2-(3-Fluorbenzyl)-N,N-dimethyl-4-phenyl-2',3',4',9'-tetrahydrospiro[cyclohexan-1,1'-pyrido[3,4-b]indol]-4-amin
- N,N-Dimethyl-4-phenyl-2-(phenylthio)-4',9'-dihydro-3'H-spiro-[cyclohexan-1,1'-pyrano[3,4-b]indol]-4-amin
- N,N-Dimethyl-4-phenyl-2-(phenylthio-2',3',4',9'-tetrahydrospiro[cyclohexan-1,1'-pyrido[3,4-b]indol]-4-amin
- 2-(3-Fluorbenzyl)-N,N-dimethyl-4-phenyl-2',3',4',9'-tetrahydrospiro[cyclohexan-1,1'-pyrido[3,4-b]indol]-4-amin
- 2-(4-(Dimethylamino)-4-phenyl-4',9'-dihydro-3'H-spiro[cyclohexan-1,1'-pyrano[3,4-b]indol]-2-yl)essigsäure-methylester
- 2-Allyl-N,N-dimethyl-4-phenyl-4',9'-dihydros-3'H-piro[cyclohexan-1,1'-pyrano[3,4-b]indol]-4-amin
- 2-Fluor-N,N-dimethyl-4-phenyl-4',9'-dihydro-3'H-spiro[cyclohexan-1,1'-pyrano[3,4-b]indol]-4-amin
- 2-Allyl-N,N-dimethyl-4-phenyl-4',9'-dihydros-3'H-piro[cyclohexan-1,1'-pyrano[3,4-b]indol]-4-amin
- 2,6'-Difluor-N,N-dimethyl-4-phenyl-4',9'-dihydro-3'H-spiro[cyclohexan-1,1'-pyrano[3,4-b]indol]-4-amin
- 4-(Dimethylamino)-4-phenyl-4',9'-dihydro-3'H-spiro[cyclohexan-1,1'-pyrano[3,4-b]indol]-2-yl)methanol
- 2,6'-Difluor-N,N-dimethyl-4-phenyl-4',9'-dihydro-3'H-spiro[cyclohexan-1,1'-pyrano[3,4-b]indol]-4-amin
- 2-Fluor-N,N-dimethyl-4-phenyl-2',3',4',9'-tetrahydrospiro[cyclohexan-1,1'-pyrido[3,4-b]indol]-4-amin
- 2-Fluor-N,N-dimethyl-4-phenyl-2',3',4',9'-tetrahydrospiro[cyclohexan-1,1'-pyrido[3,4-b]indol]-4-amin
- 2-(4-(Dimethylamino)-4-phenyl-4',9'-dihydro-3'H-spiro[cyclohexan-1,1'-pyrano[3,4-blindol]-2-yl)ethanol
- 2-(4-(Dimethylamino)-4-phenyl-4',9'-dihydro-3'H-spiro[cyclohexan-1,1'-pyrano[3,4-b]indol]-2-yl)methyl)isoindolin-1,3-dion
- N-((4-(Dimethy)amino)-4-(3-fluorphenyl)-4',9'-dihydro-3'H-spiro[cyclohexan-1,1'-pyrano[3,4-b]indol]-2-yl)methyl)-3-phenylzimtsäureamid
- tert-Butyl 2-(2-(4-(dimethy)amino)-4-pheny)-4',9'-dihydro-3'H-spiro[cyclohexan-1,1'-pyrano[3,4-b]indol]-2-yl)ethoxy)acetat
- 2-(4-(Dimethylamino)-4-phenyl-4',9'-dihydro-3'H-spiro[cyclohexan-1,1'-pyrano[3,4-b]indol]-2-yl)acetonitril
oder deren physiologisch verträgliche Salze und/oder Solvate.

Die erfindungsgemäßen Verbindungen wirken beispielsweise auf den im Zusammenhang mit verschiedenen Erkrankungen relevanten ORL1-Rezeptor, sodass sie sich als pharmazeutischer Wirkstoff in einem Arzneimittel eignen.

Ein weiterer Gegenstand der Erfindung betrifft daher Arzneimittel, welche wenigstens eine erfindungsgemäße Verbindung enthalten, sowie ggf. geeignete Zusatz- und/oder Hilfsstoffe und/oder ggf. weitere Wirkstoffe.

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfinungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens einer erfindungsgemäßen Verbindung appliziert.

Für alle vorstehenden Formen der erfindungsgemäßen Arzneimittel ist es besonders bevorzugt, wenn das Arzneimittel neben wenigstens einer erfindungsgemäßen Verbindung noch einen weiteren Wirkstoff, insbesondere ein Opioid, vorzugsweise ein starkes Opioid, insbesondere Morphin, oder ein Anästhetikum, vorzugsweise Hexobarbital oder Halothan, enthält.

In einer bevorzugten Form des Arzneimittels liegt eine enthaltene erfindungsgemäße Verbindung als reines Diastereomer und/oder Enantiomer vor.

Der ORL1-Rezeptor wurde insbesondere im Schmerzgeschehen identifiziert. Entsprechend können erfindungsgemäße Verbindungen zur Herstellung eines Arzneimittels zur Behandlung von Schmerz, insbesondere von akutem, neuropathischem oder chronischem Schmerz, verwendet werden.

Ein weiterer Gegenstand der Erfindung betrifft daher die Verwendung einer erfindungsgemäßen Verbindung zur Herstellung eines Arzneimittels zur Behandlung von Schmerz, insbesondere von akutem, viszeralem, neuropathischem oder chronischem Schmerz.

Ein weiterer Gegenstand der Erfindung betrifft die Verwendung einer erfindungsgemäßen Verbindung zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anästhetikum bzw. zur Coadministration bei Behandlung mit einem opioiden Analgetikum oder mit einem Anästhetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn eine verwendete Verbindung als reines Diastereomer und/oder Enantiomer, als Razemat oder als nichtäquimolare oder äquimolare Mischung der Diastereomere und/oder Enantiomere vorliegt.

Ein weiterer Gegenstand der Erfindung betrifft ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wirksamen Dosis einer erfindungsgemäßen Verbindung, oder eines erfindungsgemäßen Arzneimittels.

Ein weiterer Gegenstand der Erfindung betrifft ein Verfahren zur Herstellung der erfindungsgemäßen Verbindungen wie in der folgenden Beschreibung und Beispielen ausgeführt.

### a) Derivatisierung in der 2,3,5 und/oder 6-Position der Cyclohexandionketale

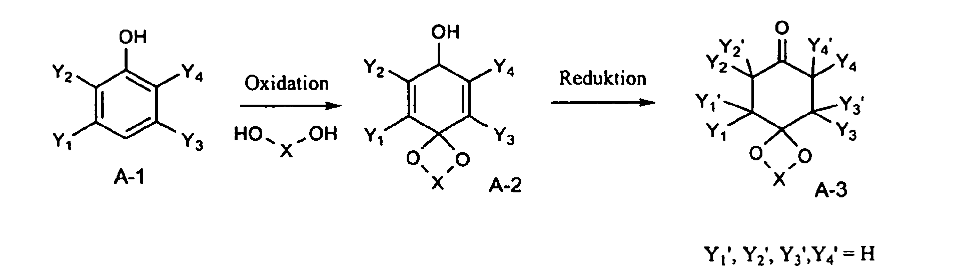

Substituierte Cyclohexandionketale des Typs A-3 lassen sich durch dem Fachmann bekannte Methoden aus den bekannten Edukten A-1 synthetisieren. In der Literatur wird die Oxidation von Phenolen A-1 mittels hypervalenten lodreagenzien zu den intermediären Cyclohexadienonketalen A-2 beschrieben (Rose et al.Can. J. Chem., 74, 1996, 1836.). Verbindungen der Formel A-3 können dann aus den entsprechenden Ketalen A-2, nach dem Fachmann bekannten Methoden durch Reduktion unter Wasserstoffatmosphäre und in Anwesenheit von Metallkatalysatoren z.B. auf Rhodiumbasis erhalten werden.

### b) Derivatisierung in 2-Position der Cyclohexandionketale

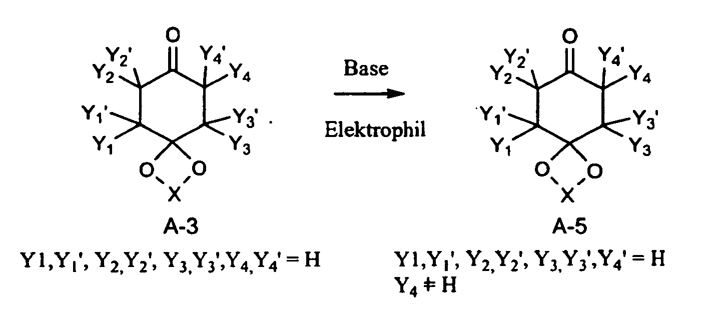

α-substituierte Cyclohexandionketale der allgemeinen Formel A-5 können durch Umsatz der unsubstituierten Ketale A-3 mit einer Base, beispielsweise Lithiumdüsopropylamid (LDA), Lithiumhexamethyldisilazid (LHMDS), Kaliumhexamethyldisilazid (KHMDS), Natriumhydrid (NaH), Kaliumhydrid (KH), Natriummethanolat (NaOMe), Kaliumtertbutoxylat (K^{t}OBu), Aminbasen wie z.B. Diethylamin (HNEt₂), Diisopropylethylamin (Hünig's Base), Piperidin, Pyrrolidin, Prolin, und mit den entsprechenden Elektrophilen z. B. vom Typ Y₄-X (mit X= z. B. Br, I, OTos, OTf etc. und Y₄ = z.B. Alkyl, Benzyl) in organischen Lösungsmitteln oder Lösungsmittelgemischen, beispielsweise Dichlormethan (DCM), Dichlorethan (DCE), Diethylether (Et₂O), Tehtrahydrofuran (THF), Dimethoxyethan (DME), Methanol (MeOH), Ethanol (EtOH), Dimethylformamid (DMF), Dimethylsulfoxid (DMSO) bei Temperaturen zwischen -78°C und 150°C umgesetzt werden. Desweiteren kann das generierte Anion mit entsprechenden Michael-Akzeptorsystemen umgesetzt werden. Die Einführung von Heteroatomen kann durch Umsetzung mit Dischwefelverbindungen (Y₄ = S-alkyl oder S-aryl), entsprechenen elektrophilen Fluorierungsreagenzien wie z.B. Selectfluor™ (Y₄ = F), entsprechenen elektrophilen Aminierungsreagenzien wie z.B. N-alkoxycarbonyl- oder N-carboxamido-Oxaziridine (Y₄ = NR₂) oder entsprechenen elektrophilen Hydroxylierungsreagenzien wie z.B. Oxodiperoxymolybdän(pyridin)(hexamethylphosphortriamid)-Komplex (MoOPH (Y₄ = OH) erfolgen.
Aldolartige Umsetzungen können auch im sauren Milieau erfolgen. Substiuenten können darüber hinaus mittels einer Mannichreaktion unter sauren Bedingungen (Camphersulfonsäure , p-TosOH etc.) eingeführt werden.

### c) Synthese der Aminocvclohexanone

### (1) Aminonitril-/ Triazol-Route

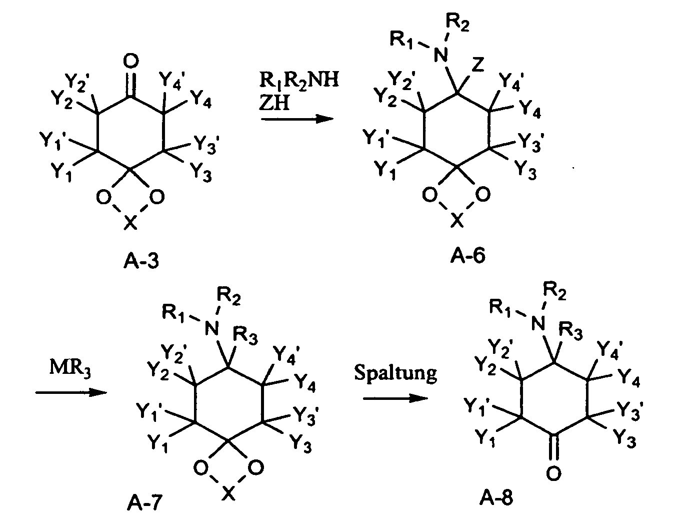

Strukturen der Formel A-6 lassen sich durch Reaktion von Ketonen A-3 mit Aminen und aciden Reaktanden Z-H herstellen. Geeignete Reaktanden Z-H sind z. B. Cyanwasserstoff, 1,2,3-Triazol, Benzotriazol oder Pyrazol.

Ein besonders bevorzugter Weg zu Verbindungen der Struktur A-6 ist die Umsetzung von Ketonen mit Metallcyaniden und dem entsprechenden Amin in Gegenwart von Säure, vorzugsweise in einem Alkohol, bei Temperaturen von - 40 bis 60°C, vorzugsweise bei Raumtemperatur mit Alkalimetallcyaniden in Methanol.

Ein weiterer besonders bevorzugter Weg zu Verbindungen der Struktur A-6 ist die Umsetzung von Ketonen mit 1,2,3-Triazol und dem entsprechenden Amin in Gegenwart unter wasserentziehenden Bedingungen, vorzugsweise unter Verwendung eines Wasserabscheiders bei erhöhter Temperatur in einem inerten Löungsmittel oder unter Verwendung von Molsieb oder einem anderen Trockenmittel. Analog lassen sich A-6 analoge Strukturen mit Benzotriazol- oder Pyrazol- statt Triazolgruppen einführen.

Allgemein können Ketale A-7 auch durch Substitution von geeigneten Abgangsgruppen Z in Strukturen der Formel A-6 erhalten werden. Geeignete Abgangsgruppen sind bevorzugt Cyanogruppen; 1,2,3-Triazol-1-yl-Gruppen. Weitere geeignete Abgangsgruppen sind 1H-Benzo[d][1,2,3]triazol-1-yl-Gruppen und Pyrazol-1-yl-Gruppen (Katritzky et al., Synthesis 1989, 66-69).

Ein besonders bevorzugter Weg zu Verbindungen der Struktur A-7 ist die Umsetzung von Aminonitrilen A-6 mit entsprechenden Organometallverbindungen, vorzugsweise Grignardverbindungen, vorzugsweise in Ethern, vorzugsweise bei RT. Die Organometallverbindungen sind entweder käuflich erhältlich oder können nach bekannten Methoden hergestellt werden. Ein weiterer besonders bevorzugter Weg zu Verbindungen der Struktur A-7 ist die Umsetzung von Aminotriazolen A-6 mit entsprechenden Organometallverbindungen, vorzugsweise Grignardverbindungen, vorzugsweise in Ethern, vorzugsweise bei RT.

Die Organometallverbindungen sind entweder käuflich erhältlich oder können nach literaturbekannten Methoden hergestellt werden.

Verbindungen der Formel A-8 können aus entsprechenden Ketalen A-7, oder aus deren Salzen, nach dem Fachmann bekannten Methoden durch Entschützen mittels Säuren freigesetzt werden. Dabei ist X aus der Gruppe Alkyl, Alkyl/ Alkyliden/ mit Aryl oder Alkyl (gesättigt/ungesättigt) substituiertem Alkyliden ausgewählt.

### (2) lminroute

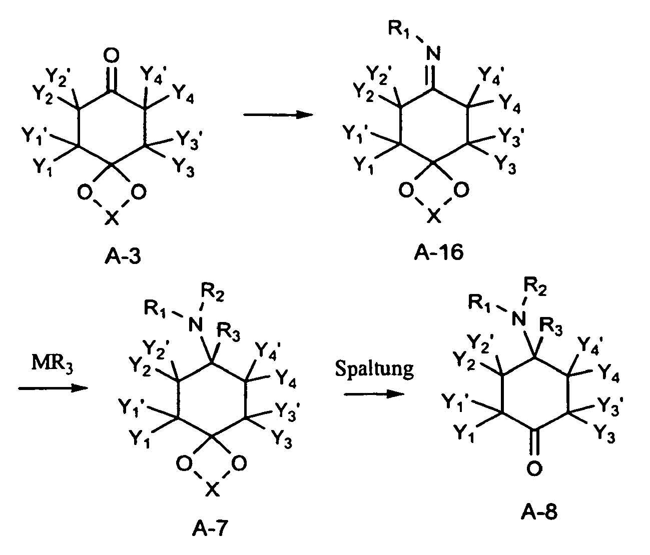

In der Imin-Route wird aus einem Ketonvorläufer A-3 das Imin A-16 synthetisiert, welches unter Verwendung eines Nukleophils MR3 in den Baustein A-7 und weiter in A-8 überführt wird. Die benötigten Iminbausteine A-16 können nach einer dem Fachmann bekannten Methode hergestellt werden (Layer, Chem. Rev., 1963, 8, 489-510). Für Addition der Organometallspezies MR3 an das Imin A-16 wurde auf literaturbekannte Verfahren (z.B. Maddox et al., J. Med. Chem., 1965, 8, 230-235. Kudzma et al., J. Med. Chem., 1989, 32, 2534-2542.) zurückgegriffen.

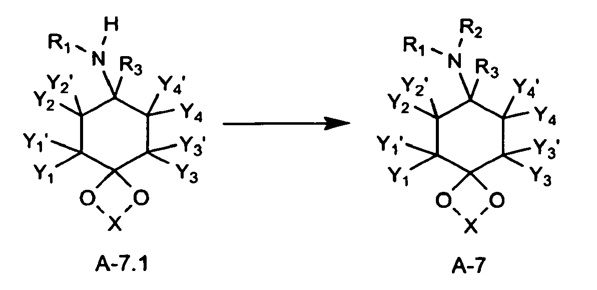

Aminoacetale A-7.1 mit maximal einem Substituenten am Stickstoffatom können nach dem Fachmann prinzipiell bekannte Verfahren, z.B. durch reduktive Aminierung, in entsprechende Amino-acetale A-7 mit einem oder zwei weiteren Substituenten (R2 ≠H) am Stickstoff übergeführt werden.

### d) Derivatisierung in 2-Position der Aminocyclohexanone

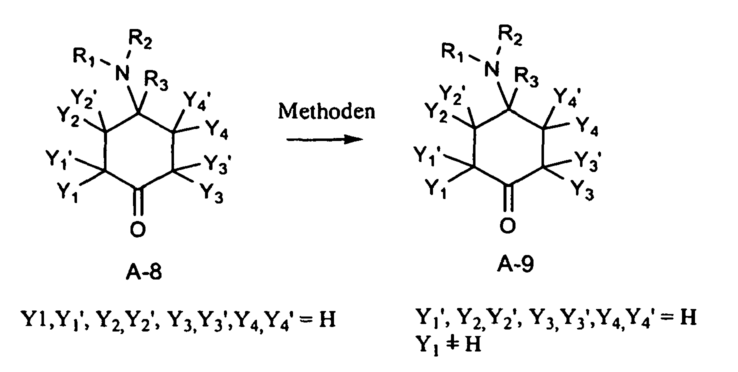

Substituierte Aminocyclohexanone des Typs A-9 lassen sich durch dem Fachmann bekannte Methoden aus den bekannten Edukten A-8 synthetisieren.

### Methode 1:

In der Literatur wird die α-Arylierung von Ketonen A-8 mit den entsprechenden Arylhalogeniden z. B. vom Typ Y₁-X (mit Y₁ = Aryl/Hetaryl und X= Br, I) durch Palladiumkatalyse in Anwesenheit von geeigneten Phosphinliganden wie z.B. Xantphos beschrieben (Elliott et al. Bioorg. Med. Chem. Lett.; EN; 16; 11; 2006; 2929; Dirat et al. Tetrahedron Lett.; EN; 47; 8; 2006; 1295.)

### Methode 2:

α-Substituierte Aminocyclohexanone des Typs A-9 können durch Umsatz der unsubstituierten Ketale A-8 mit einer Base, beispielsweise Lithiumdiisopropylamid (LDA), Lithiumhexamethyldisilazid (LHMDS), Kaliumhexamethyldisilazid (KHMDS), Natriumhydrid (NaH), Kaliumhydrid (KH), Natriummethanolat (NaOMe), Kaliumtertbutoxylat (K^{t}OBu), Aminbasen wie z.B. Diethylamin (HNEt₂), Diisopropylethylamin (Hünig's Base), Piperidin, Pyrrolidin, Prolin, und mit den entsprechenden Elektrophilen z. B. vom Typ Y₄-X (mit X= z. B. Br, I, OTos, OTf etc.) in organischen Lösungsmitteln oder Lösungsmittelgemischen, beispielsweise Dichlormethan (DCM), Dichlorethan (DCE), Diethylether (Et₂O), Tehtrahydrofuran (THF), Dimethoxyethan (DME), Methanol (MeOH), Ethanol (EtOH), Dimethylformamid (DMF), Dimethylsulfoxid (DMSO) bei Temperaturen zwischen -78°C und 150°C umgesetzt werden. Desweiteren kann das generierte Anion mit entsprechenden Michael-Akzeptorsystemen umgesetzt werden. Die Einführung von Heteroatomen kann durch Umsetzung mit Dischwefelverbindungen (Y₄ = S-alkyl oder S-aryl), entsprechenen elektrophilen Fluorierungsreagenzien wie z.B. Selectfluor™ (Y₄ = F), entsprechenen elektrophilen Aminierungsreagenzien wie z.B. N-alkoxycarbonyl- oder N-carboxamido-Oxaziridine (Y₄ = NR₂) oder entsprechenen elektrophilen Hydroxylierungsreagenzien wie z.B. Oxodiperoxymolybdän-(pyridin)(hexamethylphosphortriamid)-Komplex (MoOPH (Y₄ = OH) erfolgen. Aldolartige Umsetzungen können auch im sauren Milieau erfolgen. Substiuenten können darüber hinaus mittels einer Mannichreaktion unter sauren Bedingungen (Camphersulfonsäure , p-TosOH etc.) eingeführt werden.

### e) Synthese spirozyklischer Verbindungen vom Typ A-11 und A-13

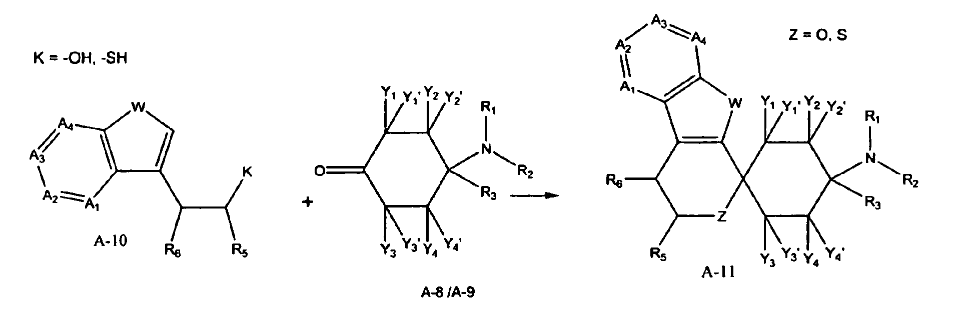

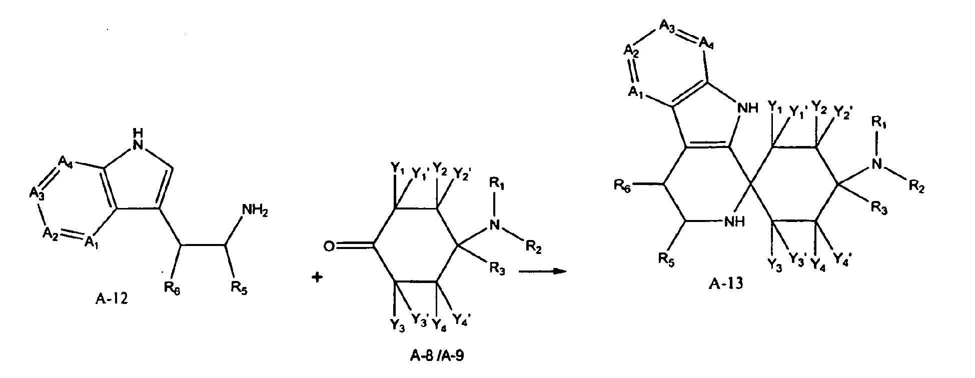

Tryptophole bzw. andere Heterocyclen des Typs A-10 (K = O können in Reaktionen von Typ der Oxa-Pictet-Spengler-, und Tryptamine bzw. andere Heterocyclen des Typs A-12 in Reaktionen von Typ der Pictet-Spengler-Reaktion, mit Ketonen von Typ A-8/A-9 unter Zugabe von mindestens einem geeigneten Reagens aus der Gruppe Säuren, Säureanhydride, Ester oder schwach sauer reagierende Salze oder Lewissäuren unter Bildung von Produkten der Formel A-11/A-13 zur Reaktion gebracht werden. Für X = SH verläuft die Reaktion analog. Bevorzugt kommt dabei mindestens ein Reagens aus der Gruppe der Carbonsäuren, Phosphorsäuren oder Sulfonsäuren oder deren jeweiligen Anhydriden, Carbonsäuretrialkylsilylester, sauer reagierenden Salze, Mineralsäuren oder Lewissäuren ausgewählt aus der Gruppe bestehend aus Bortrifluorid, Indium(III)chlorid, Titantetrachlorid, Aluminium-(III)-chlorid, oder unter Zusatz wenigstens eines Übergangsmetallsalzes, vorzugsweise unter Zusatz wenigstens eines Übergangsmetalltriflats (Übergangsmetalltrifluormethansulfonats), besonders bevorzugt unter Zusatz wenigstens eines Übergangsmetalltrifluormethansulfonats ausgewählt aus der Gruppe bestehend aus Scandium(III)trifluormethansulfonat, Ytterbium-(III)-trifluormethansulfonat und Indium(III)trifluormethansulfonat, ggf. unter Zusatz von Celite, mit festphasengebundenen Reaktanden oder Reagenzien, bei erhöhter oder erniedrigter Temperatur, mit oder ohne Mikrowelleneinstrahlung, ggf. in einem geeigneten Lösungsmittel oder Lösungsmittelgemisch wie beispielsweise, chlorierten oder unchlorierten, vorzugsweise aromatischen, Kohlenwasserstoffen, Acetonitril; in etherischen Lösungsmitteln, vorzugsweise in Diethylether oder THF; oder in Nitromethan, in geeigneten Fällen auch in Alkoholen oder Wasser, zum Einsatz.

Besonders bevorzugt werden dabei Pyridinium-para-Toluolsulfonat, Phosphorpentoxid in Gegenwart von Celite, Bortrifluorid-etherat, Trifluoressigsäure, Orthotitansäure-tetraisopropylester zusammen mit Trifluoressigsäure, Trifluormethansulfonsäuretrimethylsilylester, Trifluormethansulfonsäure, Methansulfonsäure, Trifluoressigsäure, Essigsäure, Phosphorsäure, Polyphosphorsäure, Polyphosphatester, p-Toluolsulfonsäure, Salzsäure HCl-Gas, Schwefelsäure zusammen mit Acetatpuffer, Zinntetrachlorid, eingesetzt.

### f) Synthese spirozyklischer Verbindungen vom Typ A-14

### Bausteinsynthesen- Alkine

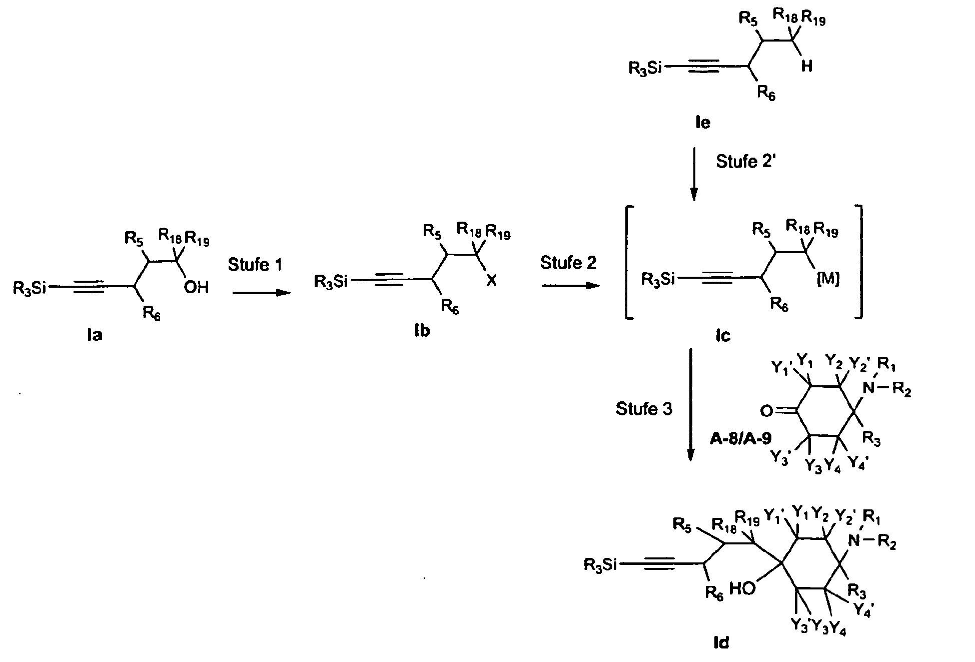

In Stufe 1 werden Alkohole der allgemeinen Formel Ia entweder nach Überführung in eine Abgangsgruppe (z. B. -OSO₂-Me -OSO₂-p-Toluol. -OTf) oder direkt (im Sinne einer Mukaiyama Redoxkondensation) in Halogenide der allgemeinen Formel Ib (X= Cl, Br, I) überführt. Diese weredn durch Halogen-Metallaustausch entweder zu den entsprechenden Lithiumorganylen ([M]=Li] oder Grignardreagenzien ([M]= MgX] vom Typ Ic umgesetzt (Stufe 2).

Alternativ dazu werden in Stufe 2' ausgehend von Alkinen der allgemeinen Formel Ie (mit R₁₈ oder R₁₉ = z.B. SO₂Ph, SOPh, -CN, -C(=O)N(CH₃)OCH₃) durch Deprotonierung mit Lithiumamiden (z. B. LDA) Lithiumorganyle vom Typ Ic hergestellt.

In Stufe 3 werden die metalierten Organyle der allgemeinen Formel Ic im Sinne einer 1,2-Addition an die Carbonylgruppe von Cyclohexanonen der allgemeinen Formel A-8/A-9 zu den entsprechenden Alkinbausteine vom Typ Id umgesezt.

### Larockreaktion und Spirozyklisierung

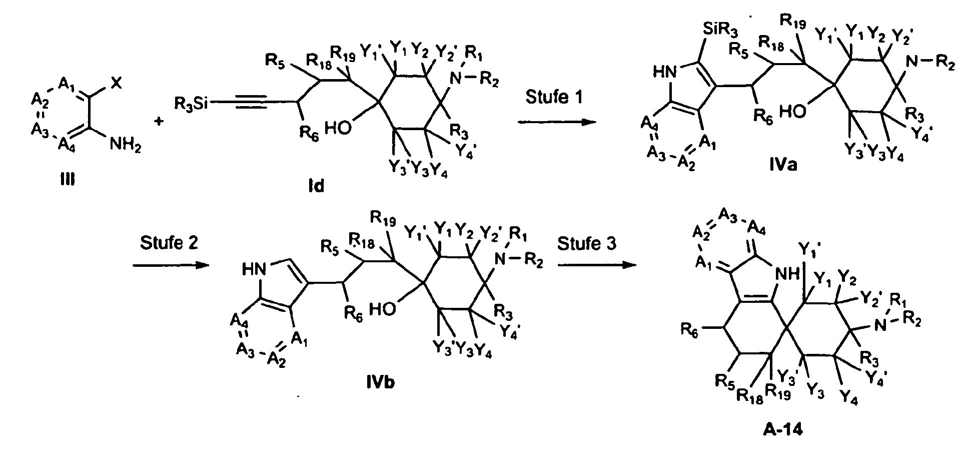

In Stufe 1 werden Verbindungen der allgemeinen Formel III, worin X für einen Halogenrest oder einen Sulfonsäureester steht im Sinne einer Indolsynthese nach Larock unter Zusatz eines Palladiumkatalysators mit Alkinen der allgemeinen Formel Id zu Indolen der allgemeinen Formel IVa umgesetzt. Verbindungen der allgemeinen Formel III sind kommerziell erhältlich (exemplarische Synthesen siehe auch W02008009416). In Stufe 2 werden Verbindungen der allgememeinen Formel IVa in Gegenwart von Fluorid oder in Gegenwart einer oganischen oder anorganischen Säure desilyliert und zu Verbindungen der allgemeinen Formel IVb umgesetzt. Zur Herstellung der spirocyclischen Verbindungen der allgemeinen Formel A-14 werden die Alkohole der allgemeinen Formel IVb unter Zusatz einer organischen Säure oder deren Trimethylsilylester oder einer anorganischen Säure oder unter Zuatz eines Übergangsmetallsalzes umgesetzt.

### g) Derivatisierung spirozyklischer Verbindungen (sekundäre Amine)

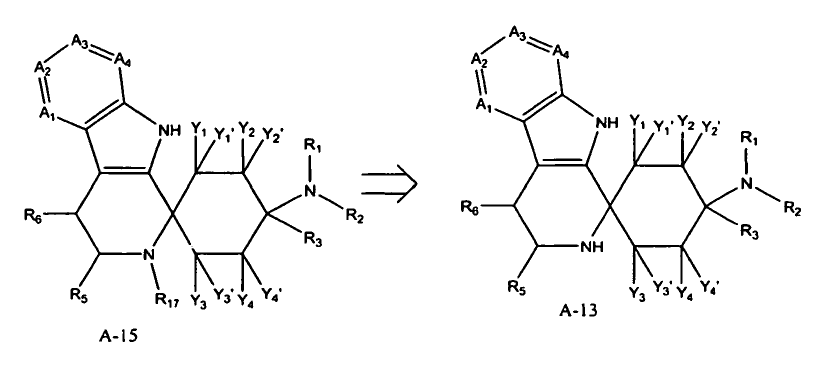

Sekundäre Amine des Typs A-13 können nach dem Fachmann bekannten Verfahren zu Verbindungen des Typs A-15 acyliert, sulfonyliert oder carbamoyliert werden. Bevorzugt werden diese Reaktionen bei erhöhter Temperatur, besonders bevorzugt unter Mikrowelleneinstrahlung, durchgeführt.

Eine solche, dem Fachmann bekannte Methode kann die Umsetzung mit einem Anhydrid oder einem Säurechlorid unter Zugabe einer Base, beispielsweise Triethylamin, darstellen.

Verbindungen der allgemeinen Formel A-13 können mit Isocyanaten in die entsprechende Harnstoffderivate vom Typ A-15 überführt werden.

Desweiteren können Verbindungen der allgemeinen Formel A-13 mit Aldehyden unter Zusatz wenigstens eines Reduktionsmittels im Sinne einer reduktiven Aminierung zu Verbindungen des Typs A-15 umgesetzt werden

### h) Vorstufen

Verbindungen der allgemeinen Formeln A-1, A-3, A-10 und A-12 sind entweder kommerziell erhältlich, oder ihre Herstellung ist aus dem Stand der Technik bekannt oder in für den Fachmann offensichtlicher Weise aus dem Stand der Technik ableitbar. Insbesondere relevant sind hierfür die folgenden Zitate: Jirkovsky et al., J. Heterocycl. Chem., 12, 1975, 937-940; Beck et al., J. Chem. Soc. Perkin 1, 1992, 813-822; Shinada et al., Tetrahedron Lett., 39, 1996, 7099-7102; Garden et al., Tetrahedron, 58, 2002, 8399-8412; Lednicer et al., J. Med. Chem., 23, 1980, 424-430; Bandini et al. J. Org. Chem. 67, 15; 2002, 5386 - 5389; Davis et al., J.Med.Chem. 35, 1, 1992, 177-184; Yamagishi et al., J.Med.Chem. 35, 11, 1992, 2085-2094; Gleave et al.; Bioorg.Med.Chem.Lett. 8, 10, 1998, 1231-1236; Sandmeyer, Helv.Chim.Acta; 2; 1919; 239; Katz et al.; J. Med. Chem. 31, 6, 1988; 1244-1250; Bac et al. Tetrahedron Lett. 1988, 29, 2819; Ma et al. J. Org. Chem. 2001, 66, 4525; Kato et al. J. Fluorine Chem. 99, 1, 1999, 5-8.

Bezüglich weiterer Einzelheiten zur Synthese der erfindungsgemäßen Verbindungen, insbesondere im Hinblick auf die Synthese geeigneter Eduktbausteine, wird ferner vollumfänglich verwiesen auf WO2004/043967, W02005/063769, W02005/066183, WO2006/018184, WO2006/108565, W02007/124903 und WO2008/009416. Ein Fachmann erkennt, dass geeignete Eduktbausteine für die Synthese der erfindungsgemäßen Verbindungen in Analogie zu den in diesen Druckschriften offenbarten Syntheseschemata und Ausführungsbeispielen hergestellt werden können.

### Beispiele

Die folgenden Beispiele dienen zur näheren Erläuterung der Erfindung, sind jedoch nicht einschränkend auszulegen.

Die Ausbeuten der hergestellten Verbindungen sind nicht optimiert. Alle Temperaturen sind unkorrigiert. Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur, "abs." absolut (wasserfrei),"rac." racemisch, "konz." konzentriert, "min" Minuten, "h" Stunden, "d" Tage, "Vo).%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt. Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Laufmitteln für chromatographische Untersuchungen sind stets in Volumen/Volumen angegeben.

### 1. Bausteinsynthese:

### Ketonbausteine

### (±)-4-Dimethylamino-2-methyl-4-phenylcyclohexanon

Diisopropylamin (2,1 ml, 15 mmol) wurde in trockenem THF unter Argon vorgelegt und bei 30 °C mit n-Butyllithium in Hexan (2,5M, 6 ml, 15 mmol) versetzt. Das Reaktionsgemisch wurde auf -78 °C gekühlt. Zu dieser Lösung wurde 4-(Dimethylamino)-4-phenylcyclohexanon (2,17 g, 10 mmol), gelöst in 10 ml trockenem THF, innerhalb von 10 min zugetropft. Der Ansatz wurde 30 min bei -78 ° C gerührt und anschliessend mit Methyljodid (1,86 ml, 30 mmol) versetzt. Es wurde auf RT erwärmt und weitere 18 h gerührt. Das Lösungsmittel wurde am Rotationsverdampfer entfernt und der Rückstand in DCM aufgenommen. Es wurde mit 1 N HCl (3 x 30 ml) extrahiert. Die wässrige Phase wurde mit 5N NaOH (20 ml) basisch gestellt und mit DCM (3 x 30 ml) extrahiert. Die vereinigten organischen Phasen wurden mit H₂O gewaschen (2 x 10 ml) und über Na₂SO₄ getrocknet. Der nach Entfernung des Lösungsmittels erhaltene halbfeste Rückstand (1,2 g) wurde aus Ethylacetat (1 ml) umkristallisiert.
*Ausbeute:* 549 mg (23 %)
*Schmelzpunkt:* 50 °C

### (±)-2-Benzyl-4-dimethylamino-4-phenylcyclohexanon

Diisopropylamin (2,1 ml, 15 mmol) wurde in trockenem THF (30 ml) unter Argon vorgelegt und bei -30 °C (Badtemperatur) mit einer Lösung von Butyllithium in Hexan (2,5M, 6 ml, 15 mmol) versetzt. Das Reaktionsgemisch wurde auf -78 °C abgekühlt. Nach Erreichen dieser Temperatur wurde 4-(Dimethylamino)-4-phenylcyclohexanon (2,17 g, 10 mmol), gelöst in trockenem THF (10 ml), innerhalb von 15 min zugetropft. Der Ansatz wurde 30 min bei -78 ° C belassen. Dann wurde Benzylbromid (3,6 ml, 30 mmol), gelöst in trockenem THF (20 ml), innerhalb von 10 min zugegeben. Das Reaktionsgemisch wurde weitere 15 min bei 78 °C gerührt, dann wurde die Kühlung entfernt. Nach Erreichen von Raumtemperatur wurde der Ansatz weitere 18 h gerührt. - Zur Aufarbeitung wurde der Ansatz vorsichtig mit Wasser (1 ml) versetzt, dann erfolgte Zugabe von gesättigter NH4CI-Lösung (40 ml). Die organische Phase wurde abgetrennt und die wäßrige Phase mit Ethylacetat (3 x 20 ml) extrahiert. Die vereinigten organischen Phasen wurden mit gesättigter NH4CI-Lösung (2 x 40 ml) gewaschen. Anschließend wurden die organischen Phasen mit 1 N HCl (50 ml) versetzt und gründlich ausgeschüttelt. Die saure, wäßrige Phase wurde abgetrennt, die organische Phase mit Wasser (2 x 20 ml) gewaschen. Die vereinigten wäßrigen Phasen wurden mit Ethylacetat (1 x 30 ml) gewaschen. Dann wurde die wäßrige Lösung auf 70 ml 2N NaOH gegeben. Aus der alkalischen Lösung fiel ein Öl aus. Die entstandene Mischung wurde mit Ethylacetat (3 x 30 ml) extrahiert. Die vereinigten organischen Phasen wurden über MgS04 getrocknet und anschließend eingeengt. Aus dem erhaltenen Rückstand (2,27 g) wurde durch chromatographische Reinigung [Kieselgel 60 (50 g); Ethylacetat (1000 ml)] eins der möglichen Diastereoisomeren isoliert.
*Ausbeute:* 1,2 g (40 %).
*¹³C NMR* (101 MHz. CDCl₃) δ ppm: 32.8, 33.2, 34.9, 37.3, 38.1, 38.3, 38.7, 47.6, 61.5, 126.1, 127.0, 127.6, 128.2, 128.3, 129.0, 135.4, 139.8, 211.4.

### (±)-2-(3-Fluorbenzyl)-4-dimethylamino-4-phenylcyclohexanon

Diisopropylamin (2,1 ml, 15 mmol) wurde in trockenem THF (30 ml) unter Argon vorgelegt und bei -30 °C (Badtemperatur) mit einer Lösung von Butyllithium in Hexan (2,5M, 6 ml, 15 mmol) versetzt. Das Reaktionsgemisch wurde auf -78 °C gekühlt. Nach Erreichen dieser Temperatur wurde 4-(Dimethylamino)-4-phenylcyclohexanon (2,17 g, 10 mmol), gelöst in trockenem THF (20 ml), innerhalb von 15 min zugetropft. Der Ansatz wurde 30 min bei -78° C belassen. Dann wurde 1-(Brommethyl)-3-fluorbenzol (3,7 ml, 30 mmol), gelöst in trockenem THF (10 ml), innerhalb von 1 min zugegeben. Das Reaktionsgemisch wurde weitere 10 min bei -78 °C gerührt, dann wurde die Kühlung entfernt. Nach Erreichen von Raumtemperatur wurde der Ansatz weitere 18 h gerührt. - Zur Aufarbeitung wurde der Ansatz vorsichtig mit Wasser (1 ml) versetzt, dann erfolgte Zugabe von gesättigter NH₄Cl-Lösung (40 ml). Die organische Phase wurde abgetrennt und die wäßrige Phase mit Ethylacetat (3 x 20 ml) extrahiert. Die vereinigten organische Phasen wurden mit gesättigter NH₄Cl-Lösung (2 x 40 ml) gewaschen. Anschließend wurden die organischen Phasen mit 1 N HCl (30 ml) versetzt und gründlich ausgeschüttelt. Die saure, wäßrige Phase wurde abgetrennt, die organische Phase mit Wasser (2 x 20 ml) gewaschen. Die vereinigten wäßrigen Phasen wurden mit Ethylacetat (1 x 30 ml) gewaschen. Dann wurde die wäßrige Lösung auf 70 ml 2N NaOH gegeben. Aus der alkalischen Lösung fiel ein Öl aus. Die entstandene Mischung wurde mit Ethylacetat (3 x 30 ml) extrahiert. Die vereinigten organischen Phasen wurden über MgS04 getrockneten und anschließend eingeengt. Aus dem erhaltenen Rückstand (2,1 g) wurde durch chromatographische Reinigung [Kieselgel 60 (70 g); Ethylacetat (700 ml)] eins der möglichen Diastereoisomeren, isoliert.
*Ausbeute:* 1,11 g (ca. 34 %), Reinheit ca. 90%.
*¹³C NMR* (101 MHz, CDCl3) δ ppm: 33.0, 34.7, 38.1, 38.3, 38.7, 38.8, 47.3, 61.5, 113.0, 113.1, 115.7, 115.9, 124.7, 127.0, 127.1, 127.3, 127.5, 127.9, 128.3, 129.7, 129.8, 135.4, 142.4, 142.5, 161.7, 164.1, 211.0.

### (±)-4-Dimethylamino-4-phenyl-2-thiophenylcyclohexanon (unpolareres Diastereoisomer und polareres Diastereoisomer)

Diisopropylamin (1,65 ml, 11,4 mmol) wurde in trockenem THF (20 ml) unter Argon vorgelegt und bei -30 °C (Badtemperatur) mit einer Lösung von Butyllithium in Hexan (2,5M, 5 ml, 12,5 mmol) versetzt. Das Reaktionsgemisch wurde auf -78 °C gekühlt. Nach Erreichen dieser Temperatur wurde das 4-(Dimethylamino)-4-phenylcyclohexanon (2,17 g, 10 mmol), gelöst in trockenem THF (10 ml), innerhalb von 1-2 min zugetropft. Der Ansatz wurde 30 min bei -78 ° C belassen. Dann wurde Diphenyldisulfid (2,18 g, 10 mmol), gelöst in trockenem THF (10 ml), innerhalb von 10 min zugegeben. Das Reaktionsgemisch wurde weitere 60 min bei -78 °C gerührt, dann wurde die Kühlung entfernt. Nach Erreichen von Raumtemperatur wurde der Ansatz weitere 18 h gerührt. - Zur Aufarbeitung wurde der Ansatz vorsichtig mit Wasser (1 ml) versetzt, dann erfolgte Zugabe von gesättigter NH₄Cl-Lösung (40 ml). Nach intensiver Durchmischung der Phasen wurde das organische Lösungsmittel abgetrennt und die wäßrige Phase mit Ethylacetat (3 x 20 ml) extrahiert. Die vereinigten org. Phasen wurden mit gesättigter NH₄Cl-Lösung (2 x 40 ml) gewaschen. Anschließend wurden die organischen Phasen mit 1N HCl (50 ml) versetzt und gründlich ausgeschüttelt. Die saure, wäßrige Phase wurde abgetrennt und die organische Phase mit Wasser (2 x 20 ml) gewaschen. Die vereinigten wäßrigen Phasen wurden mit Ethylacetat (1 x 30 ml) gewaschen. Dann wurde die wäßrige Lösung auf 2N NaOH (70 ml) gegeben. Aus der alkalischen Lösung fiel ein Öl aus. Die entstandene Mischung wurde mit Ethylacetat (3 x 30 ml) extrahiert. Die vereinigten organischen Phasen wurden über M_{g}SO₄ getrocknet und anschließend eingeengt. Aus dem erhaltenen Rückstand (2,48 g) wurde durch chromatographische Reinigung [Kieselgel 60 (50 g); Cyclohexan (500 ml), Cyclohexan/Ethylacetat 1 : 1 (500 ml), Ethylacetat (500 ml)] das unpolarere Diastereoisomer des Ketons in einer Ausbeute von 540 mg (16 %) und das polarere Diastereoisomer des Ketons in einer Ausbeute von 730 mg (22 %) als weiße Feststoffe isoliert. Die beiden Diastereoisomeren enthielten das jeweils andere Isomer in einer Menge von ca. 10 %*, so das es nicht möglich war, die Schmelzpunkte der reinen Substanzen zu bestimmen.
* Bei längerem Stehen in chloroformischer Lösung wurde eine Isomerisierung der Diastereoisomeren (das Isomerenverhältnis veränderte sich nach 14 Tagen von unpolar/polar von ca. 1 : 9 auf ca. 1 : 1,7) beobachtet, so daß es wahrscheinlich nicht möglich ist, die Isomeren in jeweils reiner Form zu isolieren.
Unpolareres Diastereoisomer: ¹³C NMR (101 MHz, CDCl₃) δ ppm: 33.8, 36.5, 38.0, 41.4, 53.5, 60.0, 126.6, 127.1, 127.5, 127.7, 128.9, 132.9, 133.9, 137.2, 206.4 (nach Subtraktion der Signale des anderen Isomers)
Polareres Diastereoisomer: ¹³C NMR (101 MHz, CDCl₃) δ ppm: 32.4, 37.2, 38.7, 40.7, 54.4, 61.6, 127.3, 127.4, 127.6, 128.4, 128.9, 132.9, 133.8, 135.9, 206.0 (nach Subtraktion der Signale des anderen Isomers)

### (±)-(5-Dimethylamino-2-oxo-5-phenylcyclohexyl)essigsäure-methylester (unpolareres Diastereoisomer und polareres Diastereoisomer)

Diisopropylamin (2,1 ml, 15 mmol) wurde in trockenem THF (30 ml) unter Argon vorgelegt und bei -30 °C (Badtemperatur) mit einer Lösung von Butyllithium in Hexan (2,5M, 5 ml, 12,5 mmol) versetzt. Das Reaktionsgemisch wurde auf -78 °C gekühlt. Nach Erreichen dieser Temperatur wurde das 4-(Dimethylamino)-4-phenylcyclohexanon (2.17 g, 10 mmol), gelöst in trockenem THF (10 ml), innerhalb von 1 min zugetropft. Der Ansatz wurde 30 min bei -78 ° C belassen. Dann wurde der Bromessigsäure-methylester (1 ml, 10,6 mmol), gelöst in trockenem THF (5 ml), innerhalb von 1 min zugegeben. Das Reaktionsgemisch wurde 1 h bei -78 °C gerührt, dann wurde die Kühlung entfernt. Nach Erreichen von Raumtemperatur wurde der Ansatz weitere 4 h gerührt. Nach ca. 2 h trübte sich der Ansatz ein und es begann ein Niederschlag auszufallen. - Zur Aufarbeitung wurde der Ansatz vorsichtig mit Wasser (1 ml) versetzt, dann erfolgte Zugabe eines Gemisches aus gesättigter NaCl-Lösung (30 ml) und 2 N HCl (20 ml). Der Ansatz wurde 10 min gerührt, die organische Phase abgetrennt und mit gesättigter NaCl-Lösung (2 x 10 ml) extrahiert. Die vereinigten wäßrigen Phasen wurden mit Ethylacetat (3 x 20 ml) gewaschen und anschließend zur Neutralisation auf eine gesättigte NaHCO₃-Lösung (ca. 30 ml) gegeben. Die entstandene Emulsion wurde mit 2N NaOH streng basisch gemacht und mit Ethylacetat (3 x 30 ml) extrahiert. Die vereinigten organischen Phasen wurden über MgSO₄ getrocknet und anschließend eingeengt. Aus dem erhaltenen Rückstand (2,53 g) wurden durch chromatographische Reinigung [Kieselgel 60 G (10 g); Cyclohexan/Ethylacetat 2 : 1 (70 ml), Cyclohexan/ Ethylacetat 1 : 1 (70 ml), Ethylacetat (70 ml)] die beiden möglichen Diastereoisomeren des Ketons in Ausbeuten von 710 mg (unpolareres Diastereoisomer, 24 %) bzw. 260 mg (polareres Diastereoisomer, mit ca. 10 % eines Diesters verunreinigt, ca. 8 %) isoliert. Da die zweifachsubstituierten Keton nach allen bisherigen Beobachtungen nicht mit Tryptophol reagieren, konnte auf eine weitere Reinigung der polareren Verbindung verzichtet werden.

### (±)-2-Allyl-4-dimethylamino-4-phenylcyclohexanon (unpolareres Diastereoisomer und polareres Diastereoisomer)

Diisopropylamin (2,1 ml, 15 mmol) wurde in trockenem THF (30 ml) unter Argon vorgelegt und bei -30 °C (Badtemperatur) mit einer Lösung von Butyllithium in Hexan (2,5M, 6 ml, 15 mmol) versetzt. Das Reaktionsgemisch wurde auf -78 °C gekühlt. Nach Erreichen dieser Temperatur wurde das 4-(Dimethylamino)-4-phenylcyclohexanon (2,17 g, 10 mmol), gelöst in trockenem THF (20 ml), innerhalb von 15 min zugetropft. Der Ansatz wurde 30 min bei -78 ° C belassen. Dann wurde das Allylbromid (2,6 ml, 30 mmol), gelöst in trockenem THF (20 ml), innerhalb von 1 min zugegeben. Das Reaktionsgemisch wurde weitere 10 min bei -78 °C gerührt, dann wurde die Kühlung entfernt. Nach Erreichen von Raumtemperatur wurde der Ansatz weitere 18 h gerührt. - Zur Aufarbeitung wurde der Ansatz vorsichtig mit Wasser (1 ml) versetzt, dann erfolgte Zugabe von gesättigter NH₄Cl-Lösung (40 ml). Die organische Phase wurde abgetrennt und die wäßrige Phase mit Ethylacetat (3 x 20 ml) extrahiert. Die vereinigten organische Phasen wurden mit gesättigter NH₄Cl-Lösung (2 x 40 ml) gewaschen. Anschließend wurden die organischen Phasen mit 1 N HCl (30 ml) versetzt und gründlich ausgeschüttelt. Die saure, wäßrige Phase wurde abgetrennt, die organische Phase mit Wasser (2 x 20 ml) gewaschen. Die vereinigten wäßrigen Phasen wurden mit Ethylacetat (1 x 30 ml) gewaschen. Dann wurde die wäßrige Lösung auf 2N NaOH (70 ml) gegeben. Aus der alkalischen Lösung fiel ein Öl aus. Die entstandene Mischung wurde mit Ethylacetat (3 x 30 ml) extrahiert. Die vereinigten organischen Phasen wurden über Na₂SO₄ getrocknet und anschließend eingeengt. Aus dem erhaltenen Rückstand (2 g) wurde durch chromatographische Reinigung [Kieselgel 60 (50 g); Cyclohexan/Ethylacetat 1 : 10 (700 ml)] das unpolarere Diastereoisomer des Ketons in einer Ausbeute von 540 mg (22 %) isoliert. Das polarere Diastereoisomer wurde in einer Ausbeute von 470 mg (19 %) erhalten.

### (±)-4-Dimethylamino-2-fluor-4-phenylcyclohexanon

Zu einer Lösung des 4-(Dimethylamino)-4-phenylcyclohexanons (5,00 g, 23,01 mmol) in absolutem Tetrahydrofuran (70 ml) wurde in einer Argonatmosphäre bei -78 °C Lithiumdiisopropylamin (16,6 ml, 29,91 mmol, 1,8M in Tetrahydrofuran, Heptan, Ethylbenzol) getropft. Die Mischung wurde bei -78 °C 10 min gerührt und anschließend auf Raumtemperatur erwärmt. Dann wurde die Reaktionsmischung wieder auf -78 °C gekühlt und N-Fluor-bis(phenylsulfonyl)amin (NFSI, 9,43 g, 29,91 mmol) in absolutem Tetrahydrofuran (100 ml) hinzugetropft. Die Reaktionsmischung wurde langsam auf Raumtemperatur erwärmt und 4 h gerührt. Dann wurden im Vakuum die flüchtigen Bestandteile vollständig entfernt. Anschließend wurde der Rückstand mit Ethylacetat (100 ml) und Wasser (80 ml) versetzt. Die Phasen wurden getrennt. Die wäßrige Phase wurde mit Ethylacetat (3 x 40 ml) extrahiert. Die vereinigten organischen Phasen wurden über Natriumsulfat getrocknet, filtriert und im Vakuum von flüchtigen Bestandteilen befreit. Das verbliebene, hellgelbe Harz wurde mit Ethylacetat (50 ml) auf grobes Kieselgel aufgezogen und chromatographisch getrennt [Kieselgel (150 g), Cyclohexan/Ethylacetat 3 : 1 (500 ml), 2 : 1 (500 ml), 1 : 1 (500 ml), 1 : 2 (1000 ml)]. Es wurden 663 mg (2,82 mmol, 12 %) des unpolaren Ketons und 1488 mg (6,32 mmol, 27 %) des polaren Ketons als farblose, mikrokristalline Pulver isoliert.
¹³C{¹H}-NMR (101 MHz, DMSO-*D*₆) δ ppm (unpolareres Diastereoisome): 32.9 (1 C), 35.2 (1 C), 37.9 (2 C), 39.3 (1 C, d, J = 18 Hz, unter DMSO-*D*₅), 61.2 (1 C, d, J = 11 Hz), 90.0 (1 C, d, J = 185 Hz), 126.7 (2 C), 126.9 (1 C), 127.5 (2 C), 136.3 (1 C), 205.0 (1 C, d, J = 13 Hz) ¹³C{¹H}-NMR (101 MHz, DMSO-*D*₆) δ ppm (polareres Diastereoisomer): 32.0 (1 C), 34.8 (1 C), 38.3 (2 C), 39.4 (1 C, d, J = 18 Hz, unter DMSO-*D*₅), 61.4 (1 C, J = 11 Hz), 89.4 (1 C, d, J = 187 Hz), 127.1 (1 C), 127.3 (2 C), 128.0 (2 C), 135.4 (1 C), 204.3 (1 C, d, J = 14 Hz)

### 2-(5-Dimethylamino-2-oxo-5-phenylcyclohexylmethyl)isoindolin-1,3-dion (eins von zwei möglichen Diastereoisomeren)

Lithiumdüsopropylamid-Lösung (1,8M in Hexan, 6 ml, 10 mmol) wurde in absolutemTetrahydrofuran (10 ml) vorgelegt. Die Lösung wurde auf -78 °C gekühlt. Nach Erreichen dieser Temperatur wurde das 4-(Dimethylamino)-4-phenylcyclohexanon (1,1 g, 5 mmol), gelöst in trockenem THF (5 ml), innerhalb von 1 min zugetropft. Der Ansatz wurde 30 min bei -78 °C belassen. Dann wurde das *N*-(Brommethyl)phthalimid (3,6 g, 15 mmol) gelöst in trockenem THF (20 ml), innerhalb von 1 min zugegeben. Das Reaktionsgemisch wurde 1 h bei -78 °C gerührt, dann wurde die Kühlung entfernt. Nach Erreichen von Raumtemperatur wurde der Ansatz weitere 18 h gerührt. - Zur Aufarbeitung wurde der Ansatz vorsichtig mit Wasser (1 ml) versetzt, dann erfolgte die Zugabe von gesättigter NH₄Cl-Lösung (40 ml). Die organische Phase wurde abgetrennt und die wäßrige Phase mit Ethylacetat (3 x 30 ml) extrahiert. Die vereinigten organischen Phasen wurden mit gesättigter NH₄Cl-Lösung (1 x 40 ml) gewaschen. Anschließend wurden die organischen Phasen mit 1N HCl (50 ml) versetzt und gründlich ausgeschüttelt. Die saure, wäßrige Phase wurde abgetrennt, die organische Phase mit Wasser (2 x 20 ml) gewaschen. Die vereinigten wäßrigen Phasen wurden mit Ethylacetat (1 x 30 ml) gewaschen. Dann wurde die wäßrige Lösung auf 2N NaOH (70 ml) gegeben. Aus der alkalischen Lösung fiel ein Öl aus. Die entstandene Mischung wurde mit Ethylacetat (3 x 30 ml) extrahiert. Die vereinigten organischen Phasen wurden über Na₂SO₄ getrocknet und anschließend eingeengt. Aus dem erhaltenen Rückstand (2 g) wurden durch chromatographische Reinigung [Kieselgel 60 G (50 g); Cyclohexan/Ethylacetat 1 : 1 (700 ml)] eins der zwei möglichen Diastereoisomeren des Ketons in einer Ausbeute von 223 mg (12 %) isoliert.

### (±)-3-(5-Dimethylamino-2-oxo-5-phenylcyclohexyl)propionitril (eins von zwei möglichen Diastereoisomeren)

Das 4-(Dimethylamino}-4-phenylcyclohexanon (2,17 g, 10 mmol) wurde zu einer Lösung von Cyclohexylamin (109 mg, 1 mmol), Eisessig (26 mg, 0,43 mmol) und 4-Methoxyphenol (26 mg, 0,21 mmol) in 10 ml Toluol gegeben. Die Mischung wurde auf 90 °C (Badtemperatur) erhitzt und innerhalb von 2 h mit Acrylnitril (4 ml, 60,8 mmol) versetzt. Anschließend wurde der Ansatz auf 120 °C erhitzt. Nach 3 h wurde die Heizung entfernt und der Ansatz nach Erreichen von RT mit 1 N NaOH (20 ml) versetzt. Das erhaltene Gemisch wurde mit Ethylacetat (3 x 20 ml) extrahiert. Die vereinigten organischen Phasen wurden mit Wasser gewaschen und anschließend über MgSO₄ getrocknet. Der nach Entfernung des Lösungsmittels erhaltene Rückstand (2,2 g) wurde beim Stehen fest und bestand hauptsächlich aus dem Ausgangsketon und einem im Verhältnis zu diesem unpolareren Produkt. Durch chromatographische Reinigung [Kieselgel 60 G (10 g); Cyclohexan/Ethylacetat 1 : 1 (120 ml)] wurde eins der beiden möglichen Diastereoisomeren des Ketons in einer Ausbeute von 400 mg (14 %) als Öl erhalten.

### Beispiel Nr. 1 und Beispiel Nr.2:

### Stufe 1:

N,N,2-Trimethyl-4-phenyl-4',9'-dihydro-3'H-spiro[cyclohexan-1,1'-pyrano[3,4-b]indol]-4-amin (unpolares Diastereomer und polares Diastereomerengemisch)

(±)-4-Dimethylamino-2-methyl-4-phenylcyclohexanon (461 mg, 2 mmol) wurde zusammen mit Tryptophol (322 mg, 2 mmol) in DCM (100 ml) gelöst und mit Trifluormethansulfonsäure (0,19 ml, 2,14 mmol) versetzt. Es wurde 20 h bei RT gerührt. Das Reaktionsgemisch wurde mit 2N NaOH (2 ml) und H2O (2 ml) versetzt und 20 min gerührt. Die organische Phase wurde zunächst mit 2N NaOH (5 ml), dann mit H2O (5 ml) gewaschen und über Na2SO4 getrocknet. Anschließend wurde das Lösungsmittel im Vakuum entfernt. Der erhaltene halbfeste Rückstand wurde in 10 ml Ethanol aufgenommen und 2 h auf 5 °C gekühlt. Der dabei ausgefallene Feststoff wurde verworfen. Der in der Mutterlauge verbliebene Rückstand wurde durch Säulenchromatographie an Kieselgel (50 g, EE/Ethanol= 4:1) gereinigt.
Ausbeute (unpolares Diastereomer): 375 mg (50 %), weisser Feststoff
*Schmelzpunkt:* 190-210 °C
*Ausbeute* (polareres Diastereomerengemisch): 83 mg (11 %), zu etwa 20 % mit einem weiteren Diastereoisomer verunreinigt.

### Stufe 2:

N,N,2-Trimethyl-4-phenyl-4',9'-dihydro-3'H-spiro[cyclohexan-1,1'-pyrano[3,4-b]indol]-4-amin; 2-hydroxypropan-1,2,3-tricarboxylat (1:1) (Beispiel Nr. 1, unpolares Diastereomer)

(±)-N,N,2-Trimethyl-4-phenyl-4',9'-dihydro-3'H-spiro[cyclohexan-1,1'-pyrano[3,4-b]indol]-4-amin (240 mg, 0,64 mmol) wurde in siedendem Ethanol (100 ml) gelöst. Anschließend wurde Citronensäure (124 mg, 0,65 mmol), gelöst in Ethanol (10 ml), hinzugegeben und das Reaktionsgemisch auf ca. 5 °C abgekühlt. ach 60 h bei dieser Temperatur wurde der ausgefallene Feststoff abgetrennt.
*Ausbeute:* 135 mg (37 %), hellgelber kristalliner Feststoff
*Schmelzpunkt:* 221-223 °C
*HPLC*/*MS-Analytik*: Rt = 2,87 min; m/z = 374,9.

N,N,2-Trimethyl-4-phenyl-4',9'-dihydro-3'H-spiro[cyclohexan-1,1'-pyrano[3,4-b]indol]-4-amin; 2-hydroxypropan-1,2,3-tricarboxylat (1:1) (Beispiel Nr. 2, polares Diastereomerengemisch)

Das polarere Spiroethergemisch (83 mg, 0,22 mmol) wurde in siedendem Ethanol (20 ml) gelöst. Anschließend wurde Citronensäure (43 mg, 0,22 mmol), gelöst in Ethanol (10 ml), hinzugefügt und das Reaktionsgemisch 1 h bei RT gerührt. Nach Entfernung des Lösungsmittels wurde Citrat als ein glasiger Festsoff erhalten.
*Ausbeute:* 124 mg (100%)
*Schmelzpunkt:* 64-83 °C
*HPLCIMS-Analytik:* Rt = 2,63 min; m/z = 375,0 (80 %) und Rt = 2,76min; m/z = 374,9 (20 %).

### Beispiel Nr. 3:

### Stufe 1:

2-Methyl-4-(dimethylamino)1-4-phenyl-spiro[cyclohexan-1,8'-(5,6,8,9-Tetrahydro-pyrano[3,4-b]-7-aza-indol)]

4-Dimethylamino-2-methyl-4-phenylcyclohexanon (0,42 g, 1,85 mmol) wurde zusammen mit 2-(1H-Pyrrolo[2,3-b]pyridin-3-yl)ethanol (0,30 g, 1,85 mmol) unter Stickstoff in Dichlormethan (5 ml) ultratrocken vorgelegt. Anschließend erfolgte eine schnelle Zugabe von Trimethylsilyltrifluormethansulfonat (1,43 ml, 7,4 mmol). Das Gemisch wurde 7 Tage bei Raumtemperatur gerührt. Nach Zugabe von Dichlormethan wurde das Gemisch mit 1 M Na2CO3-Lösung basisch gestellt und 20 min gerührt. Die organische Phase wurde abgetrennt und die wässrige Phase mit Dichlormethan (3x) extrahiert. Die vereinigten organischen Extrakte wurden mit gesättigter NaCl-Lösung gewaschen und über MgSO4 getrocknet. Nach Filtration des Trockenmittels wurde das Lösungsmittel am Rotationsverdampfer entfernt. Der erhaltene Feststoff wurde mit Methanol (5 ml) versetzt und das Gemisch für 2 h bei Raumtemperatur gerührt. Der Feststoff wurde abgesaugt, mit wenig Methanol gewaschen und am Ölpumpenvakuum bei 50°C getrocknet.
*Ausbeute:* 0,2 g (28%)

### Stufe 2:

2-Methyl-4-(dimethylamino)1-4-phenyl-spiro[cyclohexan-1,8'-(5,6,8,9-Tetrahydro-pyrano[3,4-b]-7-aza-indol)]; 2-hydroxypropan-1,2,3-tricarboxylat (1:1) (Beispiel Nr. 3, ein Diastereomer)

(±)-2-Methyl-4-(dimethylamino)1-4-phenyl-spiro[cyclohexan-1,8'-(5,6,8,9-Tetrahydro-pyrano[3,4-b]-7-aza-indol)] (0,194 g, 0,52 mmol) wurde in heißem Ethanol (6 ml) aufgeschlämmt und mit einer ebenfalls heißen Lösung von Citronensäure (0,099 g) in Ethanol (3 ml) versetzt. Die Lösung wurde 3 h bei Raumtemperatur gerührt. Anschließend wurde der Niederschlag abgesaugt, portionsweise mit Ether gewaschen und am Hochvakuum bei 60°C getrocknet.
*Ausbeute:* 0,215 g (73%).
*HPLC*/*MS-Analytik:* Rt = 2,0 min; m/z = 376,0.

### Beispiel Nr. 4:

2-Benzyl-N,N-dimethyl-4-phenyl-4',9'-dihydro-3'H-spiro[cyclohexane-1,1'-pyrano[3,4-b]indol]-4-amin (Beispiel Nr. 4, ein Diastereomer)

(±)-2-Benzy)-4-dimethylamino-4-phenylcyclohexanon (349 mg, 1,14 mmol) wurde zusammen mit Tryptophol (184 mg, 1,14 mmol) in Dichlormethan (30 ml) gelöst und mit Trifluormethansulfonsäure-trimethylsilylester (0,24 ml, 1,24 mmol) versetzt. Der Ansatz wurde 3 h bei RT gerührt. Nach einiger Zeit (ca. 1 h) begann ein Niederschlag auszufallen. Zur Aufarbeitung wurde das Reaktionsgemisch mit 2N NaOH (20 ml) versetzt und 20 min gerührt. Dabei ging der Niederschlag in Lösung. Die organische Phase wurde abgetrennt und die wäßrige Phase mit Dichlormethan (4 x 20 ml) extrahiert. Die vereinigten organischen Phasen wurden mit MgSO4 getrocknet. Anschließend wurde das Lösungsmittel im Vakuum eingedampft. Aus dem erhaltenen Rückstand (535 mg) wurde durch chromatographische Reinigung [Kieselgel 60 G (10 g); Ethylacetat 300 ml] einer der möglichen diastereoisomeren Spiroether isoliert.
*Ausbeute:* 392 mg (76 %)
*Schmelzpunkt :* 122-125 °C (aus Toluol).
13C NMR (101 MHz, CDCl3) δ ppm: 22.5, 27.8, 31.7, 31.9, 36.3, 38.2, 44.9, 60.0, 61.7, 74.7, 109.5, 110.8, 118.0, 119.4, 121.6, 125.7, 126.6, 127.0, 127.6, 127.8, 128.0, 129.0, 135.8, 136.3, 137.0, 141.2.

### Beispiel Nr. 5:

### Stufe 1:

2-Benzyl-N,N-dimethyl-4-phenyl-2',3',4',9'-tetrahydrospiro[cyclohexan-1,1'-pyrido[3,4-b]indol]-4-amin

(t)-2-Benzyl-4-dimethylamino-4-phenylcyclohexanon (307 mg, 1 mmol) wurde zusammen mit Tryptamin (160 mg, 1 mmol) in Methanol gelöst und 24 h bei RT gerührt. Anschließend wurde das Lösungsmittel im Vakuum entfernt und der Rückstand in 1,2-Dichlorethan (20 ml) gelöst. Die Mischung wurde mit Trifluoressigsäure (1,7 ml, 22,8 mmol) versetzt und 24 h bei RT gerührt. - Zur Aufarbeitung wurde das Reaktionsgemisch mit 2N NaOH (20 ml) versetzt und 20 min gerührt. Die organische Phase wurde abgetrennt und die wäßrige Phase mit Dichlormethan (4 x 20 ml) extrahiert. Die vereinigten organischen Phasen wurden mit MgS04 getrocknet. Anschließend wurde das Lösungsmittel im Vakuum eingedampft. Aus dem erhaltenen Rückstand (260 mg) wurde durch chromatographische Reinigung [Kieselgel 60 G (10 g); Ethylacetat/Ethanol 1 : 1 (80 ml), Methanol (60 ml)] eins der möglichen Diastereoisomeren isoliert.
*Ausbeute:* 80 mg (17 %), glasiger Feststoff (Reinheit ca. 95 %)
*¹³C NMR* (101 MHz, CDCI3-D6) δ ppm: 23.1, 28.4, 32.0, 33.2, 36.7, 38.1, 39.7, 45.1, 55.6, 61.9, 110.7, 110.9, 117.9, 119.2, 121.4, 125.9, 126.6, 127.5, 127.8, 127.9, 128.1, 129.1, 135.5, 135.6, 138.3, 141.0

### Stufe 2:

2-Benzyl-N,N-dimethyl-4-phenyl-2',3',4',9'-tetrahydrospiro[cyclohexan-1,1'-pyrido[3,4-b]indol]-4-amin 2-Hydroxypropan-1,2,3-tricarboxylat (1:1) (Beispiel Nr. 5, ein Diastereomer)

2-Benzyl-N,N-dimethyl-4-phenyl-2',3',4',9'-tetrahydrospiro[cyclohexan-1,1'-pyrido[3,4-b]indol]-4-amin (eins von 4 möglichen racemischen Diastereoisomerenpaaren, 62 mg, 0,14 mmol) wurde in 2-Propanol (3 ml) in der Siedehitze gelöst und mit einer heißen Lösung von Citronensäure [40 mg, 0,2 mmol, in heißem Isopropanol (1 ml)] versetzt. Es fiel sofort ein Niederschlag aus. Der Ansatz wurde zur Vervollständigung der Fällung 2 h bei 5 °C belassen, dann wurde der Feststoff mittels einer Fritte abgetrennt und getrocknet.
*Ausbeute:* 48 mg (53 %), Reinheit ca. 90 %
*Schmelzpunkt:* ab 148 °C
*¹³C NMR* (101 MHz, DMSO-D6) δ ppm: 21.8, 23.1, 26.0, 29.8, 32.5, 35.6, 37.3, 43.1, 43.9, 56.0, 61.9, 71.4, 108.6, 111.1, 117.4, 118.2, 120.6, 126.0, 126.6, 128.0, 128.2, 128.4, 128.6, 129.0, 131.7, 136.0, 137.0, 139.9, 171.2, 176.4

### Beispiel Nr. 6:

2-(3-Fluorbenzyl)-N,N-dimethyl-4-phenyl-4',9'-dihydro-3'H-spiro[cyclohexan-1,1'-pyrano[3,4-b]indol]-4-amin (Beispiel Nr. 6, ein Diastereomer)

(±)-2-(3-Fluorbenzyl)-4-dimethylamino-4-phenylcyclohexanon (325 mg, 1 mmol) wurde zusammen mit Tryptophol (161 mg, 1 mmol) in Dichlormethan (30 ml) gelöst und mit Trifluormethansulfonsäure-trimethylsilylester (0,22 ml, 1,14 mmol) versetzt. Der Ansatz wurde 2,5 h bei RT gerührt. Nach einiger Zeit (ca. 1 h) begann ein Niederschlag auszufallen. - Zur Aufarbeitung wurde das Reaktionsgemisch mit 2N NaOH (20 ml) versetzt und 20 min gerührt. Dabei ging der Niederschlag in Lösung. Die organische Phase wurde abgetrennt und die wäßrige Phase mit Dichlormethan (4 x 20 ml) extrahiert. Die vereinigten organischen Phasen wurden mit MgS04 getrocknet. Anschließend wurde das Lösungsmittel im Vakuum eingedampft. Aus dem erhaltenen Rückstand (352 mg) wurde durch chromatographische Reinigung [Kieselgel 60 G (10 g); Ethylacetat (150 ml)] eins der möglichen Diastereoisomeren isoliert.
*Ausbeute*: 240 mg (51 %)
*Schmelzpunkt*: ab 108 °C
¹³*C NMR* (101 MHz, CDCl₃) δ ppm : 22.5, 27.6, 31.6, 32.1, 36.1, 38.2, 44.7, 60.0, 61.7, 74.6, 109.6, 110.9, 112.5, 112.7, 115.5, 115.7, 118.0, 119.5, 121.7, 124.8, 126.7, 127.0, 127.9, 128.0, 129.3, 129.4, 135.7, 136.3, 136.7, 143.8, 143.9, 161.5, 164.0

### Beispiel Nr. 7

### Stufe 1:

6'-Fluor-2-(3-fluorbenzyl)-*N,N*-dimethyl-4-phenyl-4',9'-dihydrospiro[cyclohexan-1,1'-pyrano-[3,4-b]indol]-4-amin (Beispiel Nr. 7, eins von 4 möglichen racemischen Diastereoisomerenpaaren)

Das (±)-2-(3-Fluorbenzyl-4-dimethylamino-4-phenylcyclohexanon (325 mg, 1 mmol, eins von zwei möglichen racemischen Diastereoisomeren) wurde zusammen mit 5-Fluortryptophof (179 mg, 1 mmol) in Dichlormethan (30 ml) gelöst und mit Trifluormethansulfonsäure-trimethylsilylester (0,22 ml, 1,14 mmol) versetzt. Die klare hellgelbe Lösung färbte sich braun. Der Ansatz wurde 2 h bei RT gerührt. Zur Aufarbeitung wurde das Reaktionsgemisch mit 1 N NaOH (30 ml) versetzt und 15 min gerührt. Die organische Phase wurde abgetrennt und die wäßrige Phase mit Dichlormethan (2 x 20 ml) extrahiert. Die vereinigten organischen Phasen wurden mit Na₂SO₄ getrocknet. Anschließend wurde das Lösungsmittel im Vakuum eingedampft. Der erhaltene Rückstand (477 mg) sollte für die Chromatographie in Ethylacetat gelöst werden. Dabei fiel ein weißer Feststoff (86 mg) aus, der mittels NMR und im LC/MS als eins der 4 möglichen Diastereoisomeren identifiziert wurde. Das Filtrat wurde eingeengt und chromatographisch gereinigt [Kieselgel 60 (20 g); Ethylacetat (600 ml)]. Das erhaltene Produkt (242 mg) wurde mit dem Feststoff vereinigt. Eins der 4 möglichen Diastereoisomeren wurde so in einer Gesamtausbeute von 328 mg (67 %) mit einem Schmelzpunkt von 133-136 °C erhalten.
Beispiel Nr. 7: ¹³C-NMR (101 MHz, CDCl₃) δ ppm : 22.4, 27.5, 31.6, 32.2, 36.1, 38.2, 44.6, 59.9, 61.6, 74.5, 103.0, 103.3, 109.6, 109.9, 111.3, 111.4, 112.5, 112.7, 115.5, 115.7, 124.7, 126.7, 127.3, 127.4, 127.9, 128.0, 129.4, 132.2, 136.4, 138.8, 143.8, 156.7, 159.0, 161.5, 164.0

### Beispiel Nr. 8 und Beispiel Nr. 9 und Beispiel Nr. 10

### Stufe 1:

2-(3-Fluorbenzyl)-*N,N*-dimethyl-4-phenyl-2',3',4',9'-tetrahydrospiro[cyclohexan-1,1'-pyrido[3,4-b]indol]-4-amin (Beispiel Nr. 8, polareres Isomer, Beispiel Nr. 9, unpolareres Isomer, Beispiel Nr. 10, zweitpolareres Isomer, 3 von 4 racemischen Diastereoisomerenpaaren)

Das (±)-2-(3-Fluorbenzyl)-4-dimethylamino-4-phenylcyclohexanon (478 mg, 1,47 mmol, eins von zwei möglichen Diastereoisomeren) wurde zusammen mit Tryptamin (235 mg, 1,47 mmol) in Methanol (20 ml) gelöst und 24 h bei RT gerührt. Anschließend wurde das Lösungsmittel im Vakuum entfernt und der Rückstand in 1,2-Dichlorethan (20 ml) gelöst. Die Mischung wurde mit Trifluoressigsäure (1,5 ml, 20,3 mmol) versetzt und 24 h bei RT gerührt. - Zur Aufarbeitung wurde das Reaktionsgemisch mit 2N NaOH (24 ml) versetzt und 20 min gerührt. Die organische Phase wurde abgetrennt und die wäßrige Phase mit Dichlormethan (4 x 20 ml) extrahiert. Die vereinigten organischen Phasen wurden mit MgSO₄ getrocknet. Anschließend wurde das Lösungsmittel im Vakuum entfernt. Aus dem erhaltenen Rückstand (510 mg) wurde durch chromatographische Reinigung [Kieselgel 60 (30g): Methanol (1000 ml)] ein relativ polares racemisches Diastereoisomerenpaar (eins der vier möglichen Isomeren) in einer Ausbeute von 130 mg (18 %, Öl, welches beim Stehen fest wurde) in über 95 % Reinheit (polareres Isomer) als glasiger Feststoff isoliert.
Durch eine zweite chromatographische Trennung der unpolaren Fraktionen [Kieselgel 60 G (10g); Ethylacetat (140 ml), Ethylacetat/Ethanol 1 : 1 (30 ml)] konnten zwei weitere der vier möglichen racemischen Diastereoisomerenpaare isoliert werden. Aus den Fraktionen, die das unpolarere Isomer enthielten, wurde durch Auswaschen mit Methanol (1 ml) unumgesetztes Keton abgetrennt. Das racemische Diastereoisomerenpaar wurde so in einer Ausbeute von 29 mg (4 % Ausbeute, unpolareres Isomer) mit einem Schmelzpunkt ab 258 °C erhalten. Ein zweitpolareres racemisches Diastereoisomerenpaar wurde ebenfalls durch Anreiben der entsprechenden Fraktionsrückstände mit Methanol (ca. 1 ml) in kristalliner Form (Schmelzpunkt ab 138 °C unter Kristallumwandlung) in einer Ausbeute von 8 mg (1,7 %, zweitpolareres Isomer) erhalten.
Beispiel Nr. 8: ¹³C-NMR (101 MHz, CDCl₃) δ ppm: 23.0, 28.2, 32.2, 33.0, 36.5, 38.1, 39.7, 44.7, 50,5, 61.7, 110.7, 112.6, 112.8, 115.6, 115.8, 117.9, 119.1, 121.3, 124.9, 126.6, 127.3, 127.8, 128.0, 129.4, 129.5, 135.6, 135.7, 138.1, 143.8, 143.9, 161.5, 163.9
Beispiel Nr. 9: ¹³C-NMR (101 MHz, CDCl₃ δ ppm: 23.2, 28.5, 31.6, 32.3, 36.6, 37.8, 39.8, 42.8, 55.6, 59.3, 110.6, 110.9, 112.6, 112.8, 115.7, 115.9, 118.0, 119.2, 121.4, 124.8, 126.6, 126.7, 127.3, 127.6, 129.5, 129.6, 135.8, 139.0, 139.1, 144.3, 144.4, 161.5, 164.0 Beispiel Nr. 10: ¹³C-NMR (101 MHz, CDCl₃) δ ppm: 23.1, 31.2*, 36.4, 36.9, 38.1, 39.7, 45.4, 56.1, 61.5*, 111.2, 112.8, 113.0, 115.7, 115.8, 118.0, 119.2, 121.6, 124.78, 124.81, 126.6, 127.2, 127.9, 128.1, 129.6. 129.7, 135.9, 143.7, 161.7, 164.1
*stark verbreiterte Signale

### Beispiel Nr. 11

### Stufe 1:

*N,N*-Dimethyl-4-phenyl-2-(phenylthio)-4',9'-dihydro-3'*H*-spiro[cyclohexan-1,1'-pyrano[3,4-*b*]indol]-4-amin (Beispiel Nr. 11, eins von 4 möglichen racemischen Diastereoisomerenpaaren)

Ein Gemisch des (±)-4-Dimethytamino-4-pheny)-2-thiopheny)cyclohexanons (300 mg, 0,92 mmol) wurde zusammen mit Tryptophol (148 mg, 0,92 mmol) in Dichlormethan (50 ml) gelöst und mit Trifluormethansulfonsäure-trimethylsilylester (0,2 ml, 1,1 mmol) versetzt. Der Ansatz wurde 20 h bei RT gerührt. - Zur Aufarbeitung wurde das Reaktionsgemisch mit 2N NaOH (10 ml) versetzt und 2 h gerührt. Die organische Phase wurde abgetrennt und die wäßrige Phase mit Dichlormethan (4 x 20 ml) extrahiert. Die vereinigten organischen Phasen wurden mit MgSO₄ getrocknet. Anschließend wurde das Lösungsmittel im Vakuum entfernt. Aus dem erhaltenen Rückstand (420 mg) wurde durch chromatographische Reinigung [Kieselgel 60 G (10 g); Cyclohexan/Ethylacetat 4 : 1 (150 ml)] eins der vier möglichen Diastereoisomeren als glasiger Feststoff isoliert, der nach Anreiben mit Ethanol (2 ml) fest wurde. Das Produkt konnte so in einer Ausbeute von 79 mg (18 %) mit einem Schmelzpunkt von 228-233 °C (ab 120 °C Kristallumwandlung) isoliert werden.
Beispiel Nr. 11: ¹³C-NMR (101 MHz, CDCl₃) δ ppm: 22.4, 26.9. 28.0, 30.4, 36.5, 38.0, 54.4, 60.48, 60.52, 75.0, 109.5, 111.0, 118.2, 119.4, 121.6, 126.7, 126.9, 127.0, 127.4, 128.6, 133.0, 135.8, 136.1, 136.9, 138.6

### Beispiel Nr. 12 und Beispiel Nr. 13

### Stufe 1:

*N,N*-Dimethyl-4-phenyl-2-(phenylthio)-2',3',4',9'-tetrahydrospiro[cyclohexan-1,1'-pyrido[3,4-blindol]-4-amin (Beispiel Nr. 12, unpolareres Isomer und Beispiel Nr. 13, polareres Isomer, zwei von 4 möglichen racemischen Diastereoisomerenpaaren)

Das polarere 4-Dimethylamino-4-phenyl-2-thiophenylcyclohexanon (277 mg, 0,85 mmol) wurde zusammen mit Tryptamin (136 mg, 0,85 mmol) in Methanol (20 ml) gelöst und 24 h bei RT gerührt. Anschließend wurde das Lösungsmittel im Vakuum entfernt und der Rückstand in 1,2-Dichlorethan (20 ml) gelöst. Die Mischung wurde mit Trifluoressigsäure (1,5 ml, 20,3 mmol) versetzt und 24 h bei RT gerührt. - Zur Aufarbeitung wurde das Reaktionsgemisch mit 2N NaOH (20 ml) versetzt und 30 min gerührt. Die organische Phase wurde abgetrennt und die wäßrige Phase mit Dichlormethan (3 x 10 ml) extrahiert. Die vereinigten organischen Phasen wurden mit MgSO₄ getrocknet. Anschließend wurde das Lösungsmittel im Vakuum eingedampft. Aus dem erhaltenen Rückstand (410 mg) wurden durch chromatographische Reinigung [Kieselgel 60 G (10 g); Ethylacetat/Ethanol 3 : 1 (150 ml] zwei der möglichen Diastereoisomeren erhalten. Ein unpolareres Isomerenpaar fiel in einer Ausbeute von 72 mg (17 %) als Feststoff mit einem Schmelzpunkt von 190-192 °C (nach Anreiben mit MeOH) an. Ein polareres Isomerenpaar wurde durch Anreiben der entsprechenden Fraktionsrückstände mit Methanol (1 ml) in einer Ausbeute von 129 mg (32 %) und einem Schmelzpunkt von 171-173 °C erhalten.
Beispiel Nr. 12:¹³C-NMR (101 MHz, CDCl₃) δ ppm: 23.3, 29.9, 35.6, 38.1, 38.9, 39.6, 55.4, 56.2, 62.1, 111.0, 111.3, 118.1, 119.1, 121.8, 126.4, 127.2, 127.4, 127.8, 128.2, 128.8, 132.2, 132.6, 134.4, 136.0
Beispiel Nr. 13:¹³C-NMR (101 MHz, CDCl₃) δ ppm; 23.0, 27.0, 33.2, 35.2, 38.3, 39.6, 55.7, 55.8, 62.8, 110.7, 110.9, 118.0, 119.0, 121.3, 126.9, 127.2, 127.4, 127.9, 128.1, 128.4, 133.7, 134.2, 135.5, 136.3, 137.5

### Beispiel Nr. 14

### Stufe 1:

2-(4-(Dimethylamino)-4-phenyl-4',9'-dihydro-3'H-spiro[cyclohexan-1,1'-pyrano[3,4-b]indol]-2-yl)essigsäure-methylester (Beispiel Nr. 14, unpolareres Diastereoisomer aus unpolarerem Keton, eins von 4 möglichen racemischen Diastereoisomerenpaaren)

Der unpolarere (5-Dimethylamino-2-oxo-5-phenylcyclohexyl)essigsäure-methylester (310 mg, 1,07 mmol) wurde zusammen mit Tryptophol (172 mg, 1,07 mmol) in Dichlormethan (30 ml) gelöst und mit Trifluormethansulfonsäure-trimethylsilylester (0,2 ml, 1,1 mmol) versetzt. Nach 3 h wurde eine DC Probe genommen, die einen weitgehenden Umsatz anzeigte. Der Ansatz wurde 20 h bei RT gerührt. - Zur Aufarbeitung wurde das Reaktionsgemisch mit 2N NaOH (10 ml) versetzt und 20 min gerührt. Die organische Phase wurde abgetrennt und die wäßrige Phase mit Dichlormethan (3 x 20 ml) extrahiert. Die vereinigten organischen Phasen wurden mit MgSO₄ getrocknet. Anschließend wurde das Lösungsmittel im Vakuum entfernt. Der erhaltene Feststoff wurde mit Methanol (3 ml) versetzt. Die Mischung wurde zum Sieden erhitzt, wobei der Feststoff nur teilweise in Lösung ging. Das Gemisch wurde zur Vervollständigung der Fällung 2 h bei 5 °C belassen. Anschließend wurde der Feststoff mittels einer Fritte abgetrennt und im Vakuum getrocknet. Eins der 4 möglichen racemischen Diastereoisomerenpaare konnte so in einer Ausbeute von 317 mg (68 %) mit einem Schmelzpunkt von 262-268 °C isoliert werden.
Beispiel Nr. 14: ¹³C-NMR (101 MHz, CDCl) δ ppm : 22.4. 28.2, 29.5, 34.1, 34.8, 37.0, 38.1, 51.3, 59.4, 59.8, 74.4, 109.2, 111.1, 118.0, 119.3, 121.6, 126.6, 126.8, 127.3, 135.9, 137.1, 139.2, 174.3

### Beispiel Nr. 15

### Stufe 1:

2-(4-(Dimethylamino)-4-phenyl-4',9'-dihydro-3'H-spiro[cyclohexan-1,1'-pyrano[3,4-b]indol]-2-yl)essigsäure-methylester (Beispiel Nr. 15, polareres Diastereoisomer aus polarerem Keton, eins von 4 möglichen racemischen Diastereoisomerenpaaren)

Der polarere (5-Dimethylamino-2-oxo-5-phenylcyclohexyl)essigsäure-methylester (350 mg, mit ca. 10 % eines Diesters verunreinigt, berechnet auf reinen Monoester, 1,2 mmol) wurde zusammen mit Tryptophol (194 mg, 1,2 mmol) in Dichlormethan (20 ml) gelöst und mit Trifluörmethansulfonsäure-trimethylsilylester (0,24 ml, 1,33 mmol) versetzt. Der Ansatz wurde 20 h bei RT gerührt. Der aus der Lösung ausgefallene Niederschlag wurde mittels einer Fritte abgetrennt und - da es sich um das Trifluormethansulfonsäuresalz des Spiroethers handelte, 2 h in einem Gemisch aus Dichlormethan (10 ml) und 2N NaOH (5 ml) bei RT gerührt. Die organische Phase wurde abgetrennt und die wäßrige Phase mit Dichlormethan (3 x 10 ml) extrahiert. Die vereinigten organischen Phasen wurden mit MgSO₄ getrocknet. Anschließend wurde das Lösungsmittel im Vakuum entfernt. Eine erste Charge eines Isomeren des Spiroethers wurde so in einer Ausbeute von 67 mg mit einem Schmelzpunkt von 253-269 °C erhalten. Zur Vervollständigung der Aufarbeitung wurde die als Mutterlauge erhaltene Reaktionslösung mit 2N NaOH (10 ml) versetzt und 20 min gerührt. Die organische Phase wurde abgetrennt und die wäßrige Phase mit Dichlormethan (3 x 20 ml) extrahiert. Die vereinigten organischen Phasen wurden mit MgSO₄ getrocknet. Anschließend wurde das Lösungsmittel im Vakuum entfernt. Der erhaltene Feststoff wurde mit Methanol (2 ml) versetzt und zum Sieden erhitzt. Das Gemisch wurde nach Erreichen von RT zur Vervollständigung der Fällung 17 h bei 5 °C belassen. Anschließend wurde der Feststoff mittels einer Fritte abgetrennt und im Vakuum getrocknet. Eine weitere Charge des racemischen Diastereoisomerenpaars konnte so in einer Ausbeute von 198 mg [Gesamtausbeute: 265 mg, 51 % (berechnet auf 100 % Monoesterketon)] mit einem Schmelzpunkt von 256-263 °C isoliert werden.
Beispiel Nr. 15:¹³C-NMR (101 MHz, CDCl₃) δ ppm : 22.3, 28. 1, 31 .3, 33.0, 35.1, 38.3, 39.0, 51.3, 59.7, 61.9, 74.1, 109.5, 111.0, 118.0, 119.5, 121.7, 126.9, 128.0, 128.2, 135.7, 136.2, 136.4, 174.1

### Beispiel Nr. 16

### Stufe 1:

2-(4-(Dimethylamino)-4-phenyl-4',9'-dihydro-3'H-spiro[cyclohexan-1,1'-pyrano[3,4-b]indol]-2-yl)essigsäure-methylester (Beispiel Nr. 16, eins von 4 möglichen racemischen Diastereoisomerenpaaren)

Der (5-Dimethylamino-2-oxo-5-phenylcyclohexyl)essigsäure-methylester (310 mg, 1,07 mmol) wurde zusammen mit Tryptophol (172 mg, 1,2 mmol) in Dichlormethan (20 ml) gelöst und mit Trifluormethansulfonsäure-trimethylsilylester (0,2 ml, 1,1 mmol) versetzt. Der Ansatz wurde 20 h bei RT gerührt. - Zur Aufarbeitung wurde die Reaktionslösung mit 2N NaOH (10 ml) versetzt und 20 min gerührt. Die organische Phase wurde abgetrennt und die wäßrige Phase mit Dichlormethan (3 x 20 ml) extrahiert. Die vereinigten organischen Phasen wurden mit MgSO₄ getrocknet. Anschließend wurde das Lösungsmittel im Vakuum entfernt. Der feste Rückstand wurde mit Methanol (3 ml) versetzt und 2 h bei 5 °C belassen. Anschließend wurde der Feststoff mittels einer Fritte abgetrennt und im Vakuum getrocknet. Eine erste Charge des gewünschten Produktes konnte so in einer Ausbeute von 47 mg isoliert werden. Die methanolische Mutterlauge wurde eingeengt und der erhaltene Rückstand (385 mg) chromatographisch gereinigt [Kieselgel 60 G (10 g); Ethylacetat/Ethanol 2 : 1 (120 ml)]. Neben wenig bereits hydrolysiertem Produkt (freier Alkohol) konnten Fraktionen erhalten werden, die ein racemisches Diastereoisomerenpaar enthielten, das sich als mit der ersten Charge identisch herausstellte. Zur Entfernung vorhandener Verunreinigungen wurden die Fraktionen gemeinsam eingeengt, mit Methanol (1 ml) angerieben und 48 h bei 5 °C stehen gelassen. Das racemische Diastereoisomerenpaar konnte so in einer Gesamtausbeute von 98 mg (21 %) mit einem Schmelzpunkt von 233-237 °C isoliert werden.
Beispiel Nr. 16: ¹³C-NMR (101 MHz, CDCl₃) δ ppm : 20.6, 22.2, 27.6, 30.9, 31.7, 38.3, 41.5, 59.8, 61.5, 65.7, 73.0, 108.7, 110.8, 118.0, 119.5, 121.7, 126.9, 127.0, 127.8, 128.2, 135.9, 136.3, 136.5, 170.9

### Beispiel Nr. 17

### Stufe 1:

2-Allyl-*N,N*-dimethyl-4-phenyl-4',9'-dihydros-3'*H*-piro[cyclohexan-1,1'-pyrano[3,4-*b*]indol]-4-amin (Beispiel Nr. 17, aus dem polareren Keton, eins von 4 möglichen racemischen Diastereoisomerenpaaren)

Das polarere 2-Allyl-4-dimethylamino-4-phenylcyclohexanon (230 mg, 0,89 mmol) wurde zusammen mit Tryptophol (143 mg, 0,89 mmol) in Dichlormethan (20 ml) gelöst und mit Trifluormethansulfonsäure-trimethylsilylester (0,21 ml, 1,2 mmol) versetzt. Der Ansatz wurde 24 h bei RT gerührt. Zur Aufarbeitung wurde das Reaktionsgemisch mit 2N NaOH (20 ml) versetzt und 20 min gerührt. Die organische Phase wurde abgetrennt und die wäßrige Phase mit Dichlormethan (4 x 20 ml) extrahiert. Die vereinigten organischen Phasen wurden mit Na₂SO₄ getrocknet. Anschließend wurde das Lösungsmittel im Vakuum entfernt. Aus dem erhaltenen Rückstand (390 mg) wurde durch chromatographische Reinigung [Kieselgel 60 (50 g); Ethylacetat (500 ml)] eins der möglichen Diastereoisomeren in einer Ausbeute von 130 mg (37 %) mit einem Schmelzpunkt von 69-71 °C isoliert.
Beispiel Nr. 17: ¹³C-NMR (101 MHz, CDCl₃) δ ppm: 22.4, 27.5, 31.7, 32.1, 34.5, 38.2, 42.2, 59.8, 62.1, 74.5, 109.3, 110.8, 116.2, 117.9, 119.4, 121.5, 126.8, 126.9, 128.0, 128.1, 135.6, 136.7, 136.9, 137.3

### Beispiel Nr. 18

### Stufe 1:

2-Allyl-*N,N*-dimethyl-4-phenyl-4',9'-dihydros-3'*H*-piro[cydohexan-1,1'-pyrano[3,4-*b*]indol]-4-amin (aus dem unpolareren Keton, eins von 4 möglichen racemischen Diastereoisomerenpaaren)

Das unpolarere 2-Allyl-4-dimethylamino-4-phenylcyclohexanon (250 mg, 0,97 mmol) wurde zusammen mit Tryptophol (156 mg, 0,97 mmol) in Dichlormethan (25 ml) gelöst und mit Trifluormethansulfonsäure-trimethylsilylester (0,245 ml, 1,35 mmol) versetzt. Der Ansatz wurde 24 h bei RT gerührt. Zur Aufarbeitung wurde das Reaktionsgemisch mit 2N NaOH (20 ml) versetzt und 20 min gerührt. Die organische Phase wurde abgetrennt und die wäßrige Phase mit Dichlormethan (4 x 20 ml) extrahiert. Die vereinigten organischen Phasen wurden mit Na₂SO₄ getrocknet. Anschließend wurde das Lösungsmittel im Vakuum eingedampft. Aus dem erhaltenen Rückstand (400 mg) wurde durch chromatographische Reinigung [Kieselgel 60 (50 g); Ethylacetat (500 ml)] eins der möglichen Diastereoisomeren in einer Ausbeute von 290 mg (74 %) isoliert.

### Stufe 2:

2-Allyl-*N,N*-dimethyl-4-phenyl-4',9'-dihydros-3'*H*-piro[cyclohexan-1,1'-pyrano[3,4-*b*]indol]-4-amin Hydroxypropan-1,2,3-tricarbonsäure (Beispiel Nr. 18, aus dem unpolareren Keton, eins von 4 möglichen racemischen Diastereoisomerenpaaren)

Zur Herstellung des Citrates wurde das 2-Allyl-*N,N*-dimethyl-4-phenyl-4',9'-dihydros-3'*H-*piro[cyclohexan-1,1'-pyrano[3,4-*b*]indol]-4-amin aus Stufe 1 (290 mg, 0,72 mmol) in heißem Isopropanol (80 ml) gelöst und mit einer ebenfalls heißen, isopropanolischen CitronensäureLösung (140 mg, 0,72 mmol in 3 ml) versetzt. Anschließend wurde die Reaktionsmischung 16 h im Kühlschrank gelagert. Der entstandene Feststoff wurde abgesaugt. Das Citrat wurde so in einer Ausbeute von 157 mg (37 %) als weißer Feststoff (Schmelzpunkt: 233-236 °C) erhalten.
Beispiel Nr. 18:¹³C-NMR (101 MHz, DMSO-d₆) δ ppm: 22.1, 27.9, 29.4, 32.5, 34.2, 37.9, 38.9, 42.9, 59.1, 59.4, 72.2, 74.5, 106. 111.3, 115.9, 117.4, 118.3, 120.4, 126.4, 126.5, 127.4, 136.0, 137.7, 137.8, 139.2, 171.3, 175.0

### Beispiel Nr. 19 und Beispiel Nr. 20 und Beispiel Nr. 21

### Stufe 1:

2-Fluor-*N,N*-dimethyl-4-phenyl-4',9'-dihydro-3'H-spiro[cyclohexan-1,1'-pyrano[3,4-*b*]indol]-4-amin (Beispiel Nr. 19, unpolares Diastereomer und Beispiel Nr. 20, mittleres Diastereomer und Beispiel Nr. 21, polares Diastereomer)

Das (±)-4-Dimethylamino-2-fluor-4-phenylcyclohexanon (1,61 g, 6,83 mmol, Mischung beider Diastereoisomere, hauptsächlich polares Diastereoisomer) wurde zusammen mit Tryptophol (1,10 g, 6,83 mmol) in absolutem Dichlormethan (75 ml) bei 0 °C (Innentemperatur) gelöst. Anschließend wurde Trimethylsilyltriflat (1,67 g, 7,51 mmol, 1,36 ml, 1,225 g/ml) in absoluten Dichlormethan (10 ml) schnell hinzugetropft. Die Reaktionsmischung verfärbte sich sofort von gelb zu rotbraun. Das Reaktionsgemisch wurde bei Raumtemperatur 5 h gerührt und trübte sich dabei ein. Es wurde 5N Natriumhydroxid-Lösung (100 ml) zu der Reaktionsmischung hinzugegeben und 10 min gerührt. Nach Phasentrennung wurde die wäßrige Phase mit Dichlormethan (4 x 50 ml) extrahiert. Die vereinigten organischen Phasen wurden über Natriumsulfat getrocknet, filtriert und anschließend im Vakuum vollständig von flüchtigen Bestandteilen befreit. Es wurde versucht, den Rückstand chromatographisch zu trennen [Kieselgel (150 g), Chloroform/Ethanol 50 : 1 (500 ml), 19 : 1 (500 ml), 9 : 1 (1000 ml), 1 : 1 (500 ml), Methanol (1000 ml)]. Es wurden 467 mg (1,23 mmol, 18 %, Smp. 259-263 °C) eines unpolareren Diastereomers, 506 mg (1,34 mmol, 20%, Smp. 217-222 °C) eines Diastereomers mittlerer Polarität und 168 mg (0,44 mmol, 6 %, Smp. 245-247 °C) eines polareren Diastereoisomers isoliert. Die einzelnen Fraktionen wurden aus Methanol umkristallisiert.
Beispiel Nr. 19: ¹³C{¹H}-NMR (101 MHz. DMSO-*D*₆) δ ppm (unpolareres Diastereoisomer): 21.9 (1 C), 27.2 (1 C), 28.2 (1 C), 33.3 (1 C, d, J = 18 Hz), 38.4 (2 C), 58.5 (1 C), 60.0 (1 C), 72.6 (1 C, J = 22 Hz), 92.1 (1 C, d, J = 181 Hz), 106.5 (1 C), 111.7 (1 C), 117.6 (1 C), 118.3 (1 C), 120.7 (1 C), 125.8 (1 C), 126.5 (2 C), 127.4 (2 C), 128.2 (1 C, d, J = 26 Hz), 135.0 (1 C), 136.2 (1 C), 137.7 (1 C)
Beispiel Nr. 20: ¹³C{¹H}-NMR (101 MHz, DMSO-*D*₆) δ ppm (Diastereoisomer mittlerer Polarität): 21.8 (1 C), 26.6 (1 C), 30.6 (1 C, d, J = 5 Hz), 33.3 (1 C, d, J = 19 Hz), 38.0 (2 C), 60.7 (1 C), 62.4 (1 C, d, J = 13 Hz), 73.6 (1 C, J =16 Hz), 92.5 (1 C, d, J = 182 Hz), 107.7 (1 C), 111.0 (1 C), 117.6 (1 C), 118.3 (1 C), 120.8 (1 C), 126.4 (1 C, d, J = 8 Hz), 126.8 (1 C), 127.3 (2 C), 127.9 (2 C), 134.4 (1 C), 135.9 (1 C), 136.2 (1 C)
Beispiel Nr. 21: ¹³C{¹H}-NMR (101 MHz, DMSO-*D*₆) δ ppm (polareres Diastereoisomer): 21.4 (1 C), 27.3 (1 C), 31.3 (1 C, d, J = 5 Hz), 33.9 (1 C, d, J = 20 Hz), 38.7 (2 C), 61.4 (1 C), 63.2 (1 C, br, d, J = 15 Hz), 74.3 (1 C, J = 16 Hz), 93.2 (1 C, d, J = 182 Hz), 108.4 (1 C), 111.7 (1 C), 118.2 (1 C), 119.1 (1 C), 121.5 (1 C), 127.5 (1 C), 127.0 (1 C), 128.1 (2 C), 128.6 (2 C), 135.1 (1 C), 136.4 (1 C, br), 136.6 (1 C)

### Beispiel Nr. 22 und Beispiel Nr. 23

### Stufe 1:

2,6'-Difluor-*N,N*-dimethyl-4-phenyl-4'9'-dihydro-3'*H*-spiro[cyclohexan-1,1'-pyrano[3,4-*b*]indol]-4-amin (Beispiel Nr. 22, unpolareres Diastereomer und Beispiel Nr. 23, polareres Diastereomer)

Das polarere 4-Dimethylamino-2-fluor-4-phenylcyclohexanon (950 mg, 4,04 mmol) wurde zusammen mit Tryptophol (723 mg, 4,04 mmol) in absolutem Dichlormethan (45 ml) bei 0 °C (Innentemperatur) gelöst. Anschließend wurde Trimethylsilyltriflat (987 mg, 4,44 mmol, 0,81 ml, 1,225 g/ml) in absoluten Dichlormethan (10 ml) schnell hinzugetropft. Die Reaktionsmischung verfärbte sich sofort von gelb zu rotbraun. Das Reaktionsgemisch wurde bei Raumtemperatur 5 h gerührt und trübte sich dabei ein. Es wurde 5N Natriumhydroxid-Lösung (60 ml) zu der Reaktionsmischung hinzugegeben und 10 min gerührt. Aus dem ZweiPhasen-Gemisch fiel ein farbloser Feststoff aus. Dieser wurde mit Hilfe einer Fritte abfiltriert und anschließend mit Methanol (50 ml) gewaschen. Bei dem Feststoff handelte es sich um das polarere Diastereoisomer (241 mg). Aus dem Filtrat fiel erneut das polarere Diastereoisomer aus, welcher ebenfalls abfiltriert wurde. Nach Phasentrennung wurde die wäßrige Phase mit Dichlormethan (4 x 40 ml) extrahiert. Die vereinigten organischen Phasen wurden über Natriumsulfat getrocknet, filtriert und anschließend im Vakuum vollständig von flüchtigen Bestandteilen befreit. Es wurde versucht, den Rückstand chromatographisch zu trennen [Kieselgel (150 g), Chloroform/Ethanol 19 : 1 (1000 ml), Methanol (500 ml)]. Es wurden 676 mg (1,70 mmol, 42 %, Smp. 260-265 °C) eines unpolareren Diastereomers und insgesamt 572 mg (1,44 mmol, 36 %, Smp. 237-242 °C) des polareren Diastereoisomers isoliert.
Beispiel Nr. 22: ¹³C{¹H}-NMR (101 MHz, DMSO-*D*₆) δ ppm (unpolareres Diastereoisomer): 21.8 (1 C), 27.2 (1 C), 28.1 (1 C), 33.2 (1 C, d, J = 18 Hz), 38.3 (2 C), 58.5 (1 C), 60.0 (1 C), 72.5 (1 C, J = 22 Hz), 92.0 (1 C, d, J = 179 Hz), 102.3 (1 C, d, J = 23 Hz), 106.9 (1 C, d, J = 5 Hz), 108.7 (1 C, d, J = 26 Hz), 112.5 (1 C, d, J = 10 Hz), 125.9 (1 C, d, J = 10 Hz), 126.4 (1 C), 126.5 (2 C), 127.3 (2 C), 132.8 (1 C), 137.0 (1 C), 137.6 (1 C), 156.7 (1 C, d, J = 231 Hz) Beispiel Nr. 23: ¹³C{H}-NMR (101 MHz, DMSO-*D*₆) δ ppm (polareres Diastereoisomer): 21.7 (1 C), 26.6 (1 C), 30.5 (1 C, d, J = 5 Hz), 33.3 (1 C, d, J = 19 Hz), 38.0 (2 C), 60.6 (1 C), 62.4 (1 C, d, J = 13 Hz), 73.6 (1 C, J = 16 Hz), 92.5 (1 C, d, J = 182 Hz), 102.4 (1 C, d, J = 23 Hz), 108.1 (1 C, d, J = 5 Hz), 108.6 (1 1C, d, J = 26 Hz), 111.8 (1C, d, J = 10 Hz), 126.5 (1 C, d, J = 10 Hz), 126.8 (1 C), 127.3 (2 C), 127.9 (2 C), 132.5 (1 C), 135.7 (1 C), 136.6 (1 C), 156.7 (1 C, d, J = 231 Hz)

### Beispiel Nr. 24

### Stufe 1:

2,6'-Difluor-*N,N*-dimethyl-4-phenyl-4',9'-dihydro-3'*H*-spiro[cyclohexan-1,1'-pyrano[3,4-*b*]indol]-4-amin (Beispiel Nr. 24, ein Diastereomer)

Das unpolarere 4-Dimethylamino-2-fluor-4-phenylcyclohexanon (400 mg, 1,70 mmol) wurde zusammen mit 5-Fluortryptophol (305 mg, 1,70 mmol) in absolutem Dichlormethan (20 ml) bei 0 °C (Innentemperatur) gelöst. Anschließend wurde Trimethylsilyltriflat (416 mg, 1,87 mmol, 0,34 ml, 1,225 g/ml) in absolutem Dichlormethan (10 ml) schnell hinzugetropft. Die Reaktionsmischung verfärbte sich sofort von gelb zu rotbraun. Das Reaktionsgemisch wurde bei Raumtemperatur 48 h gerührt und trübte sich dabei ein. Danach wurde 5N Natriumhydroxid-Lösung (60 ml) zugegeben und das Gemisch 10 min gerührt. Nach der Phasentrennung wurde die wäßrige Phase mit Dichlormethan (4 x 40 ml) extrahiert. Die vereinigten organischen Phasen wurden über Natriumsulfat getrocknet, filtriert und anschließend im Vakuum vollständig von flüchtigen Bestandteilen befreit. Der Rückstand wurde chromatographisch getrennt [Kieselgel 60 (80 g), Trichlormethan/Ethanol 19 : 1 (500 ml); Trichlormethan/Ethanol 9 : 1 (1000 ml), Trichlormethan/Ethanol 9 : 1 + 1 % wäßrige Ammoniak-Lösung (1000 ml), Methanol + 1 % wäßrige Ammoniak-Lösung (500 ml)]. Eins der zwei möglichen Diastereoisomeren (50 mg, 0,13 mmol, 7 %) konnte als farbloser Feststoff (Smp. 258-264 °C) isoliert werden.
Beispiel Nr. 24: ¹³C{H}-NMR (101 MHz, DMSO-*D*₆, δ ppm, AS 03391): 21.7 (1 C), 27.2 (1 C), 29.9 (1 C, d, J = 4 Hz), 33.2 (1 C, d, J = 20 Hz), 37.9 (2 C), 60.6 (1 C), 61.4 (1 C, d, J = 12), 73.6 (1 C, J = 16 Hz), 92.4 (1 C, d, J = 180 Hz), 102.5 (1 C, d, J = 23 Hz), 108.0 (1 C, d, J = 5 Hz), 108.6 (1 C, d, J = 26 Hz), 112.0 (1 C, d, J = 10 Hz), 126.4 (2 C), 126.7 (1 C), 126.8 (1 C), 127.4 (2 C), 132.7 (1 C), 137.2 (1 C), 138.2 (1 C), 156.7 (1 C, d, J = 231 Hz)

### Beispiel Nr. 25 und Beispiel Nr. 26 und Beispiel Nr. 27 und Beispiel Nr. 28

### Stufe 1:

2-Fluor-*N,N*-dimethyl-4-phenyl-2',3',4',9'-tetrahydrospiro[cyclohexan-1,1'-pyrido[3,4-*b*]indol]-4-amin (Beispiel Nr. 25, Beispiel Nr. 26, Beispiel Nr. 27, Beispiel Nr. 28, 4 racemische Diastereoisomerenpaare)

### Umsetzung des unpolareren 4-Dimethylamino-2-fluor-4-phenylcyclohexanons

Das unpolarere 4-Dimethylamino-2-fluor-4-phenylcyclohexanon (481 mg, 2,04 mmol) und Tryptamin (327 mg, 2,04 mmol) wurden in einer Argonatmosphäre in trockenem Methanol (25 ml) gelöst und die entstandene Reaktionsmischung 12 h gerührt. Anschließend wurde das Methanol im Vakuum vollständig entfernt und der Rückstand in trockenem 1,2-Dichlorethan (25 ml) suspendiert. Die Reaktionsmischung wurde mit Trifluoressigsäure (2,5 ml) versetzt und 2 h bei Raumtemperatur gerührt. Die Reaktionsmischung wurde mit Wasser (50 ml) und 1,2-Dichlorethan (25 ml) verdünnt. Unter Eiskühlung wurde der pH-Wert der Reaktionsmischung mit 1 N Natriumhydroxid-Lösung auf pH 11 eingestellt und 1 h gerührt. Es entstand kaum Niederschlag. Beim Abtrennen mit Hilfe einer Fritte blieb kein Rückstand zurück. Die Phasen wurden getrennt. Die organische Phase wurde mit Natriumsulfat getrocknet und im Vakuum vollständig von flüchtigen Bestandteilen befreit. Der Rückstand wurde chromatographisch getrennt [Kieselgel 60 (80 g), Ethylacetat/Methanol 2 : 1 (1000 ml), Methanol (500 ml), Tetrahydrofuran (500 ml)]. Das Keton isomerisierte während der Reaktion, daher konnten mehr als zwei Diastereoisomere der Zielverbindung isoliert werden. Es wurde zum einen ein Gemisch aus zwei Diastereoisomeren (9 %, 71 mg, 0,189 mmol, Smp. 206-236 °C) isoliert (Beispiel Nr. 28). Weiterhin konnte ein unpolareres Diastereoisomer (81 mg, 0,21 mmol, 10 %, Smp. 197-237 °C) isoliert werden (Beispiel Nr. 25). Darüber hinaus wurde ein polareres Diastereoisomer (73 mg, 0,19 mmol, 9 %, Smp. 180-182 °C) isoliert (Beispiel Nr. 26).

### Umsetzung des polareren 4-Dimethylamino-2-fluor-4-phenylcyclohexanon

Das polarere 4-Dimethylamino-2-fluor-4-phenylcyclohexanon (1,47 g, 6,27 mmol) und Tryptamin (1,00 g, 6,27 mmol) wurden in einer Argonatmosphäre in trockenem Methanol (63 ml) gelöst und 10 h gerührt. Anschließend wurde das Methanol vollständig im Vakuum entfernt und der Rückstand in trockenem 1,2-Dichlorethan (63 ml) suspendiert. Die Reaktionsmischung wurde mit Trifluoressigsäure (6,3 ml) versetzt und 2 h bei Raumtemperatur gerührt. Dann wurde die Reaktionsmischung mit Wasser (50 ml) und 1,2-Dichlorethan (25 ml) verdünnt. Der pH-Wert der Reaktionsmischung wurde unter Eiskühlung mit 1 N Natriumhydroxid-Lösung auf pH 11 eingestellt. Es entstand ein Niederschlag. Die Mischung wurde 1 h gerührt. Der Niederschlag wurde mit Hilfe einer Fritte abgetrennt. Da der Niederschlag laut NMR nicht einheitlich war, wurde er mit Hilfe einer Flashchromatographie aufgetrennt [Kieselgel 60 (80 g); Ethylacetat/Methanol 2 : 1 (1000 ml), Methanol (500 ml), Tetrahydrofuran (500 ml)]. Das Keton isomerisierte während der Reaktion. Daher konnten mehr als die zwei möglichen Diastereoisomere isoliert werden. So wurde ein Gemisch aus zwei Diastereoisomeren in einer Ausbeute von 1 % (20 mg, 0,05 mmol) isoliert (Beispiel Nr. 28). Es wurde ein weiteres unpolareres Diastereoisomer (122 mg, 0,32 mmol, 5 %, Beispiel Nr. 25) isoliert. Außerdem konnte noch ein Gemisch aus zwei Diastereoisomeren isoliert werden (6 %, 145 mg, 0,38 mmol). - Die Phasen des Filtrates wurden getrennt. Die organische Phase wurde mit Natriumsulfat getrocknet, filtriert und im Vakuum von flüchtigen Bestandteilen befreit. Der Rückstand wurde chromatographisch gereinigt [Kieselgel 60 (80 g); Ethylacetat/Methanol 2 : 1 (1000 ml), Methanol (500 ml), Tetrahydrofuran (500 ml)]. Es wurde das polarere der beiden Diastereoisomere aus dem Gemisch (Beispiel Nr. 28) (84 mg, 0,22 mmol, 4 %, Smp. 239-246 °C, Beispiel Nr. 27) isoliert. Ein unpolareres Diastereoisomer (241 mg, 0,64 mmol, 10 %) konnte ebenfalls isoliert werden (Beispiel Nr. 25). Ferner wurde ein polareres Diastereoisomer (163 mg, 0,43 mmol, 7 %) isoliert (Beispiel Nr. 26).
Beispiel Nr. 25: ¹³C-NMR (101 MHz, DMSO-*D*₆, δ ppm, ein Diastereoisomer): 22.5 (1 C), 26.4 (1 C), 31.4 (1 C, d, J = 6 Hz), 32.8 (1 C, d, J = 19 Hz), 38.0 (2 C), 55.1 (1 C, d, J = 16 Hz), 62.7 (1 C, d, 13 Hz), 93.9 (1 C, J = 178 Hz), 109.2 (1 C), 110.9 (1 C), 117.3 (1 C), 118.0 (1 C), 120.5 (1 C), 126.7 (1 C), 127.0 (1 C), 127.4 (2 C), 127.9 (2 C), 135.7 (1 C), 136.3 (1 C), 136.9 (1 C), n.b. (1 C)
Beispiel Nr. 26: ¹³C-NMR (101 MHz, DMSO-*D*₆, δ ppm, ein Diastereoisomer): 22.6 (1 C), 26.5 (1 C), 29.9 (1 C), 33.7 (1 C, d, J = 19 Hz), 37.6 (2 C), 53.7 (1 C, d, J = 20 Hz), 59.5 (1 C, d, 2 Hz), 94.2 (1 C, J = 174 Hz), 108.2 (1 C), 111.3 (1 C), 117.3 (1 C), 117.9 (1 C), 120.3 (1 C), 126.1 (1 C), 126.3 (1 C), 127.0 (2 C), 127.3 (2 C), 135.7 (1 C), 136.7 (1 C), 137.0 (1 C), n.b. (1 C)

### Beispiel Nr. 27: ¹³C-NMR (101 MHz, DMSO-D₆, δ ppm, identisch mit dem polareren

Diastereoisomer aus dem Beispiel Nr. 28): 22.6 (1 C), 27.1 (1 C), 30.4 (1 C), 30.7 (1 C, d, J = 5 Hz), 32.9 (1 C, d, J = 19 Hz), 38.0 (2 C), 55.1 (1 C, d, J = 16 Hz), 61.6 (1 C, d, 12 Hz), 93.7 (1 C, J = 176 Hz), 109.0 (1 C), 111.1 (1 C), 117.4 (1 C), 118.1 (1 C), 120.5 (1 C), 126.4 (2 C), 126.5 (1 C), 127.0 (1 C), 127.3 (2 C), 135.8 (1 C), 137.5 (1 C), 138.6 (1 C)

### Beispiel Nr. 29

### Stufe 1:

4-(Dimethylamino-4-phenyl-4',9'-dihydro-3'*H*-spiro[cyclohexan-1,1'-pyrano[3,4-*b*]indol]-2-yl)methanol (Beispiel Nr. 29, eins von 4 möglichen racemischen Diastereoisomerenpaaren)

Der 2-{4-(Dimethylamino)-4-phenyl-4',9'-dihydro-3'H-spiro[cyclohexan-1,1'-pyrano[3,4-b]indol]-2-yl)essigsäure-methylester (Beispiel Nr. 16, eins von 4 möglichen racemischen Diastereoisomerenpaaren) (190 mg, 0,44 mmol) wurde in einem Gemisch aus 2N HCl (20 ml) und Ethanol (20 ml) gelöst und 18 h bei RT gerührt. Zur Aufarbeitung wurde das Ethanol im Vakuum abgezogen, der wäßrige Rückstand mit NaHCO₃ neutralisiert und mit 2 N NaOH streng basisch gemacht. Die wäßrige Lösung wurde mit Ethylacetat (3 x 10 ml) extrahiert. Die vereinigten organischen Phasen wurden über MgSO₄ getrocknet und anschließend eingeengt. Der erhaltene feste Rückstand erwies sich als eines der vier möglichen Diastereoisomeren des gewünschten Alkohols in reiner Form. Das Produkt wurde so in einer Ausbeute von 153 mg (89 %) mit einem Schmelzpunkt von 219-233 °C (aus Propan-2-ol) erhalten.
Beispiel Nr. 29: ¹³C-NMR (101 MHz, DMSO-d₆, δ ppm): 22.1, 27.9, 30.5, 31.0, 37.9, 43.9, 59.1, 60.8, 61.6, 73.8, 106.5, 111.0, 117.3, 118.2, 120.4, 126.2, 126.3, 127.59, 127.63, 135.9, 136.6, 137.4

### Beispiel Nr. 30

### Stufe 1:

2-(4-(Dimethylamino)-4-phenyl-4',9'-dihydro-3'*H*-spiro[cyclohexan-1,1'-pyrano[3,4-*b*]indol]-2-yl)ethanol (Beispiel Nr. 30, eins von 4 möglichen racemischen Diastereoisomerenpaaren) Der polarere 2-(4-(Dimethylamino)-4-phenyl-4',9'-dihydro-3'H-spiro[cyclohexan-1,1'-pyrano[3,4-b]indol]-2-yl)essigsäure-methylester (254 mg, 0,58 mmol) wurde in trockenem THF (20 ml) gelöst und bei RT innerhalb von 10 min portionsweise mit LiAlH₄ (50 mg, 1,16 mmol) versetzt. Nach beendeter Zugabe wurde der Ansatz 90 min bei RT gerührt. - Zur Aufarbeitung wurde überschüssiges LiAlH₄ durch Zugabe von feuchtem THF (2 ml eines Gemisches aus Wasser (ca. 0,3 ml) und THF (2 ml) zersetzt. Die erhaltene Mischung wurde auf eine Fritte mit Kieselgur (Schichthöhe ca. 2 cm) gegeben und der Filterkuchen mit Ethylacetat (3 x 30 ml) und Tetrahydrofuran (3 x 30 ml) gewaschen. Das Filtrat wurde bis zur Trockne eingeengt. Bei dem so erhaltenen Feststoff (230 mg) handelte es sich um das gewünschte racemische Diastereoisomerenpaar. Der gewünschten Alkohol konnte so in einer Ausbeute von 230 mg (98 %,Zersetzung ab 215°C) isoliert werden.
Beispiel Nr. 30: ¹³C-NMR (101 MHz, DMSO-d₆) δ ppm: 22.1, 27.5, 30.4, 31.0, 33.0, 33.4, 34.3, 37.9, 38.3, 59.0, 59.5, 61.0, 74.8, 106.8, 111.1, 117.3, 118.1, 120.3, 124.8, 126.2, 126.4, 127.5. 127.6, 135.8, 136.7, 137.8, 139.1

### Beispiel Nr. 31

### Stufe 1:

2-(4-(Dimethylamino)-4-phenyl-4',9'-dihydro-3'*H*-spiro[cyclohexan-1'-pyrano[3,4-*b*]indol]-2-yl)ethanol (Beispiel Nr. 31, eins von 4 möglichen racemischen Diastereoisomerenpaaren)

Der 2-(4-(Dimethylamino)-4-phenyl-4',9'-dihydro-3'H-spiro[cyclohexan-1,1'-pyrano[3,4-b]indol]-2-yl)essigsäure-methylester (279 mg, 0,65 mmol) wurde in trockenem THF (20 ml) gelöst und bei RT innerhalb von 10 min portionsweise mit LiAlH₄ (50 mg, 1,32 mmol) versetzt. Nach beendeter Zugabe wurde der Ansatz 90 min bei RT gerührt. - Zur Aufarbeitung wurde überschüssiges LiAlH₄ durch Zugabe von feuchtem THF (2 ml eines Gemisches aus Wasser (ca. 0,3 ml) und THF (2 ml) zersetzt. Die erhaltene Mischung wurde auf eine Fritte mit Kieselgur (Schichthöhe ca. 2 cm) gegeben und der Filterkuchen mit Ethylacetat (3 x 30 ml) gewaschen. Aus dem Filtrat fiel ein Feststoff aus, der auch beim Ausschütteln mit 2N NaOH in der organischen Phase verblieb. Der Feststoff wurde mittels Filtration aus dem Lösungsmittelgemisch abgetrennt. Eine erste Charge des gewünschten racemischen Diastereoisomerenpaares konnte so in einer Ausbeute von 77 mg mit einem Schmelzpunkt von 311-314 ° (ab 280°C Kristallumwandlung) erhalten werden. Die organische Phase der Mutterlauge wurde aus dem Lösungsmittelgemisch abgetrennt und eingeengt. Der entstandene Rückstand wurde mit Chloroform (1 ml) angerieben und die entstandenen Kristalle mittels einer Fritte isoliert. Bei dem so erhaltenen Feststoff handelte es sich ebenfalls um den gewünschten Alkohol der somit in einer Gesamtausbeute von 130 mg (49 %) isoliert werden konnte.
Beispiel Nr. 31: ¹³C-NMR (101 MHz, DMSO-d₆) δ ppm: 22.2, 27.9, 29.5, 33.1, 33.9, 36.5, 38.0, 58.6, 59.0, 59.7, 74.9, 106.7, 111.3. 117.3, 118.1, 120.3, 126.2, 126.4, 127.2, 136.0, 138.4, 139.8

### Beispiel Nr. 32

### Stufe 1:

2-(4-(Dimethylamino)-4-phenyl-4',9'-dihydro-3'*H*-spiro[cyclohexan-1,1'-pyrano[3,4-b]indol]-2-yl)methyl)isoindolin-1,3-dion (Beispiel Nr. 32, eins von vier möglichen Diastereoisomeren)

Trypthophol (97 mg, 0,6 mmol) wurde unter Feuchtigkeitsausschluß zusammen mit dem 2-(5-Dimethylamino-2-oxo-5-phenylcyclohexylmethyl)isoindolin-1,3-dion (220 mg, 0,6 mmol) in trockenem Dichlormethan (20 ml) vorgelegt und schnell mit Trifluormethansulfonsäure-trimethylsilylester (0,26 ml, 1,44 mmol) versetzt. Der Ansatz wurde bei RT 24 h gerührt. - Zur Aufarbeitung des Ansatzes wurde die Mischung mit 2N Natronlauge (10 ml) versetzt und 15 min gerührt. Die wäßrige Phase wurde mit Dichlormethan (2 × 30 ml) extrahiert. Die vereinigten organischen Phasen wurden nach dem Trocknen (Na₂SO₄) eingeengt, wobei ein braunes Öl (300 mg) erhalten wurde. Nach Zugabe von Methanol (3 ml) fiel ein weißer Feststoff aus, welcher abgesaugt und anschließend getrocknet wurde. Eins der vier möglichen Diastereoisomeren konnte so in 100 mg Ausbeute (32 %) mit einem Schmelzpunkt von 148-166 °C erhalten werden.
Beispiel Nr. 32: ¹³C NMR (101 MHz, CDCl₃, δ ppm, AS 05791): 22.0. 27.8, 29.2. 32.0, 37.7, 37.9, 38.4, 58.5, 59.4, 73.2, 106.9. 111.1, 117.1, 117.9, 120.3, 122.3, 126.1, 126.3, 127.3, 131.1, 133.7, 136.1, 136.8, 139.4, 167.6

### Beispiel Nr. 33

### Stufe 1:

2-(Aminomethyl)-4-(3-fluorphenyl-*N,N*-dimethyl-4',9'-dihydro-3'*H*-spiro[cyclohexan-1,1'-pyrano[3,4-b]indol]-4-amin (eins von vier möglichen Diastereoisomeren)

Zu einer Lösung des 2-(4-(Dimethylamino)-4-phenyl-4',9'-dihydro-3'*H*-spiro[cyclohexan-1,1'-pyrano[3,4-*b*]indol]-2-yl)methyl)isoindolin-1,3-dions (190 mg, 0,35 mmol, Beispiel Nr. 31) in Methanol (50 ml) wurde unter Rühren bei Raumtemperatur Hydrazinhydrat (97 µl, 1,99 mmol) gegeben. Der Ansatz wurde 2 d bei 150°C im Teflongefäß gerührt und dann mit H₂O (50 ml) versetzt. Das im Lösungsmittelgemisch enthaltene Methanol wurde am Rotationsverdampfer abdestilliert. Der wäßrige Rückstand wurde mit Ethylacetat (3 x 20 ml) extrahiert. Die vereinigten organischen Phasen wurden über Na₂SO₄ getrocknet und anschließend eingeengt. Der ölige Rückstand (130 mg, 90 % Ausbeute) erwies sich im NMR als weitgehend einheitliches Produkt.

### Stufe 2:

*N*-((4-(Dimethylamino)-4-(3-fluorphenyl)-4',9'-dihydro-3'*H*-spiro[cyclohexan-1,1'-pyrano[3,4-*b*]indol)-2-yl)methyl)-3-phenylzimtsäureamid (Beispiel Nr. 33, eins von vier möglichen Diastereoisomeren)

Unter Argon wurde das 2-(Aminomethyl)-4-(3-fluorphenyl)-*N,N*-dimethyl-4',9'-dihydro-3'*H-*spiro[cyclohexan-1,1'-pyrano[3,4-*b*]indol]-4-amin (130 mg, 0,31 mmol) in absolutem Tetrahydrofuran (20 ml) gelöst. Zur klaren Lösung wurden bei Raumtemperatur nacheinander Hünig-Base (0,1 ml, 0,63 mmol) und Zimtsäurechlorid (105 mg, 0,63 mmol), gelöst in absolutem Tetrahydrofuran (8 ml), hinzugefügt. Nach einer Reaktionszeit von 45 min wurde der Ansatz mit Wasser (10 ml) und 2N Natronlauge (10 ml) versetzt und 1 h gerührt. Zur Aufarbeitung wurde Tetrahydrofuran vollständig abdestilliert. Das wäßrige Gemisch wurde mit Dichlormethan (3 × 20 ml) extrahiert. Die vereinigten organischen Extrakte wurden noch einmal mit Wasser (20 ml) gewaschen, über Na₂SO₄ getrocknet und eingeengt. Das Rohprodukt (247 mg) wurde chromatographisch aufgetrennt [Kieselgel 60 (30 g); Ethylacetat/Cyclohexan 5 : 1 (600 ml)]. Eins von vier möglichen Diastereoisomeren wurde in einer Ausbeute von 108 mg (65 %) mit einem Schmelzpunkt von 134-140 °C als weißer Feststoff erhalten.
Beispiel Nr. 33: ¹³C NMR (101 MHz, DMSO-d₆, δppm): 22,2, 28.0, 29.3, 30.4, 31.7, 37.9, 58.6, 59.3, 74.0, 106.7, 111.4, 112.9, 113.1, 113.4, 117.4, 118.2, 120.4, 122.3, 122.5, 126.3, 127.4, 128.8, 128.9, 129.0, 129.2, 134.9, 136.2, 137.4, 138.2, 142.9, 142.9, 160.7, 163.1, 164.8

### Beispiel Nr. 34

### Stufe 1:

tert-Butyl 2-(2-(4-(dimethylamino)-4-phenyf-4',9'-dihydro-3'H-spiro[cyclohexan-1,1'-pyrano[3,4-b]indol]-2-yl)ethoxy)acetat (Beispiel Nr. 34, eins von 4 möglichen Diastereoisomeren, aus unpolarerem Keton)

Zu einer Lösung des 2-(4-(Dimethylamino)-4-phenyl-4',9'-dihydro-3'*H*-spiro[cyclohexan-1,1'-pyrano[3,4-*b*]indol]-2-yl)ethanol (285 mg, 0,7 mmol) und Tetra-n-butylammoniumbromid (10 mg, 0,03 mmol) in Tetrahydrofuran (20 ml) wurde 50-proz. wäßrige Natronlauge (15 ml) bei 0°C gegeben. Anschließend wurde ebenfalls bei 0 °C eine Lösung von Bromessigsäure-tert-butylester (275 mg, 208 µl, 1,4 mmol) in Tetrahydrofuran (2 ml) zugetropft. Das heterogene Gemisch wurde 24 h bei Raumtemperatur gerührt. Anschließend wurde nochmals eine Lösung von Bromessigsäure-tert-butylester (550 mg, 416 µl, 2,8 mmol) in Tetrahydrofuran (2 ml) zugetropft und 24 h bei Raumtemperatur gerührt. Zur Aufarbeitung wurde die wäßrige Phase abgetrennt und die organische Phase mit Wasser (4 × 30 ml) neutral gewaschen. Die organische Phase wurde mit Natriumsulfat getrocknet und im Vakuum eingeengt. Aus dem erhaltenen Rückstand (410 mg) wurde durch chromatographische Reinigung [Kieselgel 60 (30 g); Ethylacetat (500 ml)] eins der vier möglichen Diastereoisomeren in einer Ausbeute von 30 mg (8 %) als Öl isoliert.
Beispiel Nr. 34: ¹³C-NMR (101 MHz, CDCl₃, δ ppm): 22.4, 28.1, 28.2, 29.9, 34.1, 36.1, 38.1, 59.6, 59.7, 68.5, 69.9, 75.0, 81.5, 108.2, 111.2, 117.8, 119.0, 121.5, 126.5, 126.9, 127.3, 136.0, 138.3, 139.6, 169.9

### Beispiel Nr. 35 und Beispiel Nr. 36

### Stufe 1:

2-(4-(Dimethy)amino)-4-pheny)-4',9'-dihydro-3'H-spiro[cyclohexan-1,1'-pyrano[3,4-b]indol]-2-yl)acetonitril (Beispiel Nr. 35, unpolares Diastereomer und Beispiel Nr. 36, polares Diastereomer)

Das (±)-3-(5-Dimethylamino-2-oxo-5-pheny)cyclohexy))propionitril (400 mg, 1,48 mmol) wurde zusammen mit Tryptophol (238 mg, 1,48 mmol) in Dichlormethan (20 ml) gelöst und mit Trifluormethansulfonsäure-trimethylsilylester (0,3 ml, 1,66 mmol) versetzt. Der Ansatz wurde 18 h bei RT gerührt. - Zur Aufarbeitung wurde die Reaktionslösung mit 2N NaOH (10 ml) versetzt und 20 min gerührt. Die organische Phase wurde abgetrennt und die wäßrige Phase mit Dichlormethan (3 x 20 ml) extrahiert. Die vereinigten organischen Phasen wurden mit MgSO₄ getrocknet. Anschließend wurde das Lösungsmittel im Vakuum entfernt. Der Rückstand (601 mg) wurde mit Methanol (20 ml) versetzt, wobei eine klare Lösung entstand. Die Lösung wurde auf etwa die Hälfte ihres Volumens eingeengt und 2 h bei bei 5 °C belassen. Der entstandene Niederschlag wurde mittels einer Fritte abgetrennt und im Vakuum getrocknet. Ein unpolareres Diastereoisomer konnte so in einer ersten Charge (254 mg, 41 %) als beiger Feststoff mit einem Schmelzpunkt von 208-213 °C isoliert werden. Die Kristalle enthielten etwa 1 Äquivalent Methanol. Im Eindampfrückstand der Mutterlauge (390 mg) befand sich neben weiterem unpolaren Produkt auch ein polareres Diastereoisomer. Durch Säulenchromatograpie [Kieselgel 60 G (10 g); Cyclohexan/Ethylacetat 1 : 1 (120 ml), Ethylacetat (100 ml), Ethylacetat/Ethanol 1 : 1 (100 ml)] konnte weiteres unpolareres racemisches Diastereoisomer [61 mg (nach Umkrsitallisation aus Methanol), [Gesamtausbeute 51 %] sowie das polarere racemische Diastereoisomer (29 mg, 4 %, Schmelzpunkt 260-267 °C) isoliert werden.
Beispiel Nr. 35: ¹³C-NMR (101 MHz, CDCl₃, δ ppm): 15.4, 22.5, 25.9, 28.1, 29.6, 33.4, 38.2, 38.8, 59.4, 59.8, 74.5, 108.8, 111.3, 118.0, 119.5, 120.2, 121.8, 126.77, 126.79, 127.5, 135.8, 137.1, 138.9
Beispiel Nr. 36: ¹³C-NMR (101 MHz, CDCl₃, δ ppm): 15.1, 22.3, 26.1, 27.4, 31.5, 32.7, 38.3, 40.9, 59.8, 61.7, 74.3, 109.2, 111.1, 118.0, 119.6, 120.0, 121.9, 126.7, 127.1, 127.9, 128.3, 135.7, 136.2

### Untersuchungen zur Wirksamkeit der erfindungsgemäßen Verbindungen

### Messung der ORL1-Bindung

Die Verbindungen wurden in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (AmershamPharmacia, Freiburg), durch einstündige Inkubation des Ansatzes bei RT und anschließende Messung im Szintillationscounter Trilux (Wallac, Finnland), bestimmt. Die Affinität wird in Tabelle 1 als nanomolarer Kᵢ-Wert in oder % Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden Verbindung mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem ß-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten. In einigen Fällen wurde auf die Bestimmung des Ki-Wertes verzichtet und nur die Hemmung bei einer Testkonzentration von 1 µM bestimmt.

### Nephelometrische Löslichkeitsuntersuchung (Phosphatpuffer pH 7.4):

Diese Methode untersucht die Löslichkeit einer Substanz bei festgelegten Konzentrationen (1 µM, 3 µM, 10 µM, 30 µM und 100 µM) in 10 mM Phosphatpufferlösung bei pH 7.4. Initial wird eine 10 mM Lösung der Substanzen in DMSO benötigt, aus der 100-fach Stammlösungen der oben genannten Konzentrationslevel wiederum in DMSO hergestellt werden, die finale DMSO Konzentration im Versuchsansatz beträgt 1 % (v/v). Das Experiment wird in mehrfach Bestimmung durchgeführt. Nach Zugabe der DMSO Stammlösungen zum Puffer, wird der Ansatz 2h bei 37°C inkubiert, bevor eine Absorptionsbestimmung bei 620 nm stattfindet. Steigt die Absorption der Proben über die der reinen Puffer/DMSO Lösung an, so gilt dies als Indikator für eine Präzipitatbildung. Die untere Löslichkeitsgrenze ("lower bund") ist die Konzentration, die derjenigen mit erster Präzipitatbildung vorangegangen ist (z.B. 3 µM, wenn Präzipitatbildung bei 10 µM detektiert wurde).

| Nr. | % Hemmung (ORL1) [1 µM] | Ki (ORL1) Mittel [µM] | % Hemmung (µ) [1 µM] | Ki (µ) Mittel [µM] |
|---|---|---|---|---|
| Bsp-1 | 99 | 0,001 | 77 | 0,001 |
| Bsp-2 | 97 | 0,006 | 106 | 0,02 |
| Bsp-3 | 50 | 0,23 | 84 | 0,086 |
| Bsp-5 | 93 | n.d. | 96 | n.d. |
| Bsp.-8 | 92 | 0,053 | 100 | 0,22 |
| Bsp.-9 | 97 | 0,107 | 103 | 0,098 |
| Bsp.-10 | nd | 0,016 | nd | 0,021 |
| Bsp.-11 | 53 | nd | 90 | nd |
| Bsp.-12 | 73 | 2,05 | 88 | 0,11 |
| Bsp.-14 | 94 | 0,006 | 101 | 0,003 |
| Bsp.-15 | 33 | 0,335 | 68 | 0,165 |
| Bsp.-16 | 50 | 0,34 | 75 | 0,215 |
| Bsp.-17 | 54 | 0,62 | 93 | 0,45 |
| Bsp.-18 | 89 | 0,026 | 97 | 0,003 |
| Bsp.-20 | 96 | nd | 96 | nd |
| Bsp.-21 | 76 | nd | 99 | nd |
| Bsp.-22 | 90 | nd | nd | nd |
| Bsp.-23 | 29 | 2,815 | nd | 0,12 |
| Bsp.-24 | 68 | nd | 99 | nd |
| Bsp.-25 | 87 | 0,027 | 100 | 0,017 |
| Bsp.-26 | 58 | nd | 87 | nd |
| Bsp.-27 | 95 | nd | 102 | nd |
| Bsp.-28 | 98 | nd | 99 | nd |
| Bsp.-29 | 80 | 0,063 | 99 | 0,067 |
| Bsp.-30 | 96 | 0,007 | 100 | 0,021 |
| Bsp.-31 | 94 | 0,014 | 99 | 0,002 |
| Bsp.-32 | 83 | nd | 101 | nd |
| Bsp.-33 | 83 | 0,07 | 97 | 0,013 |

Die erfindungsgemäßen Verbindungen vom Typ 1 mit X = O oder -NH, R₆ = H und Y₃ ≠ H (Bsp. 29, 30 und 25) wurden mit entsprechenden Verbindungen vom Typ 1 mit X = O oder - NH, R₆ = H und Me und Y₃ = H (V-1 und V-2) verglichen:

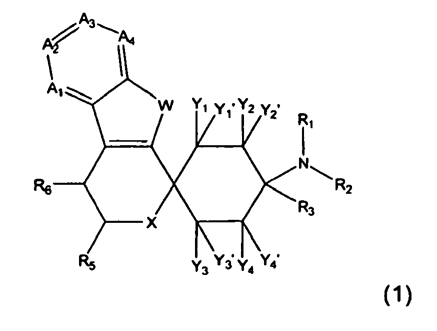

| Bsp. | X | Y₃ | R₃ | R₆ | Nephelometrie (lower bound) µM |
|---|---|---|---|---|---|
| 30 | O | 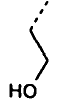 | 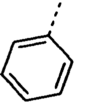 | H | 30 |
| 29 | O | 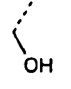 | 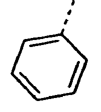 | H | 10 |
| V-1: | O | H | 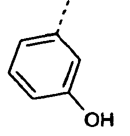 | H | 1 |
| 25 | NH | F | 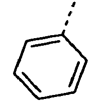 | H | 100 |
| V-2: | NH | H | 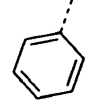 | Me | 10 |

Wie der vorstehende Vergleich belegt, weisen die erfindungsgemäßen Verbindungen im Vergleich zu strukturell ähnlichen Spiroverbindungen (Y₃ = H) eine bessere Löslichkeit in wässrigen Medien auf, was insbesondere Vorteile im Hinblick auf die Resorptionseigenschaften und/oder die Bioverfügbarkeit mit sich bringen sollte.

## Patentansprüche

1. Verbindung der allgemeinen Formel (1), 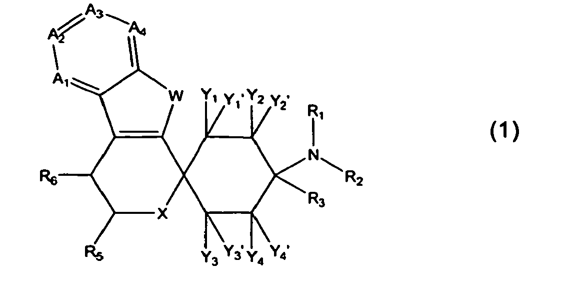 worin
A₁ für -N= oder -CR₇= steht,
A₂ für -N= oder -CR₈= steht,
A₃ für -N= oder -CR₉= steht,
A₄ für -N= oder -CR₁₀= steht;
mit der Maßgabe, dass höchstens zwei der Reste A₁, A₂, A₃ und A₄ für -N= stehen;
W für -NR₄-, -O- oder -S- steht;
X für -NR₁₇-, -O-, -S(=O)₀₋₂- oder -CR₁₈R₁₉- steht;
Y₁, Y₁', Y₂. Y₂', Y₃, Y3', Y₄ und Y₄ jeweils unabhängig voneinander ausgewählt sind aus der Gruppe bestehend aus -H, -F, -Cl, -Br, -I, -CN, -NO₂, -CHO, -R₀, -C(=O)R₀, -C(=O)-H, -C(=O)-OH. -C(=O)OR₀, -C(=O)NH₂, -C(=O)NHR₀, -C(=O)N(R₀)₂, -OH, -OR₀, -OC(=O)-R₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀ -N(R₀)₂, -N⁺(R₀)₃, -N⁺(R₀)₂O, -NHC(=O)R₀, -NHC(=O)OR₀, -NHC(=O)NH₂, -NHC(=O)NHR₀ und -NHC(=O)-N(R₀)₂; oder Y₁ und Y₁', oder Y₂ und Y₂ , oder Y₃ und Y₃', oder Y₄ und Y₄' gemeinsam für =O stehen;
mit der Maßgabe, dass wenigstens einer der Reste Y₁, Y₁', Y₂, Y₂', Y₃, Y₃', Y₄ und Y₄' nicht für -H steht;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroal, -C₃₋₈-Cycloaliphat-C₁₋₈-Aliphat, -C₃₋₈-Cycloaliphat-Aryl oder -C₃₋₈-Cycloaliphat-Heteroaryl steht;
R₁ und R₂, unabhängig voneinander für -H oder -R₀ stehen; oder R₁ und R₂ zusammen für -CH₂CH₂OCH₂CH₂-, -CH₂CH₂NR₁₁CH₂CH₂- oder -(CH₂)₃₋₆- stehen:
R₃ für -R₀ steht;
R₄ für -H, -R₀, -COR₁₂ oder -S(=O)₂R₁₂ steht;
R₅, R₆, R₇, R₈, R₉, R₁₀, R₁₈ und R₁₉ jeweils unabhängig voneinander für -H, -F, -Cl, -Br, -I, -NO₂, -CF₃, -OR₁₃, -SR₁₃, -SO₂R₁₃, -S(=O)₂OR₁₃, -CN, -COOR₁₃, -CONR₁₃, -NR₁₄R₁₅, =O oder -R₀ stehen; oder R₅ und R₆ gemeinsam für -(CH₂)₂₋₆- stehen, wobei einzelne Wasserstoffatome auch durch -F, -Cl, -Br, -I, -NO₂, -CF₃, -OR₁₃, -CN oder -C₁₋₆-Aliphat ersetzt sein können;
R₁₁ jeweils unabhängig für -H, -R₀ oder -C(=O)R₀ steht;
R₁₂ jeweils unabhängig für -H, -R₀, -OR₁₃, oder -NR₁₄R₁₅ steht;
R₁₃ jeweils unabhängig für -H oder R₀ steht;
R₁₄ und R₁₅ unabhängig voneinander für -H oder R₀ stehen; oder R₁₄ und R₁₅ zusammen für -CH₂CH₂OCH₂CH₂-, -CH₂CH₂NR₁₆CH₂CH₂- oder -(CH₂)₃₋₆- stehen;
R₁₆ für -H oder -C₁₋₈-Aliphat steht;
R₁₇ für -H, -R₀, -COR₁₂ oder -S(=O)₂R₁₂ steht;
wobei
"Aliphat" jeweils ein verzweigter oder unverzweigter, gesättigter oder ein ein- oder mehrfach ungesättigter, unsubstituierter oder ein- oder mehrfach substituierter, aliphatischer Kohlenwasserstoffrest ist;
"Cycloaliphat" jeweils ein gesättigter oder ein ein- oder mehrfach ungesättigter, unsubstituierter oder ein- oder mehrfach substituierter, alicyclischer, mono- oder multicyclischer Kohlenwasserstoffrest ist;
wobei in Bezug auf "Aliphat" und "Cycloaliphat" unter "ein- oder mehrfach substituiert" die ein- oder mehrfache Substitution eines oder mehrerer Wasserstoffatome durch -F, - Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)-OR₀, -OC(=0)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)-N(R₀)₂, -Si(R₀)₃ oder -PO(OR₀)₂ verstanden wird;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁. ₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)-N(R₀)₂, -Si(R₀)₃ und -PO(OR₀)₂ verstanden wird, wobei ggf. vorhandene N-Ringatome jeweils oxidiert sein können;
in Form eines einzelnen Stereoisomers oder deren Mischung, der freien Verbindungen und/oder ihrer physiologisch verträglichen Salze.

2. Verbindung nach Anspruch 1, welche die allgemeine Formel (1.1), (1.2), (1.3), (1.4), (1.5), (1.6) oder (1.7) 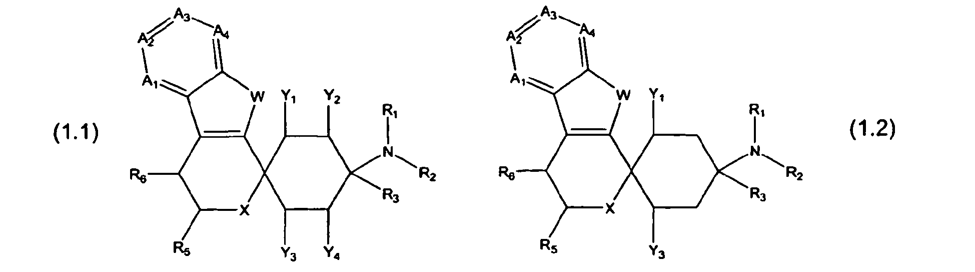 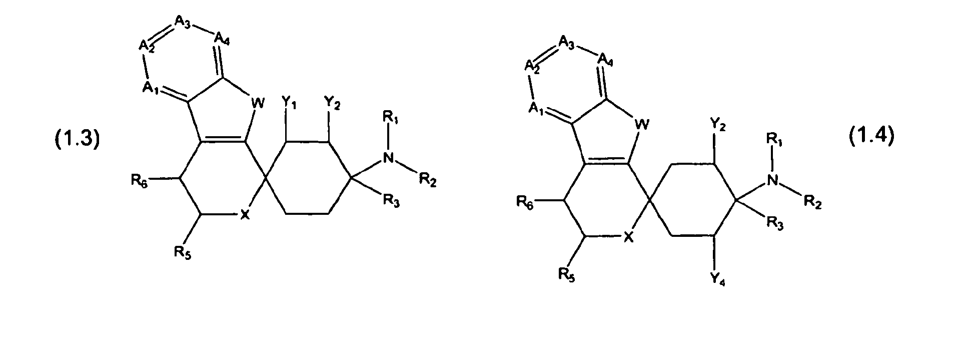 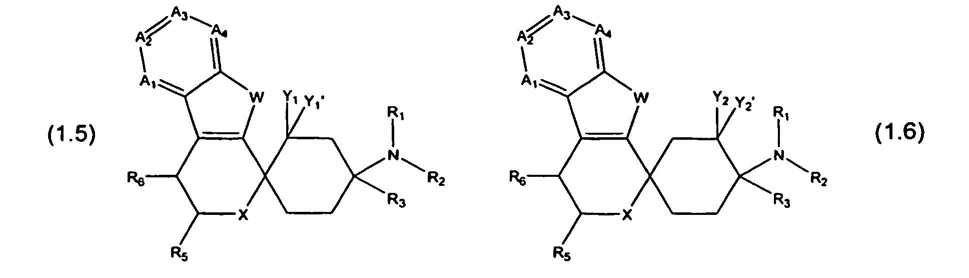 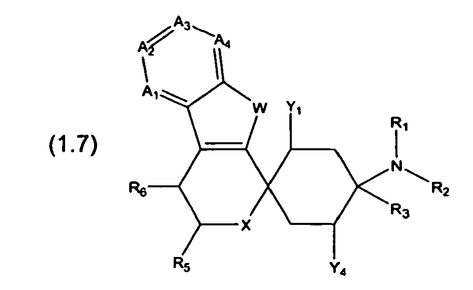 aufweist.

3. Verbindung nach Anspruch 1 oder 2, welche die allgemeine Formel (2) 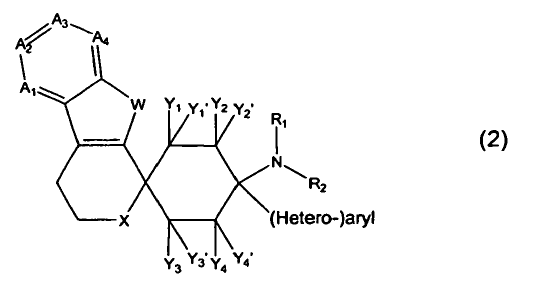 aufweist, worin (Hetero-)aryl für -Aryl oder -Heteroaryl steht.

4. Verbindung nach Anspruch 3, welche die allgemeine Formel (2.1), (2.2), (2.3) oder (2.4) 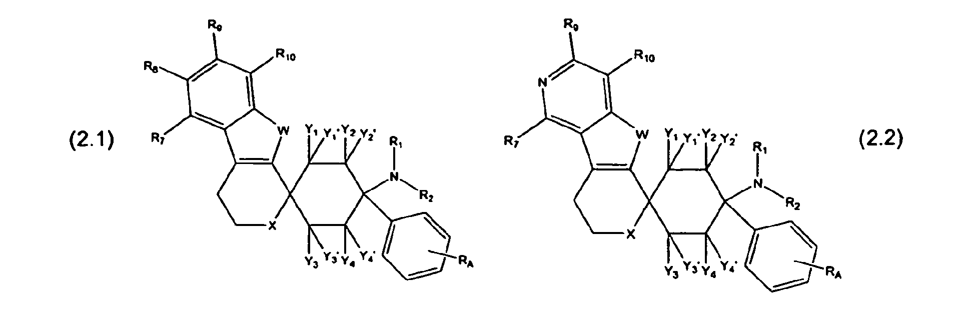 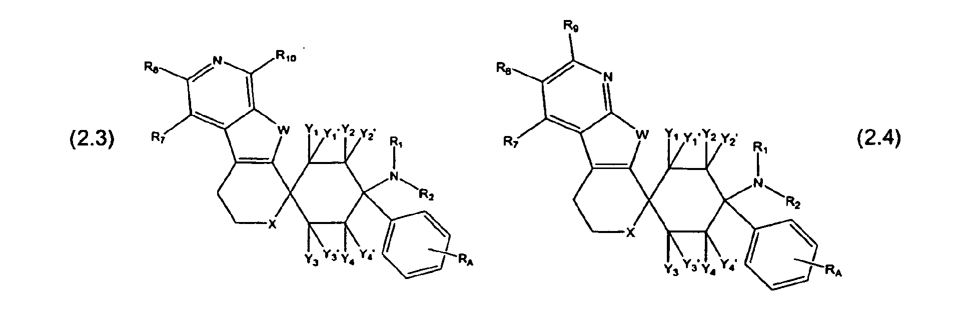 aufweist, worin R_{A} für -H, -F, -Cl, -CN oder -CH₃ steht.

5. Verbindung nach Anspruch 4, welche die allgemeine Formel (2.1.1) oder (2.1.4) 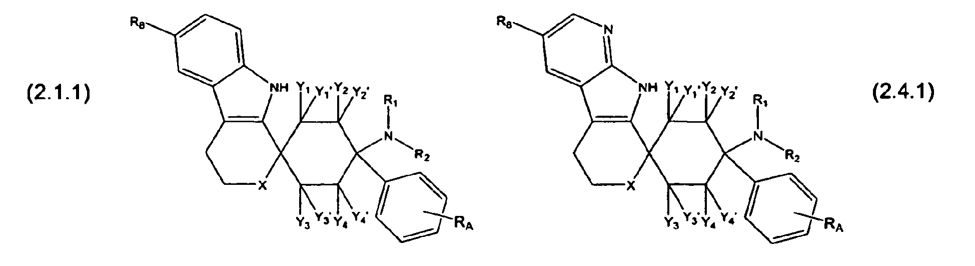 aufweist.

6. Verbindung nach Anspruch 5, worin
X für -O- oder -NR₁₇- steht;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
R₁ für -CH₃ steht;
R₂ für -H oder -CH₃ steht;
R₈ für -H oder -F steht;
R₁₂ jeweils unabhängig für -H, -R₀, -OR₁₃, oder -NR₁₄R₁₅ steht;
R₁₃ jeweils unabhängig für -H oder R₀ steht;
R₁₄ und R₁₅ unabhängig voneinander für -H oder R₀ stehen; oder R₁₄ und R₁₅ zusammen für -CH₂CH₂OCH₂CH₂-, -CH₂CH₂NR₁₆CH₂CH₂- oder -(CH₂)₃₋₆- stehen; R₁₆ für -H oder -C₁₋₆-Aliphat steht;
R₁₇ für -H, -R₀, -COR₁₂ oder -S(=O)₂R₁₂ steht; und
Y₁, Y₁', Y_{2,} Y₂', Y₃, Y₃', Y₄ und Y₄' jeweils unabhängig voneinander ausgewählt sind aus der Gruppe bestehend aus -H, -F, -Cl, -CN, -C₁₋₈-Aliphat, -C₁₋₈-Aliphat-NHC₁₋₈-Aliphat, - C₁₋₈-Aliphat-N(C₁₋₈-Aliphath, -S-C₁₋₈-Aliphat, -S-Aryl, -Aryl und -C₁₋₈-Aliphat-Aryl; mit der Maßgabe, dass wenigstens einer der Reste Y₁, Y₁', Y₂, Y₂', Y₃, Y₃', Y₄ und Y₄' ungleich - H ist; und
R_{A} für -H, -F, -Cl, -CN oder -CH₃ steht.

7. Verbindung nach Anspruch 1, ausgewählt aus der Gruppe bestehend aus
• (±)-N,N,2-Trimethyt-4-pheny)-4',9'-dihydro-3'H-spiro[cyclohexan-1,1'-pyrano[3,4-b]indol]-4-amin; 2-hydroxypropan-1,2,3-tricarboxylat;
• (±)-N,N,2-Trimethyl-4-phenyl-4',9'-dihydro-3'H-spiro[cyclohexan-1,1'-pyrano[3,4-b]indol]-4-amin; 2-hydroxypropan-1,2,3-tricarboxylat;
• (±)-2-Methyl-4-(dimethylamino)l-4-phenyl-spiro[cyclohexan-1,8'-(5,6,8,9-Tetra-hydro-pyrano[3,4-b]-7-aza-indol)]; 2-hydroxypropan-1,2,3-tricarboxylat;
• (±)-2-Benzyl-N,N-dimethyl-4-phenyl-4',9'-dihydro-3'H-spiro[cyclohexane-1,1'-pyrano[3,4-bjindol]-4-amin;
• (±)-2-Benzyl-N,N-dimethyl-4-phenyl-2',3',4',9'-tetrahydrospiro[cyclohexan-1,1'-pyrido[3,4-b]indol]-4-amin; 2-hydroxypropan-1,2,3-tricarboxylat;
• (±)-2-(3-Fluorbenzyl)-N,N-dimethyl-4-phenyl-4',9'-dihydro-3'H-spiro[cyclohexan-1,1'-pyrano[3,4-b]indol]-4-amin;
• N,N,3,5-Tetramethyl-4-phenyl-4',9'-dihydro-3'H-spiro[cyclohexan-1,1'-pyrano[3,4-b]indol]-4-amin;
• N,N,2,6-Tetramethyl-4-phenyl-4',9'-dihydro-3'H-spiro[cyclohexan-1,1'-pyrano[3,4-b]indol]-4-amin;
• N,N,2,5-Tetramethyl-4-phenyl-4',9'-dihydro-3'H-spiro[cyclohexan-1,1'-pyrano[3,4-b]indol]-4-amin;
• 2-Benzyl-N,N-dimethyl-4-phenyl-4',9'-dihydro-3'H-spiro[cyclohexan-1,1'-pyrano-[3,4-b]indol]-4-amin;
• 2-(4-Fluorobenzyl)-N,N-dimethyl-4-phenyl-4',9'-dihydro-3'H-spiro[cyclohexan-1,1'-pyrano[3,4-b]indol]-4-amin;
• N,N,2,3-Tetramethyl-4-phenyl-4',9'-dihydro-3'H-spiro[cyclohexan-1,1'-pyrano[3,4-b]indol]-4-amin;
• N,N-Dimethyl-3,4-diphenyl-4',9'-dihydro-3'H-spiro[cyclohexan-1,1'-pyrano[3,4-b]indol]-4-amin;
• 2-(4-Fluorophenyl)-N,N-dimethyl-4-phenyl-4',9'-dihydro-3'H-spiro(cyclohexan-1,1'-pyrano[3,4-b]indol]-4-amin;
• 3-((Dimethylamino)methylyN,N-dimethyl-4-phenyl-4',9'-dihydro-3'H-spiro[cyclohexan-1,1'-pyrano[3,4-b]indol]-4-amin;
• N,N,3-Trimethyl-4-phenyl-4',9'-dihydro-3'H-spiro[cyclohexan-1,1'-pyrano[3,4-b]indol]-4-amin;
• 3-Fluoro-N,N-dimethyl-4-phenyl-4',9'-dihydro-3'H-spiro[cyclohexan-1,1'-pyrano[3,4-b]indol]-4-amin
• 2-Fluoro-N,N-dimethyl-4-phenyl-4',9'-dihydro-3'H-spiro[cyclohexan-1,1'-pyrano[3,4-b]indol]-4-amin;
• N,N-Dimethyl-2-(methylthio)-4-phenyl-4',9'-dihydro-3'H-spiro[cyclohexan-1,1'-pyrano[3,4-b]indol]-4-amin;
• N,N-Dimethyl-4-phenyl-2-(phenylthio}-4',9'-dihydro-3'H-spiro[cyclohexan-1,1'-pyrano[3,4-b]indol]-4-amin;
• 3,3-Difluoro-N,N-dimethyl-4-phenyl-4',9'-dihydro-3'H-spiro[cyclohexan-1,1'-pyrano-[3,4-b]indol]-4-amin;
• 2,2-Difluoro-N,N-dimethyl-4-phenyl-4',9'-dihydro-3'H-spiro[cyclohexan-1,1'-pyrano-[3,4-b]indol]-4-amin;
• 2-Allyl-N,N-dimethyl-4-phenyl-4',9'-dihydro-3'H-spiro[cyclohexan-1,1'-pyrano[3,4-b]indol]-4-amin;
• N,N,3,5-Tetramethyl-4-phenyl-2',3',4',9'-tetrahydrospiro[cyclohexan-1,1'-pyrido[3,4-b]indol]-4-amin;
• N,N,2,6-Tetramethyl-4-phenyl-2',3',4',9'-tetrahydrospiro[cyclohexan-1,1'-pyrido[3,4-b]indol]-4-amin;
• N,N,2,5-Tetramethyl-4-phenyl-2',3',4',9'-tetrahydrospiro[cyclohexan-1,1'-pyrido[3,4-b]indol]-4-amin;
• 2-Benzyl-N,N-dimethyl-4-phenyl-2',3',4',9'-tetrahydrospiro[cyclohexan-1,1'-pyrido-[3,4-b]indol]-4-amin;
• 2-(4-Fluorobenzyl)-N,N-dimethyl-4-phenyl-2',3',4',9'-tetrahydrospiro[cyclohexan-1,1'-pyrido[3,4-b]indol]-4-amin;
• N,N,2,3-Tetramethyl-4-phenyl-2',3',4',9'-tetrahydrospiro[cyclohexan-1,1'-pyrido[3,4-b]indol]-4-amin;
• N,N-Dimethyl-3,4-diphenyl-2',3',4',9'-tetrahydrospiro[cyclohexan-1,1'-pyrido[3,4-b]indol]-4-amin;
• 2-(4-Fluorophenyl)-N,N-dimethyl-4-phenyl-2',3',4',9'-tetrahydrospiro[cyclohexan-1,1'-pyrido[3,4-b]indol]-4-amin;
• 3-((Dimethylamino)methyl)-N,N-dimethyl-4-phenyl-2',3',4',9'-tetrahydrospiro[cyclohexan-1,1'-pyrido[3,4-b]indol]-4-amin;
• N,N,3-Trimethyl-4-phenyl-2',3',4',9'-tetrahydrospiro[cyclohexan-1,1'-pyrido[3,4-b]indol]-4-amin;
• 3-Fluoro-N,N-dimethyl-4-phenyl-2',3',4',9'-tetrahydrospiro[cyclohexan-1,1'-pyrido-[3,4-b]indol]-4-amin;
• 2-Fluoro-N,N-dimethyl-4-phenyl-2',3',4',9'-tetrahydrospiro[cyclohexan-1,1'-pyrido-[3,4-b]indol]-4-amin;
• N,N-Dimethyl-2-(methylthio)-4-phenyl-2',3',4',9'-tetrahydrospiro[cyclohexan-1,1'-pyrido[3,4-b]indol]-4-amin;
• N,N-Dimethyl-4-phenyl-2-(phenylthio)-2',3',4',9'-tetrahydrospiro[cyclohexan-1,1'-pyrido[3,4-b]indol]-4-amin;
• 3,3-Difluoro-N,N-dimethyl-4-phenyl-2',3',4',9'-tetrahydrospiro[cyclohexan-1,1'-pyrido[3,4-b]indol]-4-amin;
• 2,2-Difluoro-N,N-dimethyl-4-phenyl-2',3',4',9'-tetrahydrospiro[cyclohexan-1,1'-pyrido[3,4-b]indol]-4-amin;
• 2-Allyl-N,N-dimethyl-4-phenyl-2',3',4',9'-tetrahydrospiro[cyclohexan-1,1'-pyrido[3,4-b]indol]-4-amin;
• 2-Benzyl-6'-fluor-N,N-dimethyl-4-phenyl-4',9'-dihydro-3'H-spiro[cyclohexan-1,1'-pyrano[3,4-b]indol]-4-amin;
• 6'-Fluor-2-(3-fluorbenzyl)-N,N-dimethyl-4-phenyl-4',9'-dihydro-3'H-spiro[cyclohexan-1,1'-pyrano[3,4-b]indol]-4-amin;
• 2-(3-Fluorbenzyl)-N,N-dimethyl-4-phenyl-2',3',4',9'-tetrahydrospiro[cyclohexan-1,1'-pyrido[3,4-b]indol]-4-amin;
• 3,6'-Difluor-N,N-dimethyl-4-phenyl-4',9'-dihydro-3'H-spiro[cyclohexan-1,1'-pyrano-[3,4-b]indol]-4-amin;
• 2,6'-Difluor-N,N-dimethyl-4-phenyl-4',9'-dihydro-3'H-spiro[cyclohexan-1,1'-pyrano-[3,4-b]indol]-4-amin;
• 3,3,6'-Trifluoro-N,N-dimethyl-4-phenyl-4',9'-dihydro-3'H-spiro[cyclohexan-1,1'-pyrano[3,4-b]indol]-4-amin;
• 2,2,6'-Trifluoro-N,N-dimethyl-4-phenyl-4',9'-dihydro-3'H-spiro[cyclohexan-1,1'-pyrano[3,4-b]indol]-4-amin;
• 2,6,6'-Trifluoro-N,N-dimethyl-4-phenyl-4',9'-dihydro-3'H-spiro[cyclohexan-1,1'-pyrano[3,4-b]indol]-4-amin;
• 2,2,6'-Trifluoro-N-methyl-4-phenyl-4',9'-dihydro-3'H-spiro[cyclohexan-1,1'-pyrano-[3,4-b]indol]-4-amin;
• 2,6'-Difluoro-N-methyl-4-phenyl-4',9'-dihydro-3'H-spiro[cyclohexan-1,1'-pyrano[3,4-b]indol]-4-amin;
• 2,6,6'-Trifluoro-N-methyl-4-phenyl-4',9'-dihydro-3'H-spiro[cyclohexan-1,1'-pyrano-[3,4-b]indol]-4-amin;
• 3,3,6'-Trifluoro-N,N-dimethyl-4-(thiophen-2-yl)-4',9'-dihydro-3'H-spiro[cyclohexan-1,1'-pyrano[3,4-b]indol]-4-amin;
• 4-Butyl-3,3,6'-trifluoro-N,N-dimethyl-4',9'-dihydro-3'H-spiro[cyclohexan-1,1'-pyrano-[3,4-b]indol]-4-amin;
• 2,2,6'-Trifluoro-N,N-dimethyl-4-(thiophen-2-yl)-4',9'-dihydro-3'H-spiro[cyclohexan-1,1'-pyrano[3,4-b]indol]-4-amin;
• 4-Butyl-2,2,6'-trifluoro-N,N-dimethyl-4',9'-dihydro-3'H-spiro[cyclohexan-1,1'-pyrano-[3,4-b]indol]-4-amin;
• 2,6,6'-Trifluoro-N,N-dimethyl-4-(thiophen-2-yl)-4',9'-dihydro-3'H-spiro[cyclohexan-1,1'-pyrano[3,4-b]indol]-4-amin;
• 4-Butyl-2,6,6'-trifluoro-N,N-dimethyl-4',9'-dihydro-3'H-spiro[cyclohexan-1,1'-pyrano-[3,4-b]indol]-4-amin;
• 3,6'-Difluoro-N,N-dimethyl-4-(thiophen-2-yl)-4',9'-dihydro-3'H-spiro[cyclohexan-1,1'-pyrano[3,4-b]indol]-4-amin;
• 4-Butyl-3,6'-difluoro-N,N-dimethyl-4',9'-dihydro-3'H-spiro[cyclohexan-1,1'-pyrano-[3,4-b]indol]-4-amin;
• 2,6'-Difluoro-N,N-dimethyl-4-(thiophen-2-yl)-4',9'-dihydro-3'H-spiro[cyclohexan-1,1'-pyrano[3,4-b]indol]-4-amin;
• 4-Butyl-2,6'-difluoro-N,N-dimethyl-4',9'-dihydro-3'H-spiro[cyclohexan-1,1'-pyrano-[3,4-b]indol]-4-amin;
• 3,3,6'-Trifluoro-4-(3-fluorophenyl)-N,N-dimethyl-2',3',4',9'-tetrahydrospiro[cyclohexan-1,1'-pyrido[3,4-b]indol]-4-amin;
• 2,2,6'-Trifluoro-4-(3-fluorophenyl)-N,N-dimethyl-2',3',4',9'-tetrahydrospiro[cyclohexan-1,1'-pyrido[3,4-b]indol]-4-amin;
• 3,6'-Difluoro-4-(3-fluorophenyl)-N,N-dimethyl-2',3',4',9'-tetrahydrospiro[cyclohexan-1,1'-pyrido[3,4-b]indol]-4-amin;
• 2,6'-Difluoro-4-(3-fluorophenyl)-N,N-dimethyl-2',3,4',9'-tetrahydrospiro[cyclohexan-1,1'-pyrido[3,4-b]indol]-4-amin;
• 2,6,6'-Trifluoro-4-(3-fluorophenyl)-N,N-dimethyl-2',3',4',9'-tetrahydrospiro[cyclohexan-1,1'-pyrido[3,4-b]indol]-4-amin;
• 3,3-Difluoro-N,N-dimethyl-4-phenyl-2',3',4',9'-tetrahydrospiro[cyclohexan-1,1'-pyrido[3,4-b]indol]-4-amin;
• 2,2-Difluoro-4-{3-fluorophenyl)-N,N-dimethyl-2',3',4',9'-tetrahydrospiro[cyclohexan-1,1'-pyrido[3,4-b]indol]-4-amin;
• 3-Fluoro-4-(3-fluorophenyl)-N,N-dimethyl-2',3',4',9'-tetrahydrospiro[cyclohexan-1,1'-pyrido[3,4-b]indol]-4-amin;
• 2-Fluoro-4-(3-fluorophenyl)-N,N-dimethyl-2',3',4',9'-tetrahydrospiro[cyclohexan-1,1'-pyrido[3,4-b]indol]-4-amin;
• 2,6-Difluoro-4-(3-fluorophenyl)-N,N-dimethyl-2',3',4',9'-tetrahydrospiro[cyclohexan-1,1'-pyrido[3,4-b]indol]-4-amin;
• (E)-1-(4-(Dimethylamino)-3-fluoro-4-(3-fluorophenyl)-3',4'-dihydrospiro[cyclohexan-1,1'-pyrido[3,4-b]indol]-2'(9'H )-yl)-3-phenylprop-2-en-1-on;
• (E)-1-(4-(Dimethylamino)-2-fluoro-4-(3-fluorophenyl)-3',4'-dihydrospiro[cyclohexan-1,1'-pyrido[3,4-b]indol]-2'(9'Hryl)-3-phenylprop-2-en-1-on;
• (E)-1-(4-(Dimethylamino)-3,3-difluoro-4-(3-fluorophenyl)-3',4'-dihydrospiro[cycloHexan-1,1'-pyrido[3,4-b]indol)-2'(9'H)-yl)-3-phenylprop-2-en-1-on;
• (E)-1-(4-(Dimethylamino)-2,2-difluoro-4-(3-fluorophenyl)-3',4'-dihydrospiro[cycloHexan-1,1'-pyrido[3,4-b]indol]-2'(9'H)-yl)-3-phenylprop-2-en-1-on;
• (E)-1-(4-(Dimethylamino)-2,6-difluoro-4-(3-fluorophenyl)-3',4'-dihydrospiro[cyclohexan-1,1'-pyrido[3,4-b]indol]-2'(9'H-yl)-3-phenylprop-2-en-1-on;
• (E)-1-(4-(Dimethylamino)-3,6'-difluoro-4-(3-fluorophenyl)-3',4'-dihydrospiro[cycloHexan-1,1'-pyrido[3,4-b]indol]-2'(9'H)-yl)-3-phenylprop-2-en-1-on;
• (E)-1-(4-(Dimethylamino)-2,6'-difluoro-4-(3-fluorophenyl)-3',4'-dihydrospiro[cyclohexan-1,1'-pyrido[3,4-b]indol]-2'(9'H)-yl )-3-phenylprop-2-en-1-on;
• (E)-1-(4-(D)methy)amino)-3,3,6'-trif)uoro-4-(3-fiuorophenyl)-3',4'-dihydrospiro[cyclohexan-1,1'-pyrido[3,4-b]indol]-2'(9'H)-yl)-3-phenylprop-2-en-1-on;
• (E)-1-(4-(Dimethylamino)-2,2,6'-trifluoro-4-(3-fluorophenyl)-3',4'-dihydrospiro[cyclohexan-1,1'-pyrido[3,4-b]indol]-2'(9'H)-yl)-3-phenylprop-2-en-1-on;
• (E)-1-(4-(Dimethylamino)-2,6,6'-trifluoro-4-(3-fluorophenyl)-3',4'-dihydrospiro[cyclohexan-1,1'-pyrido[3,4-b]indol]-2'(9'H)-yl)-3-phenylprop-2-en-1-on;
• 2-Methyl-4-(dimethylamino)1-4-phenyl-spiro[cyclohexan-1,8'-(5,6,7,8,9-Penta-hydro-pyrido[3,4-b]-7-aza-indol)]:
• N,N,2-Trimethyl-4-phenyl-2',3',4',9'-tetrahydrospiro[cyclohexan-1,1'-pyrido[3,4-b]indol]-4-amin;
• 2-Benzyl-N,N-dimethyl-4-phenyl-2',3',4',9'-tetrahydrospiro[cyclohexan-1,1'-pyrido-[3,4-b]indol]-4-amin; und
• 2-Benzyl-N,N-dimethyl-4-phenyl-2',3',4',9'-tetrahydrospiro[cyclohexan-1,1'-pyrido-[3,4-b]indol]-4-amin;
• 6'-Fluor-2g3-fluorbenzyl)-N,N-dimethyl-4-phenyl-4',9'-dihydrospiro[cyclo-hexan-1,1'-pyrano-[3,4-b]indol]-4-amin
• 2-(3-Fluorbenzyl)-N,N-dimethyl-4-phenyl-2',3',4',9'-tetrahydrospiro[cyclohexan-1,1'-pyrido[3,4-b]indol]-4-amin
• N,N-Dimethyl-4-phenyl-2-(phenylthio)-4',9'-dihydro-3'H-spiro-[cyclohexan-1,1'-pyrano[3,4-b]indol]-4-amin
• N,N-Dimethyl-4-phenyl-2-(phenylthio)-2',3',4',9'-tetrahydrospiro[cyclohexan-1,1'-pyrido[3,4-b]indol]-4-amin
• 2-(3-Fluorbenzyl)-N,N-dimethyl-4-phenyl-2',3',4',9'-tetrahydrospiro[cyclohexan-1,1'-pyrido[3,4-b]indol]-4-amin
• 2-(4-(Dimethylamino)-4-phenyl-4',9'-dihydro-3'H-spiro[cyclohexan-1,1'-pyrano[3,4-b]indol]-2-yl)essigsäure-methylester
• 2-Allyl-N,N-dimethyl-4-phenyl-4',9'-dihydros-3'H-piro[cyclohexan-1,1'-pyrano[3,4-b]indol]-4-amin
• 2-Fluor-N,N-dimethyl-4-phenyl-4',9'-dihydro-3'H-spiro[cyclohexan-1,1'-pyrano[3,4-b]indol]-4-amin
• 2-Allyl-N,N-dimethyl-4-phenyl-4',9'-dihydros-3'H-piro[cyclohexan-1,1'-pyrano[3,4-b]indol]-4-amin
• 2,6'-Difluor-N,N-dimethyl-4-phenyl-4',9'-dihydro-3'H-spiro[cyclohexan-1,1'-pyrano[3,4-b]indol]-4-amin
• 4-(Dimethylamino)-4-phenyl-4',9'-dihydro-3'H-spiro[cyclohexan-1,1'-pyrano[3,4-b]indol]-2-yl)methanol
• 2,6'-Difluor-N,N-dimethyl-4-phenyl-4',9'-dihydro-3'H-spiro[cyclohexan-1,1'-pyrano[3,4-b]indol]-4-amin
• 2-Fluor-N,N-dimethyl-4-phenyl-2',3',4',9'-tetrahydrospiro[cyclohexan-1,1'-pyrido[3,4-b]indol]-4-amin
• 2-Fluor-N,N-dimethyl-4-phenyl-2',3',4',9'-tetrahydrospiro[cyclohexan-1,1'-pyrido[3,4-b]indol]-4-amin
• 2-(4-(Dimethylamino)-4-phenyl-4',9'-dihydro-3'H-spiro[cyclohexan-1,1'-pyrano[3,4-b]indol]-2-yl)ethanol
• 2-(4-(Dimethylamino)-4-phenyl-4',9'-dihydro-3'H-spiro[cyclohexan-1,1'-pyrano[3,4-b]indol]-2-yl)methyl)isoindolin-1,3-dion
• N-((4-(Dimethylamino)-4-(3-fluorphenyl)-4',9'-dihydro-3'H-spiro[cyclohexan-1,1'-pyrano[3,4-b]indol]-2-yl)methyl)-3-phenylzimtsäureamid
• tert-Butyl2-(2-(4-(dimethylamino)-4-phenyl-4',9'-dihydro-3'H-spiro[cyclohexan-1,1'-pyrano[3,4-b]indol]-2-yl)ethoxy)acetat
• 2-(4-(Dimethylamino)-4-phenyl-4',9'-dihydro-3'H-spiro[cyclohexan-1,1'-pyrano[3,4-b]indol]-2-yl)acetonitril
oder deren physiologisch verträgliche Salze.

8. Arzneimittel enthaltend wenigstens eine Verbindung nach einem der Ansprüche 1 bis 7 in Form eines einzelnen Stereoisomers oder deren Mischung, der freien Verbindungen und/oder ihrer physiologisch verträglichen Salze, sowie ggf. geeignete Zusatz- und/oder Hilfsstoffe und/oder gegebenenfalls weiterer Wirkstoffe.

9. Verwendung einer Verbindung nach einem der Ansprüche 1 bis 7 in Form eines einzelnen Stereoisomers oder deren Mischung, der freien Verbindungen und/oder ihrer physiologisch verträglichen Salze, zur Herstellung eines Arzneimittels zur Behandlung von Schmerz.

10. Verwendung einer Verbindung nach einem der Ansprüche 1 bis 7 in Form eines einzelnen Stereoisomers oder deren Mischung, der freien Verbindungen und/oder ihrer physiologisch verträglichen Salze zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmissbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anästhetikum bzw. zur Coadministration bei Behandlung mit einem opioiden Analgetikum oder mit einem Anästhetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

1. Compound of the general formula (1) 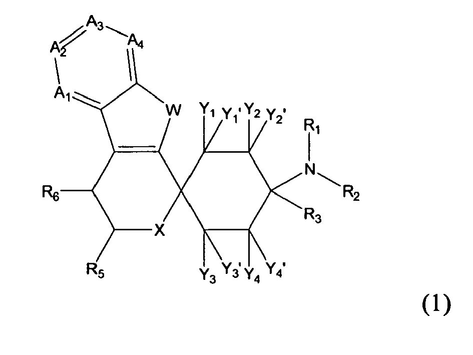 wherein
A₁ stands for -N= or -CR₇=,
A₂ stands for -N= or -CR₈=,
A₃ stands for -N= or -CR₉=,
A₄ stands for -N= or -CR₁₀=,
on condition that at most two of the residues A₁, A₂, A₃ and A₄ stand for -N=;
W stands for -NR₄-, -O- or -S-;
X stands for -NR₁₇-, -O-, -S(=O)₀₋₂- or -CR₁₈R₁₉-;
Y₁, Y₁', Y₂, Y₂', Y₃, Y₃', Y₄ and Y₄ are respectively selected independently of one another from the group comprising -H, -F, -Cl, -Br, -I, -CN, -NO₂, -CHO, -R₀, -C(=O)R₀, -C(=O)-H, -C(=O)-OH, -C(=O)OR₀, -C(=O)NH₂, -C(=O)NHR₀, -C(=O)N(R₀)₂, -OH, -OR₀, -OC(=O)-R₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O-, -NHC(=O)R₀, -NHC(=O)OR₀, -NHC(=O)NH₂, -NHC(=O)NHR₀ and -NHC(=O)-N(R₀)₂; or Y₁ and Y₁', or Y₂ and Y₂', or Y₃ and Y₃', or Y₄ and Y₄' jointly stand for =O;
on condition that at least one of the residues Y₁, Y₁', Y₂, Y₂', Y₃, Y₃', Y₄ and Y₄' does not stand for -H;
R₀ respectively independently stands for -C₁₋₈ -aliphatic, -C₃₋₁₂ -cycloaliphatic, -aryl, heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁-₈-aliphatic, -C₃₋₈-cycloaliphatic-aryl or -C₃₋₈-cycloaliphatic-heteroaryl;
R₁ and R₂, independently of one another, stand for -H or -R₀; or R₁ and R₂ together stand for -CH₂CH₂OCH₂CH₂-, -CH₂CH₂NR₁₁CH₂CH₂- or -(-CH2)₃₋₆-;
R₃ stands for -R₀;
R₄ stands for -H, -R₀, -COR₁₂ or -S(=O)₂R₁₂
R₅, R₆, R₇, R₈, R₉, R₁₀, R₁₈ and R₁₉, respectively independently of one another, stand for -H, -F, -Cl, -Br, -I, -NO₂, -CF₃, -OR₁₃, -SR₁₃, -SO₂R₁₃, -S(=O)₂OR₁₃, -CN, - COOR₁₃, -CONR₁₃, -NR₁₄R₁₅, =O or -R₀; or R₅ and R₆ jointly stand for -(CH₂)₂₋₆-, wherein individual hydrogen atoms can also be replaced by -F, -Cl, -Br, -I, -NO₂, - CF₃, -OR₁₃, -CN or -C₁₋₆-aliphatic;
R₁₁ respectively independently stands for -H, -R₀ or -C(=O)R₀;
R₁₂ respectively independently stands for -H, -R₀, -OR₁₃, or -NR₁₄R₁₅;
R₁₃ respectively independently stands for -H or R₀;
R₁₄ and R₁₅ respectively independently of one another stand for -H or R₀; or R₁₄ and R₁₅ together stand for -CH₂CH₂OCH₂CH₂-, -CH₂CH₂NR₁₆CH₂CH₂- or -(-CH2)₃₋₆-;
R₁₆ stands for -H or -C₁₋₆-aliphatic;
R₁₇ stands for -H, -R₀, -COR₁₂ or -S(=O)₂R₁₂;
wherein
"aliphatic" respectively is a branched or unbranched, saturated or a mono- or polyunsaturated, unsubstituted or mono- or polysubstituted, aliphatic hydrocarbon residue;
"cycloaliphatic" respectively is a saturated or a mono- or polyunsaturated, unsubstituted or mono- or polysubstituted, alicyclic, mono- or multicyclic hydrocarbon residue;
wherein with respect to "aliphatic" and "cycloaliphatic", "mono- or polysubstituted" is understood to mean the mono- or polysubstitution of one or more hydrogen atoms by -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)-OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)-N(R₀)₂, -Si(R₀)₃ or -PO(OR₀)₂;
"aryl", respectively independently, stands for a carbocyclic ring system with at least one aromatic ring, but without heteroatoms in this ring, wherein, if necessary, the aryl residues can be condensed with further saturated, (partially) unsaturated or aromatic ring systems, and each aryl residue can be present in unsubstituted or mono- or polysubstituted form, wherein the aryl substituents can be the same or different and in any desired and possible position of the aryl;
"heteroaryl" stands for a 5-, 6- or 7-membered cyclic aromatic residue, which contains 1, 2, 3, 4 or 5 heteroatoms, wherein the heteroatoms, the same or different, are nitrogen, oxygen or sulphur, and the heterocycle can be unsubstituted or mono- or polysubstituted; wherein in the case of the substitution on the heterocycle the substituents can be the same or different and can be in any desired and possible position of the heteroaryl; and wherein the heterocycle can also be part of a bi- or polycyclic system;
wherein with respect to "aryl" and "heteroaryl", "mono- or polysubstituted" is understood to mean the mono- or polysubstitution of one or more hydrogen atoms of the ring system by substituents selected from the group comprising -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)-N(R₀)₂, -Si(R₀)₃ and -PO(OR₀)₂; wherein if necessary N-ring atoms present can be respectively oxidised;
in the form of a single stereoisomer or mixture thereof, the free compounds and/or their physiologically compatible salts.

2. Compound according to claim 1, which has the general formula (1.1), (1.2), (1.3), (1.4), (1.5), (1.6) or (1.7)

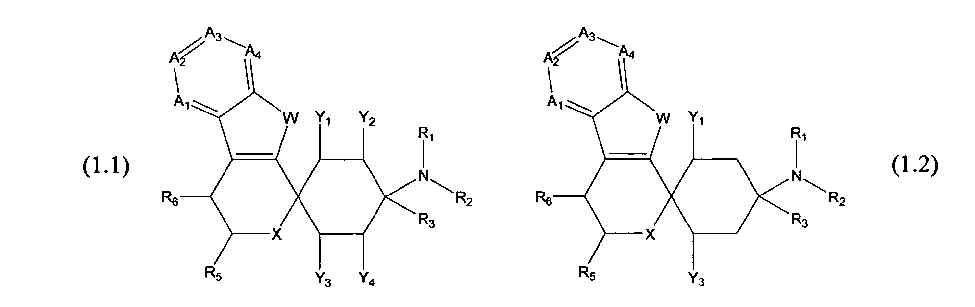

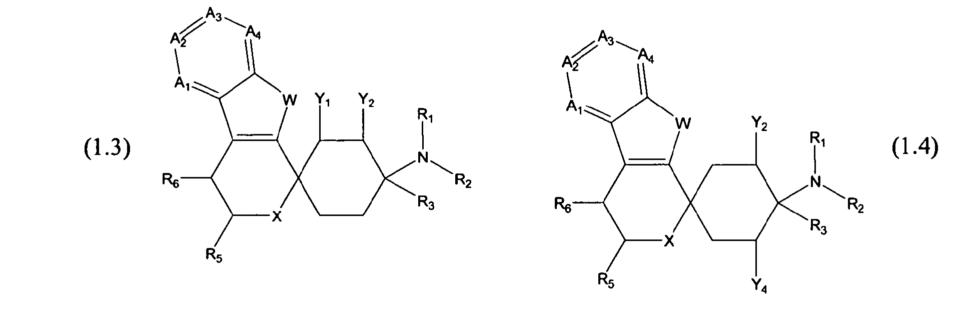

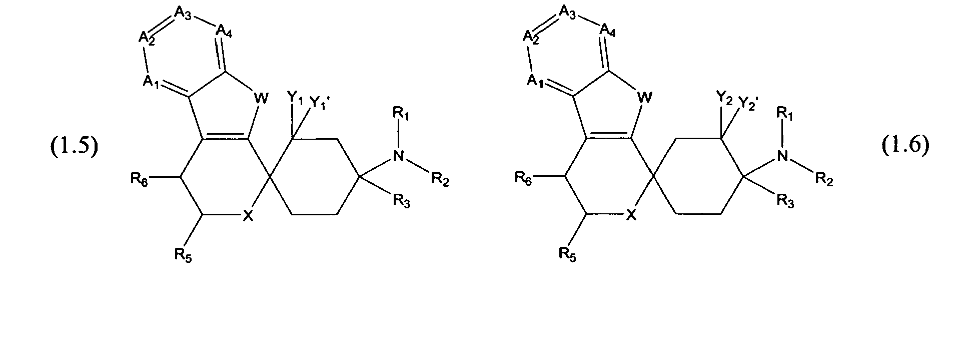

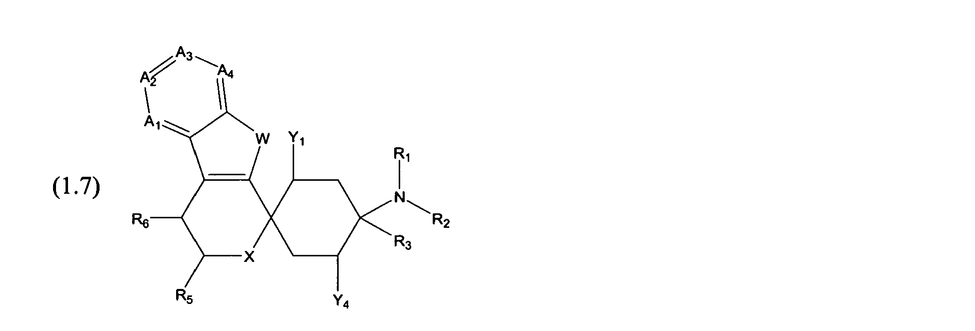

3. Compound according to claim 1 or 2, which has the general formula (2) 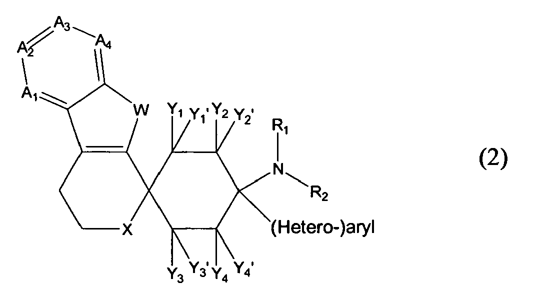 wherein (hetero)aryl stands for -aryl or -heteroaryl.

4. Compound according to claim 3, which has the general formula (2.1), (2.2), (2.3) or (2.4) 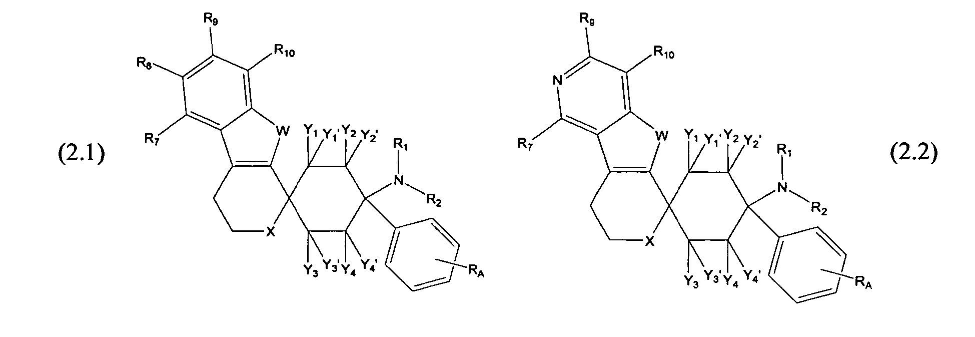 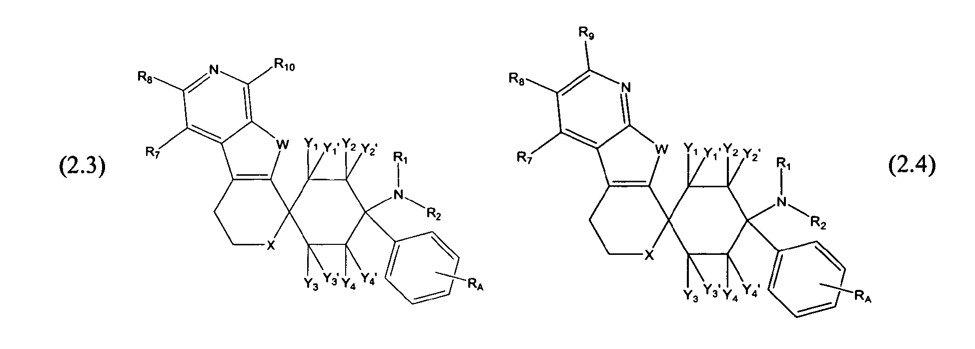 wherein R_{A} stands for -H, -F, -Cl, -CN or -CH₃.

5. Compound according to claim 4, which has the general formula (2.1.1) or (2.1.4)

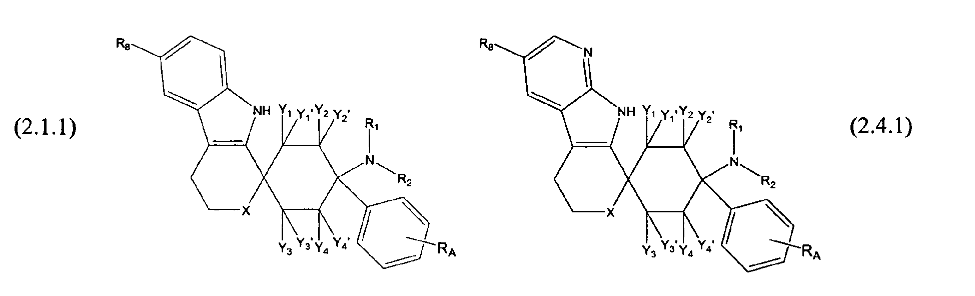

6. Compound according to claim 5, wherein
X stands for -O- or -NR₁₇-;
R₀ respectively independently stands for -C₁₋₈-aliphatic, -C₃₋₁₂-cycloaliphatic, -aryl, -heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁₋₈-aliphatic, -C₃₋₈-cycloaliphatic-aryl or -C₃₋₈-cycloaliphatic-heteroaryl;
R₁ stands for -CH₃;
R₂ stands for -H or -CH₃;
R₈ stands for -H or -F;
R₁ respectively independently stands for -H, -R₀, -OR₁₃, or -NR₁₄R₁₅;
R₁ respectively independently stands for -H or R₀;
R₁₄ and R₁₅ respectively independently of one another stand for -H or R₀; or R₁₄ and R₁₅ together stand for -CH₂CH₂OCH₂CH₂-, -CH₂CH₂NR₁₆CH₂CH₂- or -(CH₂)₃₋₆-;
R₁₆ stands for -H or -C₁₋₆-aliphatic;
R₁₇ stands for -H, -R₀, -COR₁₂ or -S(=O)₂R₁₂; and
Y₁, Y₁, Y₂, Y₂', Y₃, Y₃', Y₄ and Y₄' are respectively selected independently of one another from the group comprising -H, -F, -Cl, -CN, -C₁₋₈-aliphatic, -C₁₋₈-aliphatic-NHC₁₋₈-aliphatic, -C₁₋₈-aliphatic-N(C₁₋₈-aliphatic)₂, -S-C)₁₋₈-aliphatic, -S-aryl, -aryl and -C₁₋₈-aliphatic-aryl; on condition that at least one of the residues Y₁, Y₁', Y₂, Y₂', Y₃, Y₃', Y₄ and Y₄' differs from -H; and
R_{A} stands for -H, -F, -Cl, -CN or -CH₃.

7. Compound according to claim 1, selected from the group comprising
• (±)-N,N,2-trimethyl-4-phenyl-4',9'-dihydro-3'H-spiro[cyclohexane-1,1'-pyrano[3,4-b]indole]-4-amine; 2-hydroxypropane-1,2,3-tricarboxylate;
• (±)-N,N,2-trimethyl-4-phenyl-4',9'-dihydro-3'H-spiro[cyclohexane-1,1'-pyrano[3,4-b]indole]-4-amine; 2-hydroxypropane-1,2,3-tricarboxylate;
• (±)-2- methyl-4-(dimethylamino)1-4-phenyl-spiro[cyclohexane-1,8'-(5,6,8,9-tetrahydro-pyrano[3,4-b]-7-aza-indole)]; 2-hydroxypropane-1,2,3-tricarboxylate;
• (±)-2-benzyl-N,N-dimethyl-4-phenyl-4',9'-dihydro-3'H-spiro[cyclohexane-1,1'-pyrano[3,4-b]indole]-4-amine;
• (±)-2-benzyl-N,N-dimethyl-4-phenyl-2',3',4',9'-tetrahydrospiro[cyclohexane-1,1'-pyrido[3,4-b]indole]-4-amine; 2-hydroxypropane-1,2,3-tricarboxylate;
• (±)-2-(3-fluorobenzyl)-N,N-dimethyl-4-phenyl-4',9'-dihydro-3'H-spiro[cyclohexane-1,1'-pyrano[3,4-b]indole]-4-amine;
• N,N,3,5-tetramethyl-4-phenyl-4',9'-dihydro-3'H-spiro[cyclohexane-1,1'-pyrano[3,4-b]indole]-4-amine;
• N,N,2,6-tetramethyl-4-phenyl-4',9'-dihydro-3'H-spiro[cyclohexane-1,1'-pyrano[3,4-b]indole]-4-amine;
• N,N,2,5-tetramethyl-4-phenyl-4',9'-dihydro-3'H-spiro[cyclohexane-1,1'-pyrano[3,4-b]indole]-4-amine;
• 2-benzyl-N,N-dimethyl-4-phenyl-4',9'-dihydro-3'H-spiro[cyclohexane-1,1'-pyrano[3,4-b]indole]-4-amine;
• 2-(4-fluorobenzyl)-N,N-dimethyl-4-phenyl-4',9'-dihydro-3'H-spiro[cyclohexane-1,1'-pyrano[3,4-b]indole]-4-amine;
• N,N,2,3-tetramethyl-4-phenyl-4',9'-dihydro-3'H-spiro[cyclohexane-1,1'-pyrano[3,4-b]indole]-4-amine;
• N,N-dimethyl-3,4-diphenyl-4',9'-dihydro-3'H-spiro[cyclohexane-1,1'-pyrano[3,4-b]indole]-4-amine;
• 2-(4-fluorophenyl)-N,N-dimethyl-4-phenyl-4',9'-dihydro-3'H-spiro[cyclohexane-1,1'-pyrano[3,4-b]indole]-4-amine;
• 3-((dimethylamino)methyl)-N,N-dimethyl-4-phenyl-4',9'-dihydro-3'H-spiro[cyclohexane-1,1'-pyrano[3,4-b]indole]-4-amine;
• N,N,3-trimethyl-4-phenyl-4',9'-dihydro-3'H-spiro[cyclohexane-1,1'-pyrano[3,4-b]indole]-4-amine;
• 3-fluoro-N,N-dimethyl-4-phenyl-4',9'-dihydro-3'H-spiro[cyclohexane-1,1'-pyrano[3,4-b]indole]-4-amine;
• 2-fluoro-N,N-dimethyl-4-phenyl-4',9'-dihydro-3'H-spiro[cyclohexane-1,1'-pyrano[3,4-b]indole]-4-amine;
• N,N-dimethyl-2-(methylthio)-4-phenyl-4',9'-dihydro-3'H-spiro[cyclohexane-1,1'-pyrano[3,4-b]indole]-4-amine;
• N,N-dimethyl-4-phenyl-2-(phenylthio)-4',9'-dihydro-3'H-spiro[cyclohexane-1,1'-pyrano[3,4-b]indole]-4-amine;
• 3,3-difluoro-N,N-dimethyl-4-phenyl-4',9'-dihydro-3'H-spiro[cyclohexane-1,1'-pyrano[3,4-b]indole]-4-amine;
• 2,2-difluoro-N,N-dimethyl-4-phenyl-4',9'-dihydro-3'H-spiro[cyclohexane-1,1'-pyrano[3,4-b]indole]-4-amine;
• 2-allyl-N,N-dimethyl-4-phenyl-4',9'-dihydro-3'H-spiro[cyclohexane-1,1'-pyrano[3,4-b]indole]-4-amine;
• N,N,3,5-tetramethyl-4-phenyl-2',3',4',9'-tetrahydrospiro[cyclohexane-1,1'-pyrido[3,4-b]indole]-4-amine;
• N,N,2,6-tetramethyl-4-phenyl-2',3',4',9'-tetrahydrospiro[cyclohexane-1,1'-pyrido[3,4-b]indole]-4-amine;
• N,N,2,5-tetramethyl-4-phenyl-2',3',4',9'-tetrahydrospiro[cyclohexane-1,1'-pyrido[3,4-b]indole]-4-amine;
• 2-benzyl-N,N-dimethyl-4-phenyl-2',3',4',9'-tetrahydrospiro[cyclohexane- ,1'-pyrido[3,4-b]indole]-4-amine;
• 2-(4-fluorobenzyl)-N,N-dimethyl-4-phenyl-2',3',4',9'-tetrahydrospiro-[cyclohexane-1,1'-pyrido[3,4-b]indole]-4-amine;
• N,N,2,3-tetramethyl-4-phenyl-2',3',4',9'-tetrahydrospiro[cyclohexane-1,1'-pyrido[3,4-b]indole]-4-amine;
• N,N-dimethyl-3,4-diphenyl-2',3',4',9'-tetrahydrospiro[cyclohexane-1,1'-pyrido[3,4-b]indole]-4-amine;
• 2-(4-fluorophenyl)-N,N-dimethyl-4-phenyl-2',3',4',9'-tetrahydrospiro-[cyclohexane-1,1'-pyrido[3,4-b]indole]-4-amine;
• 3-((dimethylamino)methyl)-N,N-dimethyl-4-phenyl-2',3',4',9'-tetrahydrospiro-[cyclohexane-1,1'-pyrido[3,4-b]indole]-4-amine;
• N,N,3-trimethyl-4-phenyl-2',3',4',9'-tetrahydrospiro[cyclohexane-1,1'-pyrido[3,4-b]indole]-4-amine;
• 3-fluoro-N,N-dimethyl-4-phenyl-2',3',4',9'-tetrahydrospiro[cyclohexane-1,1'-pyrido[3,4-b]indole]-4-amine;
• 2-fluoro-N,N-dimethyl-4-phenyl-2',3',4',9'-tetrahydrospiro[cyclohexane-1,1'-pyrido[3,4-b]indole]-4-amine;
• N,N-dimethyl-2-(methylthio)-4-phenyl-2',3',4',9'-tetrahydrospiro[cyclohexane-1,1'-pyrido[3,4-b]indole]-4-amine;
• N,N-dimethyl-4-phenyl-2-(phenylthio)-2',3',4',9'-tetrahydrospiro[cyclohexane-1,1'-pyrido[3,4-b]indole]-4-amine;
• 3,3-difluoro-N,N-dimethyl-4-phenyl-2',3',4',9'-tetrahydrospiro[cyclohexane-1,1'-pyrido[3,4-b]indole]-4-amine;
• 2,2-difluoro-N,N-dimethyl-4-phenyl-2',3',4',9'-tetrahydrospiro[cyclohexane-1,1'-pyrido[3,4-b]indole]-4-amine;
• 2-allyl-N,N-dimethyl-4-phenyl-2',3',4',9'-tetrahydrospiro[cyclohexane-1,1'-pyrido[3,4-b]indole]-4-amine;
• 2-benzyl-6'-fluoro-N,N-dimethyl-4-phenyl-4',9'-dihydro-3'H-spiro-[cyclohexane-1,1'-pyrano[3,4-b]indole]-4-amine;
• 6'-fluoro-2-(3-fluorobenzyl)-N,N-dimethyl-4-phenyl-4',9'-dihydro-3'H-spiro[cyclohexane-1,1'-pyrano[3,4-b]indole]-4-amine;
• 2-(3-fluorobenzyl)-N,N-dimethyl-4-phenyl-2',3',4',9'-tetrahydrospiro-[cyclohexane-1,1'-pyrido[3,4-b]indole]-4-amine;
• 3,6'-difluoro-N,N-dimethyl-4-phenyl-4',9'-dihydro-3'H-spiro[cyclohexane-1,1'-pyrano[3,4-b]indole]-4-amine;
• 2,6'-difluoro-N,N-dimethyl-4-phenyl-4',9'-dihydro-3'H-spiro[cyclohexane-1,1'-pyrano[3,4-b]indole]-4-amine;
• 3,3,6'-trifluoro-N,N-dimethyl-4-phenyl-4',9'-dihydro-3'H-spiro[cyclohexane-1,1'-pyrano[3,4-b]indole]-4-amine;
• 2,2,6'-trifluoro-N,N-dimethyl-4-phenyl-4',9'-dihydro-3'H-spiro[cyclohexane-1,1'-pyrano[3,4-b]indole]-4-amine;
• 2,6,6'-trifluoro-N,N-dimethyl-4-phenyl-4',9'-dihydro-3'H-spiro[cyclohexane-1,1'-pyrano[3,4-b]indole]-4-amine;
• 2,2,6'-trifluoro-N-methyl-4-phenyl-4',9'-dihydro-3'H-spiro[cyclohexane-1,1'-pyrano[3,4-b]indole]-4-amine;
• 2,6'-difluoro-N-methyl-4-phenyl-4',9'-dihydro-3'H-spiro[cyclohexane-1,1'-pyrano[3,4-b]indole]-4-amine;
• 2,6,6'-trifluoro-N-methyl-4-phenyl-4',9'-dihydro-3'H-spiro[cyclohexane-1,1'-pyrano[3,4-b]indole]-4-amine;
• 3,3,6'-trifluoro-N,N-dimethyl-4-(thiophen-2-yl)-4',9'-dihydro-3'H-spiro[cyclohexane-1,1'-pyrano[3,4-b]indole]-4-amine;
• 4-butyl-3,3,6'-trifluoro-N,N-dimethyl-4',9'-dihydro-3'H-spiro[cyclohexane-1,1'-pyrano[3,4-b]indole]-4-amine;
• 2,2,6'-trifluoro-N,N-dimethyl-4-(thiophen-2-yl)-4',9'-dihydro-3'H-spiro[cyclohexane-1,1'-pyrano[3,4-b]indole]-4-amine;
• 4-butyl-2,2,6'-trifluoro-N,N-dimethyl-4',9'-dihydro-3'H-spiro[cyclohexane-1,1'-pyrano[3,4-b]indole]-4-amine;
• 2,6,6'-trifluoro-N,N-dimethyl-4-(thiophen-2-yl)-4',9'-dihydro-3'H-spiro-[cyclohexane-1,1'-pyrano[3,4-b]indole]-4-amine;
• 4-butyl-2,6,6'-trifluoro-N,N-dimethyl-4',9'-dihydro-3'H-spiro[cyclohexane-1,1'-pyrano[3,4-b]indole]-4-amine;
• 3,6'-difluoro-N,N-dimethyl-4-(thiophen-2-yl)-4',9'-dihydro-3'H-spiro-[cyclohexane-1,1'-pyrano[3,4-b]indole]-4-amine;
• 4-butyl-3,6'-difluoro-N,N-dimethyl-4',9'-dihydro-3'H-spiro[cyclohexane-1,1'-pyrano[3,4-b]indole]-4-amine;
• 2,6'-difluoro-N,N-dimethyl-4-(thiophen-2-yl)-4',9'-dihydro-3'H-spiro-[cyclohexane-1,1'-pyrano[3,4-b]indole]-4-amine;
• 4-butyl-2,6'-difluoro-N,N-dimethyl-4',9'-dihydro-3'H-spiro[cyclohexane-1,1'-pyrano[3,4-b]indole]-4-amine;
• 3,3,6'-trifluoro-4-(3-fluorophenyl)-N,N-dimethyl-2',3',4',9'-tetrahydrospiro-[cyclohexane-1,1'-pyrido[3,4-b]indole]-4-amine;
• 2,2,6'-trifluoro-4-(3-fluorophenyl)-N,N-dimethyl-2',3',4',9'-tetrahydrospiro-[cyclohexane-1,1'-pyrido[3,4-b]indole]-4-amine;
• 3,6'-difluoro-4-(3-fluorophenyl)-N,N-dimethyl-2',3',4',9'-tetrahydrospiro-[cyclohexane-1,1'-pyrido[3,4-b]indole]-4-amine;
• 2,6'-difluoro-4-(3-fluorophenyl)-N,N-dimethyl-2',3',4',9'-tetrahydrospiro-[cyclohexane-1,1'-pyrido[3,4-b]indole]-4-amine;
• 2,6,6'-trifluoro-4-(3-fluorophenyl)-N,N-dimethyl-2',3',4',9'-tetrahydrospiro-[cyclohexane-1,1'-pyrido[3,4-b]indole]-4-amine;
• 3,3-difluoro-N,N-dimethyl-4-phenyl-2',3',4',9'-tetrahydrospiro[cyclohexane-1,1'-pyrido[3,4-b]indole]-4-amine;
• 2,2-difluoro-4-(3-fluorophenyl)-N,N-dimethyl-2',3',4',9'-tetrahydrospiro-[cyclohexane-1,1'-pyrido[3,4-b]indole]-4-amine;
• 3-fluoro-4-(3-fluorophenyl)-N,N-dimethyl-2',3',4',9'-tetrahydrospiro-[cyclohexane-1,1'-pyrido[3,4-b]indole]-4-amine;
• 2-fluoro-4-(3-fluorophenyl)-N,N-dimethyl-2',3',4',9'-tetrahydrospiro-[cyclohexane-1,1'-pyrido[3,4-b]indole]-4-amine;
• 2,6-difluoro-4-(3-fluorophenyl)-N,N-dimethyl-2',3',4',9'-tetrahydrospiro-[cyclohexane-1,1'-pyrido[3,4-b]indole]-4-amine;
• (E)-1-(4-(dimethylamino)-3-fluoro-4-(3-fluorophenyl)-3',4'-dihydrospiro-[cyclohexane-1,1'-pyrido[3,4-b]indole]-2'(9'H)-yl)-3-phenylprop-2-en-1-one;
• (E)-1-(4-(dimethylamino)-2-fluoro-4-(3-fluorophenyl)-3',4'-dihydrospiro-[cyclohexane-1,1'-pyrido[3,4-b]indole]-2'(9'H)-yl)-3-phenylprop-2-en-1-one;
• (E)-1-(4-(dimethylamino)-3,3-difluoro-4-(3-fluorophenyl)-3',4'-dihydrospiro-[cyclohexane-1,1'-pyrido[3,4-b]indole]-2'(9'H)-yl)-3-phenylprop-2-en-1-one;
• (E)-1-(4-(dimethylamino)-2,2-difluoro-4-(3-fluorophenyl)-3',4'-dihydrospiro[cyclohexane-1,1'-pyrido[3,4-b]indole]-2'(9'H)-yl)-3-phenylprop-2-en-1-one;
• (E)-1-(4-(dimethylamino)-2,6-difluoro-4-(3-fluorophenyl)-3',4'-dihydrospiro[cyclohexane-1,1'-pyrido[3,4-b]indole]-2'(9'H)-yl)-3-phenylprop-2-en-1-one;
• (E)-1-(4-(dimethylamino)-3,6'-difluoro-4-(3-fluorophenyl)-3',4'-dihydrospiro[cyclohexane-1,1'-pyrido[3,4-b]indole]-2'(9'H)-yl)-3-phenylprop-2-en-1-one;
• (E)-1-(4-(dimethylamino)-2,6'-difluoro-4-(3-fluorophenyl)-3',4'-dihydrospiro-[cyclohexane-1,1'-pyrido[3,4-b]indole]-2'(9'H)-yl)-3-phenylprop-2-en-1-one;
• (E)-1-(4-(dimethylamino)-3,3,6'-trifluoro-4-(3-fluorophenyl)-3',4'-dihydrospiro[cyclohexane-1,1'-pyrido[3,4-b]indole]-2'(9'H)-yl)-3-phenylprop-2-en-1-one;
• (E)-1-(4-(dimethylamino)-2,2,6'-trifluoro-4-(3-fluorophenyl)-3',4'-dihydrospiro[cyclohexane-1,1'-pyrido[3,4-b]indole]-2'(9'H)-yl)-3-phenylprop-2-en-1-one;
• (E)-1-(4-(dimethylamino)-2,6,6'-trifluoro-4-(3-fluorophenyl)-3',4'-dihydrospiro[cyclohexane-1,1'-pyrido[3,4-b]indole]-2'(9'H)-yl)-3-phenylprop-2-en-1-one;
• 2- methyl-4-(dimethylamino)1-4-phenyl-spiro[cyclohexane-1,8'-(5,6,7,8,9-pentahydro-pyrido[3,4-b]-7-aza-indole)]:
• N,N,2-trimethyl-4-phenyl-2',3',4',9'-tetrahydrospiro[cyclohexane-1,1'-pyrido[3,4-b]indole]-4-amine;
• 2-benzyl-N,N-dimethyl-4-phenyl-2',3',4',9'-tetrahydrospiro[cyclohexane-1,1'-pyrido[3,4-b]indole]-4-amine; and
• 2-benzyl-N,N-dimethyl-4-phenyl-2',3',4',9'-tetrahydrospiro[cyclohexane-1,1'-pyrido[3,4-b]indole]-4-amine;
• 6'-fluoro-2-(3-fluorobenzyl)-N,N-dimethyl-4-phenyl-4',9'-dihydrospiro-[cyclohexane-1,1'-pyrano-[3,4-b]indole]-4-amine;
• 2-(3-fluorobenzyl)-N,N-dimethyl-4-phenyl-2',3',4',9'-tetrahydrospiro-[cyclohexane-1,1'-pyrido[3,4-b]indole]-4-amine;
• N,N-dimethyl-4-phenyl-2-(phenylthio)-4',9'-dihydro-3'H-spiro-[cyclohexane-1,1'-pyrano[3,4-b]indole]-4-amine;
• N,N-dimethyl-4-phenyl-2-(phenylthio)-2',3',4',9'-tetrahydrospiro[cyclohexane-1,1'-pyrido[3,4-b]indole]-4-amine;
• 2-(3-fluorobenzyl)-N,N-dimethyl-4-phenyl-2',3',4',9'-tetrahydrospiro-[cyclohexane-1,1'-pyrido[3,4-b]indole]-4-amine;
• 2-(4-(dimethylamino)-4-phenyl-4',9'-dihydro-3'H-spiro[cyclohexane-1,1'-pyrano[3,4-b]indole]-2-yl)methyl acetate;
• 2-allyl-N,N-dimethyl-4-phenyl-4',9'-dihydros-3'H-spiro[cyclohexane-1,1'-pyrano[3,4-b]indole]-4-amine;
• 2-fluoro-N,N-dimethyl-4-phenyl-4',9'-dihydro-3'H-spiro[cyclohexane-1,1'-pyrano[3,4-b]indole]-4-amine;
• 2-allyl-N,N-dimethyl-4-phenyl-4',9'-dihydros-3'H-spiro[cyclohexane-1,1'-pyrano[3,4-b]indole]-4-amine;
• 2,6'-difluoro-N,N-dimethyl-4-phenyl-4',9'-dihydro-3'H-spiro[cyclohexane-1,1'-pyrano[3,4-b]indole]-4-amine;
• 4-(dimethylamino)-4-phenyl-4',9'-dihydro-3'H-spiro[cyclohexane-1,1'-pyrano[3,4-b]indole]-2-yl)methanol;
• 2,6'-difluoro-N,N-dimethyl-4-phenyl-4',9'-dihydro-3'H-spiro[cyclohexane-1,1'-pyrano[3,4-b]indole]-4-amine;
• 2-fluoro-N,N-dimethyl-4-phenyl-2',3',4',9'-tetrahydrospiro[cyclohexane-1,1'-pyrido[3,4-b]indole]-4-amine;
• 2-fluoro-N,N-dimethyl-4-phenyl-2',3',4',9'-tetrahydrospiro[cyclohexane-1,1'-pyrido[3,4-b]indole]-4-amine;
• 2-(4-(dimethylamino)-4-phenyl-4',9'-dihydro-3'H-spiro[cyclohexane-1,1'-pyrano[3,4-b]indole]-2-yl)ethanol ;
• 2-(4-(dimethylamino)-4-phenyl-4',9'-dihydro-3'H-spiro[cyclohexane-1,1'-pyrano[3,4-b]indole]-2-yl)methyl)isoindolin-1,3-dione;
• N-((4-(dimethylamino)-4-(3-fluorophenyl)-4',9'-dihydro-3'H-spiro-[cyclohexane-1,1'-pyrano[3,4-b]indole]-2-yl)methyl)-3-phenylcinnamic acid amide;
• tert-butyl 2-(2-(4-(dimethylamino)-4-phenyl-4',9'-dihydro-3'H-spiro[cyclohexane-1,1'-pyrano[3,4-b]indole]-2-yl)ethoxy)acetate;
• 2-(4-(dimethylamino)-4-phenyl-4',9'-dihydro-3'H-spiro[cyclohexane-1,1'-pyrano[3,4-b]indole]-2-yl)acetonitrile
or physiologically compatible salts thereof.

8. Medication containing at least one compound according to one of claims 1 to 7 in the form of a single stereoisomer or mixture thereof, the free compounds and/or their physiologically compatible salts, and also possibly suitable additives and/or adjuvants and/or possibly further active substances.

9. Use of a compound according to one of claims 1 to 7 in the form of a single stereoisomer or mixture thereof, the free compounds and/or their physiologically compatible salts for the production of a medication for the treatment of pain.

10. Use of a compound according to one of claims 1 to 7 in the form of a single stereoisomer or mixture thereof, the free compounds and/or their physiologically compatible salts for the production of a medication for the treatment of anxiety conditions, stress and stress-related syndromes, depressive illnesses, epilepsy, Alzheimer's disease, senile dementia, general cognitive dysfunctions, learning and memory disabilities (as nootropic), withdrawal symptoms, alcohol and/or drug and/or medication misuse and/or dependence, sexual dysfunctions, cardiovascular diseases, hypotension, hypertension, tinitus, pruritus, migraine, hearing impairment, deficient intestinal motility, eating disorders, anorexia, bulimia, mobility disorders, diarrhoea, cachexia, urinary incontinence, or as muscle relaxant, anticonvulsive or anaesthetic, or for coadministration in the treatment with an opioid analgesic or with an anaesthetic, for diuresis or anti-natriuresis, anxiolysis, for modulating movement activity, for modulating neurotransmitter release and for treating neuro-degenerative diseases associated therewith, for treating withdrawal symptoms and/or for reducing the addiction potential of opioids.

## Revendications

1. Composé de formule générale (1), 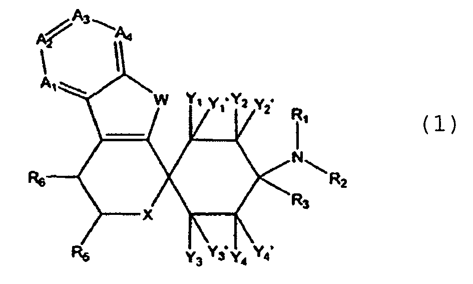 dans laquelle
A₁ représente -N= ou -CR₇=,
A₂ représente -N= ou -CR₈=,
A₃ représente -N= ou -CR₉=,
A₄ représente -N= ou -CR₁₀= ;
étant entendu qu'au maximum deux des radicaux A₁, A₂, A₃ et A₄ représentent -N= ;
W représente -NR₄-, -O- ou -S- ;
X représente -NR₁₇-, -O-, -S(=O)₀₋₂- ou -CR₁₈R₁₉-;
Y₁, Y_{1'}, Y₂, Y₂', Y₃, Y₃', Y₄ et Y₄' sont choisis chacun, indépendamment les uns des autres, dans l'ensemble constitué par -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)-R₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)2, -N⁺(R₀)₃, -N⁺(R₀)₂O⁻, -NHC(=O)R₀, -NHC(=O)OR₀, -NHC(=O)NH₂, -NHC(=O)NHR₀ et -NHC(=O)-N(R₀)₂; ou Y₁ et Y_{1'}, ou Y₂ et Y₂', ou Y₃ et Y₃', ou Y₄ et Y₄' représentent ensemble =O;
étant entendu qu'au moins l'un des radicaux Y₁, Y₁', Y₂, Y₂', Y₃, Y₃', Y₄ et Y₄' ne représente pas -H ;
R₀ représente chaque fois indépendamment un groupe -aliphatique en C₁-C₈, -cycloaliphatique en C₃-C₁₂, -aryle, -hétéroaryle, -aliphatique(C₁-C₈)-cycloaliphatique(C₃-C₁₂₎, -aliphatique(C₁-C₈)-aryle, -aliphatique(C₁-C₈)-hétéroaryle, -cycloaliphatique(C₃-C₈)-aliphatique(C₁-C₈), -cycloaliphatique(C₃-C₈)-aryle ou cycloaliphatique(C₃-C₈)-hétéroaryle ;
R₁ et R₂, indépendamment l'un de l'autre, représentent -H ou -R₀ ; ou R₁ et R₂ représentent ensemble -CH₂CH₂OCH₂CH₂-, -CH₂CH₂NR₁₁CH₂CH₂- ou - (CH₂)₃₋₆- ;
R₃ représente -R₀ ;
R₄ représente -H, -R₀, -COR₁₂ ou -S(=O)₂R₁₂ ;
R₅, R₆, R₇, R₈, R₉, R₁₀, R₁₈ et R₁₉ représentent chacun, indépendamment les uns des autres -H, -F, -Cl, -Br, -I, -NO₂, -CF₃, -OR₁₃, -SR₁₃, -SO₂R₁₃, -S(=O)₂OR₁₃, -CN, -COOR₁₃, -CONR₁₃, -NR₁₄R₁₅, =O ou -R₀ ; ou R₅ et R₆ ensemble représentent -(CH₂)₂₋₆-, des atomes d'hydrogène individuels pouvant également être remplacés par -F, -Cl, -Br, -I, -NO₂, -CF₃, -OR₁₃, -CN ou par un groupe aliphatique en C₁-C₆ ;
R₁₁ représente chaque fois indépendamment -H, -R₀ ou -C(=O)R₀ ;
R₁₂ représente chaque fois indépendamment -H, -R₀, -OR₁₃, ou -NR₁₄R₁₅ ;
R₁₃ représente chaque fois indépendamment -H ou -R₀ ;
R₁₄ et R₁₅ représentent, indépendamment l'un de l'autre, -H ou R₀ ; ou R₁₄ et R₁₅ représentent ensemble -CH₂CH₂OCH₂CH₂-, -CH₂CH₂NR₁₆CH₂CH₂- ou - (CH₂)₃₋₆- ;
R₁₆ représente -H ou un groupe -aliphatique en C₁-C₆ ; R₁₇ représente -H, -R₀, -COR₁₂ ou -S(=O)₂R₁₂ ;
un groupe « aliphatique » étant chaque fois un radical hydrocarboné aliphatique ramifié ou non ramifié, saturé ou une ou plusieurs fois insaturé, non substitué ou une ou plusieurs fois substitué ;
un groupe « cycloaliphatique » étant chaque fois un radical hydrocarboné alicyclique, mono- ou polycyclique, saturé ou une plusieurs fois insaturé, non substitué ou une ou plusieurs fois substitué ;
en entendant, eu égard à « aliphatique » et « cycloaliphatique », par « une ou plusieurs fois substitué » le remplacement simple ou multiple d'un ou plusieurs atomes d'hydrogène par -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)-N(R₀)₂, -Si(R₀)₃ OU -PO(OR₀)₂ ;
« aryle » représentant chaque fois indépendamment un système cyclique carbocyclique comportant au moins un cycle aromatique, mais sans hétéroatomes dans ce cycle, les radicaux aryle pouvant éventuellement être condensés avec d'autres systèmes cycliques saturés, (partiellement) insaturés ou aromatiques et chaque radical aryle pouvant être non substitué ou une ou plusieurs fois substitué, les substituants sur le groupe aryle pouvant être identiques ou différents et en toute position quelconque et possible du radical aryle ;
« hétéroaryle" représentant un radical cyclique aromatique à 5, 6 ou 7 chaînons, qui contient 1, 2, 3, 4 ou 5 hétéroatomes, les hétéroatomes étant des atomes d'azote, d'oxygène ou de soufre identiques ou différents, et l'hétérocycle pouvant être non substitué ou une ou plusieurs fois substitué ; dans le cas de la substitution sur l'hétérocycle les substituants pouvant être identiques ou différents, et en position quelconque et possible du radical hétéroaryle ; et l'hétérocycle pouvant également faire partie d'un système bi- ou polycyclique ;
en entendant, eu égard à « aryle » et « hétéroaryle », par « une ou plusieurs fois substitué » le remplacement simple ou multiple d'un ou plusieurs atomes d'hydrogène du système cyclique par des substituants choisis dans l'ensemble constitué par -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)-N(R₀)₂, -Si(R₀)₃ ou -PO(OR₀)₂, des atomes d'azote éventuellement présents dans le cycle pouvant chacun être oxydé ;
sous forme d'un stéréoisomère individuel ou d'un mélange de tels stéréoisomères, des composés libres et/ou de leurs sels physiologiquement acceptables.

2. Composé selon la revendication 1, qui présente la formule générale (1.1), (1.2), (1.3), (1.4), (1.5), (1.6) ou (1.7)

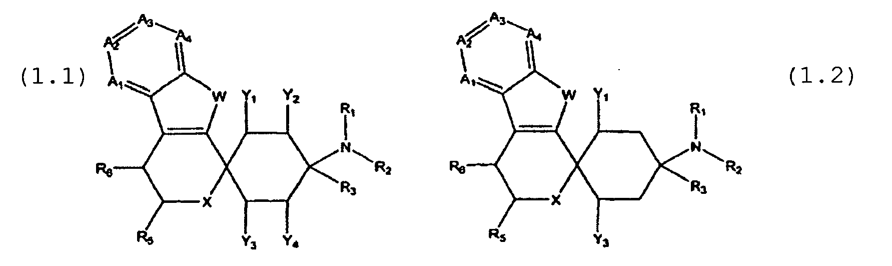

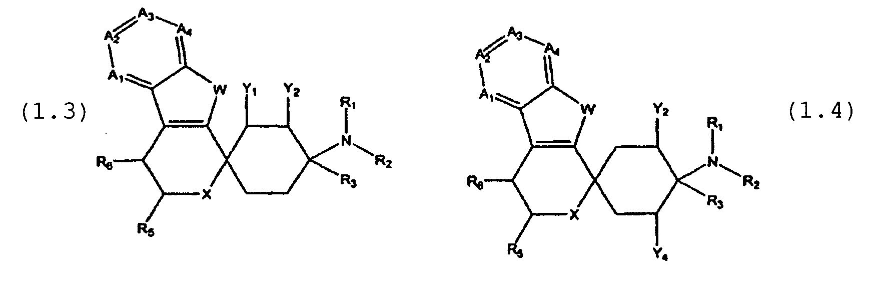

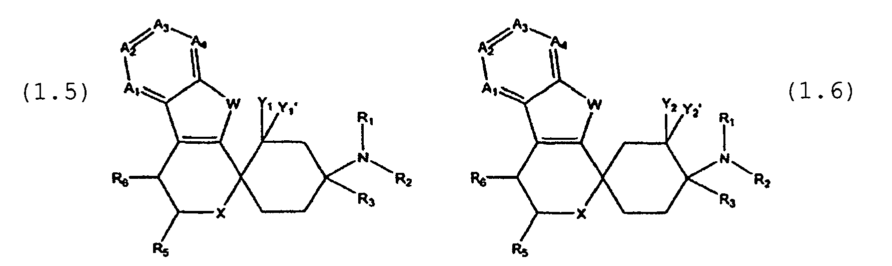

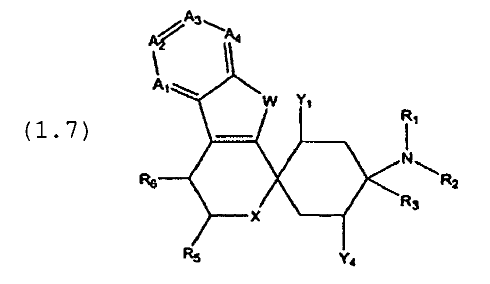

3. Composé selon la revendication 1 ou 2, qui présente la formule générale (2) 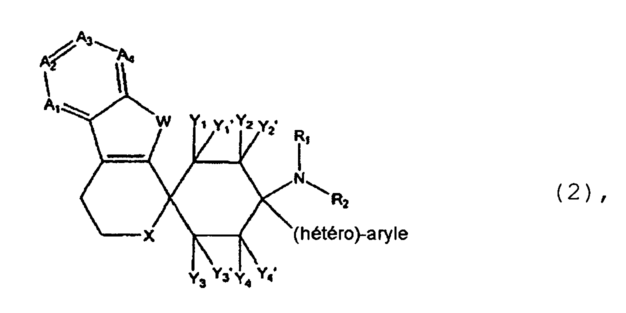 où (hétéro-)aryle représente -aryle ou -hétéroaryle.

4. Composé selon la revendication 3, qui présente la formule générale (2.1), (2.2), (2.3) ou (2.4) 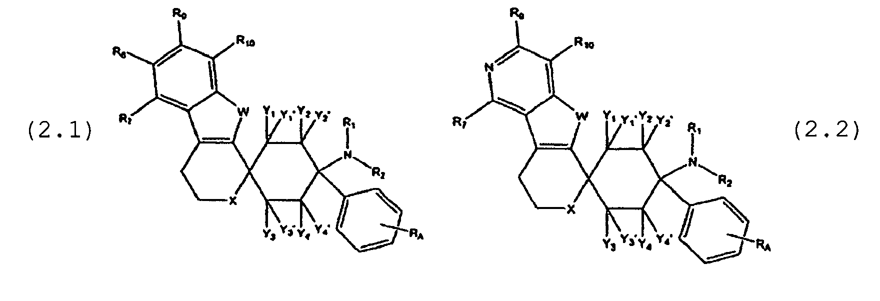 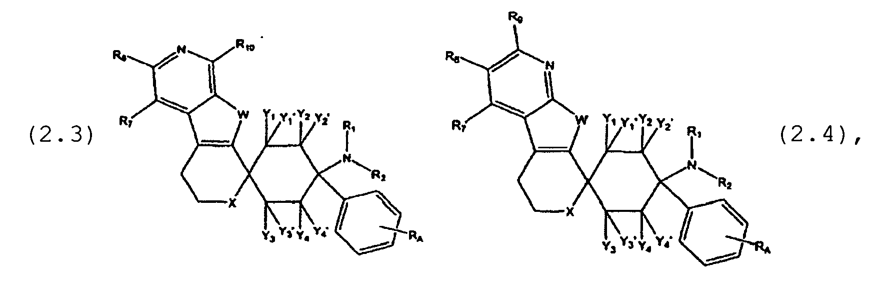 où R_{A} représente -H, -F, -Cl, -CN ou -CH₃.

5. Composé selon la revendication 4, qui présente la formule générale (2.1.1) ou (2.1.4)

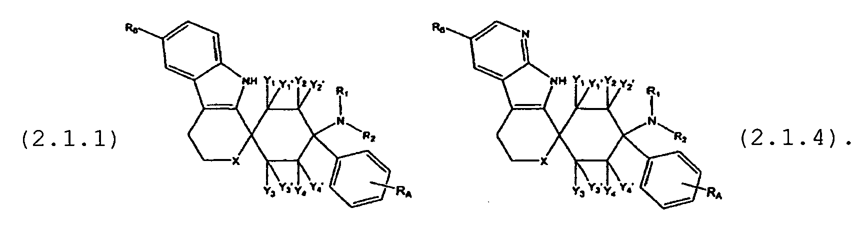

6. Composé selon la revendication 5, dans lequel X représente -0- ou -NR₁₇- ;
R₀ représente chaque fois indépendamment un groupe -aliphatique en C₁-C₈, -cycloaliphatique en C₃-C₁₂, -aryle, -hétéroaryle, -aliphatique(C₁-C₈)-cycloaliphatique(C₃-C₁₂), -aliphatique(C₁-C₈)-aryle, -aliphatique(C₁-C₈)-hétéroaryle, -cycloaliphatique (C₃-C₈) -aliphatique (C₁-C₈), -cycloaliphatique(C₃-C₈)-aryle ou cycloaliphatique(C₃-C₈)-hétéroaryle ;
R₁ représente -CH₃ ;
R₂ représente -H ou -CH₃ ;
R₈ représente -H ou -F ;
R₁₂ représente chaque fois indépendamment -H, -R₀, -OR₁₃, ou -NR₁₄R₁₅ ;
R₁₃ représente chaque fois indépendamment -H ou -R₀ ;
R₁₄ et R₁₅ représentent, indépendamment l'un de l'autre, -H ou R₀ ; ou R₁₄ et R₁₅ représentent ensemble -CH₂CH₂OCH₂CH₂-, -CH₂CH₂NR₁₆CH₂CH₂- ou -(CH₂)₃₋₆- ;
R₁₆ représente -H ou un groupe -aliphatique en C₁-C₆ ; R₁₇ représente -H, -R₀, -COR₁₂ ou -S(=O)₂R₁₂ ; et
Y₁, Y₁', Y₂, Y₂', Y₃, Y₃', Y₄ et Y₄' sont choisis chacun, indépendamment les uns des autres, dans l'ensemble constitué par -H, -F, -Cl, -CN, -aliphatique en C₁-C₈,
- aliphatique(C₁-C₈)-NH-aliphatique(C₁-C₈),
- aliphatique(C₁-C₈)-N[aliphatique (C₁-C₈)]₂,
- S-aliphatique(C₁-C₈), -S-aryle, -aryle et
- aliphatique (C₁-C₈)-aryle ; étant entendu qu'au moins l'un des radicaux Y₁, Y_{1'}, Y₂, Y_{2'}, Y₃, Y_{3'}, Y₄ et Y_{4'} est différent de -H ; et
R_{A} représente -H, -F, -Cl, -CN ou -CH₃.

7. Composé selon la revendication 1, choisi dans le groupe constitué par les composés suivants
• (±)-N,N,2-triméthyl-4-phényl-4',9'-dihydro-3'H-spiro[cyclohexane-1,1'-pyranno[3,4-b]indol]-4-amine ; 2-hydroxypropane-1,2,3-tricarboxylate ;
• (±)-N,N,2-triméthyl-4-phényl-4',9'-dihydro-3'H-spiro[cyclohexane-1,1'-pyranno[3,4-b]indol]-4-amine ; 2-hydroxypropane-l,2,3-tricarboxylate ;
• (±)-2-méthyl-4-(diméthylamino)I-4-phényl-spiro[cyclohexane-1,8'-(5,6,8,9-tétrahydro-pyranno[3,4-b]-7-aza-indole)] ; 2-hydroxypropane-1,2,3-tricarboxylate ;
• (±)-2-benzyl-N,N-diméthyl-4-phényl-4',9'-dihydro-3'H-spiro[cyclohexane-1,1'-pyranno[3,4-b]indol]-4-amine ;
• (±)-2-benzyl-N,N-diméthyl-4-phényl-2',3',4',9'-tétrahydrospiro[cyclohexane-1,1'-pyrido[3,4-b]-indol]-4-amine ; 2-hydroxypropane-1,2,3-tricarboxylate ;
• (±)-2-(3-fluorobenzyl)-N,N-diméthyl-4-phényl-4',9'-dihydro-3'H-spiro[cyclohexane-1,1'-pyranno[3,4-b]indol]-4-amine ;
• N,N,3,5-tétraméthyl-4-phényl-4',9'-dihydro-3'H-spiro[cyclohexane-1,1'-pyranno[3,4-b]indol]-4-amine ;
• N,N,2,6-tétraméthyl-4-phényl-4',9'-dihydro-3'H-spiro[cyclohexane-1,1'-pyranno[3,4-b]indol]-4-amine ;
• N,N,2,5-tétraméthyl-4-phényl-4',9'-dihydro-3'H-spiro[cyclohexane-1,1'-pyranno[3,4-b]indol]-4-amine ;
• 2-benzyl-N,N-diméthyl-4-phényl-4',9'-dihydro-3'H-spiro[cyclohexane-1,1'-pyranno[3,4-b]indol]-4-amine ;
• 2-(4-fluorobenzyl)-N,N-diméthyl-4-phényl-4',9'-dihydro-3'H-spiro[cyclohexane-1,1'-pyranno[3,4-b]-indol]-4-amine ;
• N,N,2,3-tétraméthyl-4-phényl-4',9'-dihydro-3'H-spiro[cyclohexane-1,1'-pyranno[3,4-b]indol]-4-amine ;
• N,N-diméthyl-3,4-diphényl-4',9'-dihydro-3'H-spiro[cyclohexane-1,1'-pyranno[3,4-b]indol]-4-amine ;
• 2-(4-fluorophényl)-N,N-diméthyl-4-phényl-4',9'-dihydro-3'H-spiro[cyclohexane-1,1'-pyranno[3,4-b]indol]-4-amine ;
• 3-((diméthylamino)méthyl)-N,N-diméthyl-4-phényl-4',9'-dihydro-3'H-spiro[cyclohexane-1,1'-pyranno[3,4-b]indol]-4-amine ;
• N,N,3-triméthyl-4-phényl-4',9'-dihydro-3'H-spiro[cyclohexane-1,1'-pyranno[3,4-b]indol]-4-amine ;
• 3-fluoro-N,N-diméthyl-4-phényl-4',9'-dihydro-3'H-spiro[cyclohexane-1,1'-pyranno[3,4-b]indol]-4-amine ;
• 2-fluoro-N,N-diméthyl-4-phényl-4',9'-dihydro-3'H-spiro[cyclohexane-1,1'-pyranno[3,4-b]indol]-4-amine ;
• N,N-diméthyl-2-(méthylthio)-4-phényl-4',9'-dihydro-3'H-spiro[cyclohexane-1,1'-pyranno[3,4-b]indol]-4-amine ;
• N,N-diméthyl-4-phényl-2-(phénylthio)-4',9'-dihydro-3'H-spiro[cyclohexane-1,1'-pyranno[3,4-b]indol]-4-amine ;
• 3,3-difluoro-N,N-diméthyl-4-phényl-4',9'-dihydro-3'H-spiro[cyclohexane-1,1'-pyranno-[3,4-b]indol]-4-amine ;
• 2,2-difluoro-N,N-diméthyl-4-phényl-4',9'-dihydro-3'H-spiro[cyclohexane-1,1'-pyranno-[3,4-b]indol]-4-amine ;
• 2-allyl-N,N-diméthyl-4-phényl-4',9'-dihydro-3'H-spiro[cyclohexane-1,1'-pyranno[3,4-b]indol]-4-amine ;
• N,N,3,5-tétraméthyl-4-phényl-2',3',4',9'-tétrahydrospiro[cyclohexane-1,1'-pyrido[3,4-b]-indol]-4-amine ;
• N,N,2,6-tétraméthyl-4-phényl-2',3',4',9'-tétrahydrospiro[cyclohexane-1,1'-pyrido[3,4-b]-indol]-4-amine ;
• N,N,2,5-tétraméthyl-4-phényl-2',3',4',9'-tétrahydrospiro[cyclohexane-1,1'-pyrido[3,4-b]-indol]-4-amine ;
• 2-benzyl-N,N-diméthyl-4-phényl-2',3',4',9'-tétrahydrospiro[cyclohexane-1,1'-pyrido-[3,4-b]-indol]-4-amine ;
• 2-(4-fluorobenzyl)-N,N-diméthyl-4-phényl-2',3',4',9'-tétrahydrospiro[cyclohexane-1,1'-pyrido[3,4-b]indol]-4-amine ;
• N,N,2,3-tétraméthyl-4-phényl-2',3',4',9'-tétrahydrospiro[cyclohexane-1,1'-pyrido[3,4-b]-indol]-4-amine ;
• N,N-diméthyl-3,4-diphényl-2',3',4',9'-tétrahydrospiro[cyclohexane-1,1'-pyrido[3,4-b]-indol]-4-amine ;
• 2-(4-fluorophényl)-N,N-diméthyl-4-phényl-2',3',4',9'-tétrahydrospiro[cyclohexane-1,1'-pyrido[3,4-b]indol]-4-amine ;
• 3-((diméthylamino)méthyl)-N,N-diméthyl-4-phényl-2',3',4',9'-tétrahydrospiro[cyclohexane-1,1'-pyrido[3,4-b]indol]-4-amine ;
• N,N,3-triméthyl-4-phényl-2',3',4',9'-tétrahydrospiro[cyclohexane-1,1'-pyrido[3,4-b]-indol]-4-amine ;
• 3-fluoro-N,N-diméthyl-4-phényl-2',3',4',9'-tétrahydrospiro[cyclohexane-1,1'-pyrido[3,4-b]-indol]-4-amine ;
• 2-fluoro-N,N-diméthyl-4-phényl-2',3',4',9'-tétrahydrospiro[cyclohexane-1,1'-pyrido[3,4-b]-indol]-4-amine ;
• N,N-diméthyl-2-(méthylthio)-4-phényl-2',3',4',9'-tétrahydrospiro[cyclohexane-1,1'-pyrido[3,4-b]-indol]-4-amine ;
• N,N-diméthyl-4-phényl-2-(phénylthio)-2',3',4',9'-tétrahydrospiro[cyclohexane-1,1'-pyrido[3,4-b]-indol]-4-amine ;
• 3,3-difluoro-N,N-diméthyl-4-phényl-2',3',4',9'-tétrahydrospiro[cyclohexane-1,1'-pyrido[3,4-b]-indol]-4-amine ;
• 2,2-difluoro-N,N-diméthyl-4-phényl-2',3',4',9'-tétrahydrospiro[cyclohexane-1,1'-pyrido[3,4-b]indol]-4-amine ;
• 2-allyl-N,N-diméthyl-4-phényl-2',3',4',9'-tétrahydrospiro[cyclohexane-1,1'-pyrido[3,4-b]-indol]-4-amine ;
• 2-benzyl-6'-fluoro-N,N-diméthyl-4-phényl-4',9'-dihydro-3'H-spiro[cyclohexane-1,1'-pyranno[3,4-b]indol]-4-amine ;
• 6'-fluoro-2-(3-fluorobenzyl)-N,N-diméthyl-4-phényl-4',9'-dihydro-3'H-spiro[cyclohexane-1,1'-pyranno[3,4-b]indol]-4-amine ;
• 2-(3-fluorobenzyl)-N,N-diméthyl-4-phényl-2',3',4',9'-tétrahydrospiro[cyclohexane-1,1'-pyrido[3,4-b]indol]-4-amine ;
• 3,6'-difluoro-N,N-diméthyl-4-phényl-4',9'-dihydro-3'H-spiro[cyclohexane-1,1'-pyranno-[3,4-b]indol]-4-amine ;
• 2,6'-difluoro-N,N-diméthyl-4-phényl-4',9'-dihydro-3'H-spiro[cyclohexane-1,1'-pyranno-[3,4-b]indol]-4-amine ;
• 3,3,6'-trifluoro-N,N-diméthyl-4-phényl-4',9'-dihydro-3'H-spiro[cyclohexane-1,1'-pyranno[3,4-b]-indol]-4-amine ;
• 2,2,6'-trifluoro-N,N-diméthyl-4-phényl-4',9'-dihydro-3'H-spiro[cyclohexane-1,1'-pyranno[3,4-b]-indol]-4-amine ;
• 2,6,6'-trifluoro-N,N-diméthyl-4-phényl-4',9'-dihydro-3'H-spiro[cyclohexane-1,1'-pyranno[3,4-b]-indol]-4-amine ;
• 2,2,6'-trifluoro-N-méthyl-4-phényl-4',9'-dihydro-3'H-spiro[cyclohexane-1,1'-pyranno-[3,4-b]indol]-4-amine ;
• 2,6'-difluoro-N-méthyl-4-phényl-4',9'-dihydro-3'H-spiro[cyclohexane-1,1'-pyranno[3,4-b]indol]-4-amine ;
• 2,6,6'-trifluoro-N-méthyl-4-phényl-4',9'-dihydro-3'H-spiro[cyclohexane-1,1'-pyranno-[3,4-b]indol]-4-amine ;
• 3,3,6'-trifluoro-N,N-diméthyl-4-(thiophén-2-yl)-4',9'-dihydro-3'H-spiro[cyclohexane-1,1'-pyranno[3,4-b]indol]-4-amine ;
• 4-butyl-3,3,6'-trifluoro-N,N-diméthyl-4',9'-dihydro-3'H-spiro[cyclohexane-1,1'-pyranno-[3,4-b]indol]-4-amine ;
• 2,2,6'-trifluoro-N,N-diméthyl-4-(thiophén-2-yl)-4',9'-dihydro-3'H-spiro[cyclohexane-1,1'-pyranno[3,4-b]indol]-4-amine ;
• 4-butyl-2,2,6'-trifluoro-N,N-diméthyl-4',9'-dihydro-3'H-spiro[cyclohexane-1,1'-pyranno-[3,4-b]indol]-4-amine ;
• 2,6,6'-trifluoro-N,N-diméthyl-4-(thiophén-2-yl)-4',9'-dihydro-3'H-spiro[cyclohexane-1,1'-pyranno[3,4-b]indol]-4-amine ;
• 4-butyl-2,6,6'-trifluoro-N,N-diméthyl-4',9'-dihydro-3'H-spiro[cyclohexane-1,1'-pyranno-[3,4-b]indol]-4-amine ;
• 3,6'-difluoro-N,N-diméthyl-4-(thiophén-2-yl)-4',9'-dihydro-3'H-spiro[cyclohexane-1,1'-pyranno[3,4-b]indol]-4-amine ;
• 4-butyl-3,6'-difluoro-N,N-diméthyl-4',9'-dihydro-3'H-spiro[cyclohexane-1,1'-pyranno-[3,4-b]indol]-4-amine ;
• 2,6'-difluoro-N,N-diméthyl-4-(thiophén-2-yl)-4',9'-dihydro-3'H-spiro[cyclohexane-1,1'-pyranno[3,4-b]indol]-4-amine ;
• 4-butyl-2,6'-difluoro-N,N-diméthyl-4',9'-dihydro-3'H-spiro[cyclohexane-1,1'-pyranno-[3,4-b]indol]-4-amine ;
• 3,3,6'-trifluoro-4-(3-fluorophényl)-N,N-diméthyl-2',3',4',9'-tétrahydrospiro[cyclohexane-1,1'-pyrido[3,4-b]indol]-4-amine;
• 2,2,6'-trifluoro-4-(3-fluorophényl)-N,N-diméthyl-2',3',4',9'-tétrahydrospiro[cyclohexane-1,1'-pyrido[3,4-b]indol]-4-amine ;
• 3,6'-difluoro-4-(3-fluorophényl)-N,N-diméthyl-2',3',4',9'-tétrahydrospiro[cyclohexane-1,1'-pyrido[3,4-b]indol]-4-amine ;
• 2,6'-difluoro-4(3-fluorophényl)-N,N-diméthyl-2',3',4',9'-tétrahydrospiro[cyclohexane-1,1'-pyrido[3,4-b]indol]-4-amine ;
• 2,6,6'-trifluoro-4-(3-fluorophényl)-N,N-diméthyl-2',3',4',9'-tétrahydrospiro[cyclohexane-1,1'-pyrido[3,4-b]indol]-4-amine ;
• 3,3-difluoro-N,N-diméthyl-4-phényl-2',3',4',9'-tétrahydrospiro[cyclohexane-1,1'-pyrido[3,4-b]-indol]-4-amine ;
• 2,2-difluoro-4-(3-fluorophényl)-N,N-diméthyl-2',3',4',9'-tétrahydrospiro[cyclohexane-1,1'-pyrido[3,4-b]indol]-4-amine ;
• 3-fluoro-4-(3-fluorophényl)-N,N-diméthyl-2',3',4',9'-tétrahydrospiro[cyclohexane-1,1'-pyrido[3,4-b]indol]-4-amine ;
• 2-fluoro-4-(3-fluorophényl)-N,N-diméthyl-2',3',4',9'-tétrahydrospiro[cyclohexane-1,1'-pyrido[3,4-b]indol]-4-amine ;
• 2,6-difluoro-4-(3-fluorophényl)-N,N-diméthyl-2',3',4',9'-tétrahydrospiro[cyclohexane-1,1'-pyrido[3,4-b]indol]-4-amine ;
• (E)-1-(4-(diméthylamino)-3-fluoro-4-(3-fluorophényl)-3',4'-dihydrospiro[cyclohexane-1,1'-pyrido[3,4-b]indol]-2'(9'H)-yl)-3-phénylprop-2-én-1-one;
• (E)-1-(4-(diméthylamino)-2-fluoro-4-(3-fluorophényl)-3',4'-dihydrospiro[cyclohexane-1,1'-pyrido[3,4-b]indol]-2'(9'H)-yl)-3-phénylprop-2-én-1-one ;
• (E)-1-(4-(diméthylamino)-3,3-difluoro-4-(3-fluorophényl)-3',4'-dihydrospiro[cyclohexane-1,1'-pyrido[3,4-b]indol]-2'(9'H)-yl)-3-phénylprop-2-én-1-one ;
• (E)-1-(4-(diméthylamino)-2,2-difluoro-4-(3-fluorophényl)-3',4'-dihydrospiro[cyclohexane-1,1'-pyrido[3,4-b]indol]-2'(9'H)-yl)-3-phénylprop-2-én-1-one;
• (E)-1-(4-(diméthylamino)-2,6-difluoro-4-(3-fluorophényl)-3',4'-dihydrospiro[cyclohexane-1,1'-pyrido[3,4-b]indol]-2'(9'H)-yl)-3-phénylprop-2-én-1-one ;
• (E)-1-(4-(diméthylamino)-3,6'-difluoro-4-(3-fluorophényl)-3',4'-dihydrospiro[cyclohexane-1,1'-pyrido[3,4-b]indol]-2'(9'H)-yl)-3-phénylprop-2-én-1-one ;
• (E)-1-(4-(diméthylamino)-2,6'-difluoro-4-(3-fluorophényl)-3',4'-dihydrospiro[cyclohexane-1,1'-pyrido[3,4-b]indol]-2'(9'H)-yl)-3-phénylprop-2-én-1-one ;
• (E)-1-(4-(diméthylamino)-3,3,6'-trifluoro-4-(3-fluorophényl)-3',4'-dihydrospiro[cyclohexane-1,1'-pyrido[3,4-b]indol]-2'(9'H)-yl)-3-phénylprop-2-én-1-one ;
• (E)-1-(4-(diméthylamino)-2,2,6'-trifluoro-4-(3-fluorophényl)-3',4'-dihydrospiro[cyclohexane-1,1'-pyrido[3,4-b]indol]-2'(9'H)-yl)-3-phénylprop-2-én-1-one ;
• (E)-1-(4-(diméthylamino)-2,6,6'-trifluoro-4-(3-fluorophényl)-3',4'-dihydrospiro[cyclohexane-1,1'-pyrido[3,4-b]indol]-2'(9'H)-yl)-3-phénylprop-2-én-1-one ;
• 2-méthyl-4- (diméthylamino)1-4-phényl-spiro[cyclohexane-1,8'-(5,6,7,8,9-pentahydro-pyrido[3,4-b]-7-aza-indole)] ;
• N,N,2-triméthyl-4-phényl-2',3',4',9'-tétrahydrospiro[cyclohexane-1,1'-pyrido[3,4-b]-indol]-4-amine ;
• 2-benzyl-N,N-diméthyl-4-phényl-2',3',4',9'-tétrahydrospiro[cyclohexane-1,1'-pyrido-[3,4-b]indol]-4-amine ; et
• 2-benzyl-N,N-diméthyl-4-phényl-2',3',4',9'-tétrahydrospiro[cyclohexane-1,1'-pyrido-[3,4-b]-indol]-4-amine ;
• 6'-fluoro-2-(3-fluorobenzyl)-N,N-diméthyl-4-phényl-4',9'-dihydrospiro[cyclohexane-1,1'-pyranno-[3,4-b]indol]-4-amine
• 2-(3-fluorobenzyl)-N,N-diméthyl-4-phényl-2',3',4',9'-tétrahydrospiro[cyclohexane-1,1'-pyrido[3,4-b]indol]-4-amine
• N,N-diméthyl-4-phényl-2-(phénylthio)-4',9'-dihydro-3'H-spiro-[cyclohexane-1,1'-pyranno[3,4-b]indol]-4-amine
• N,N-diméthyl-4-phényl-2-(phénylthio)-2',3',4',9'-tétrahydrospiro[cyclohexane-1,1'-pyrido[3,4-b]-indol]-4-amine
• 2-(3-fluorobenzyl)-N,N-diméthyl-4-phényl-2',3',4',9'-tétrahydrospiro[cyclohexane-1,1'-pyrido[3,4-b]indol]-4-amine
• 2-(4-(diméthylamino)-4-phényl-4',9'-dihydro-3'H-spiro[cyclohexane-1,1'-pyranno[3,4-b]indol]-2-yl)acétate de méthyle
• 2-allyl-N,N-diméthyl-4-phényl-4',9'-dihydro-3'H-spiro[cyclohexane-1,1'-pyranno[3,4-b]indol]-4-amine
• 2-fluoro-N,N-diméthyl-4-phényl-4',9'-dihydro-3'H-spiro[cyclohexane-1,1'-pyranno[3,4-b]indol]-4-amine
• 2-allyl-N,N-diméthyl-4-phényl-4',9'-dihydro-3'H-spiro[cyclohexane-1,1'-pyranno[3,4-b]indol]-4-amine
• 2,6'-difluor-N,N-diméthyl-4-phényl-4',9'-dihydro-3'H-spiro[cyclohexane-1,1'-pyranno[3,4-b]indol]-4-amine
• 4-(diméthylamino)-4-phényl-4',9'-dihydro-3'H-spiro[cyclohexane-1,1'-pyranno[3,4-b]indol]-2-yl)méthanol
• 2,6'-difluoro-N,N-diméthyl-4-phényl-4',9'-dihydro-3'H-spiro[cyclohexane-1,1'-pyranno[3,4-b]indol]-4-amine
• 2-fluoro-N,N-diméthyl-4-phényl-2',3',4',9'-tétrahydrospiro[cyclohexane-1,1'-pyrido[3,4-b]-indol]-4-amine
• 2-fluoro-N,N-diméthyl-4-phényl-2',3',4',9'-tétrahydrospiro[cyclohexane-1,1'-pyrido[3,4-b]-indol]-4-amine
• 2-(4-(diméthylamino)-4-phényl-4',9'-dihydro-3'H-spiro[cyclohexane-1,1'-pyranno[3,4-b]indol]-2-yl)éthanol
• 2-(4-(diméthylamino)-4-phényl-4',9'-dihydro-3'H-spiro[cyclohexane-1,1'-pyranno[3,4-b]indol]-2-yl)méthyl)isoindoline-1,3-dione
• N-((4-(diméthylamino)-4-(3-fluorophényl)-4',9'-dihydro-3'H-spiro[cyclohexane-1,1'-pyranno[3,4-b]-indol]-2-yl)méthyl)-3-phénylcinnamide
• 2-(2-(4-(diméthylamino)-4-phényl-4',9'-dihydro-3'H-spiro[cyclohexane-1,1'-pyranno[3,4-b]indol]-2-yl)éthoxy)acétate de tert-butyle
• 2-(4-(diméthylamino)-4-phényl-4',9'-dihydro-3'H-spiro[cyclohexane-1,1'-pyranno[3,4-b]indol]-2-yl)-acétonitrile
ou leurs sels physiologiquement acceptables.

8. Médicament contenant au moins un composé selon l'une quelconque des revendications 1 à 7, sous forme d'un stéréoisomère individuel ou d'un mélange de tels stéréoisomères, des composés libres et/ou de leurs sels physiologiquement acceptables, ainsi qu'éventuellement des additifs et/ou adjuvants appropriés et/ou éventuellement d'autres substances actives.

9. Utilisation d'un composé selon l'une quelconque des revendications 1 à 7, sous forme d'un stéréoisomère individuel ou d'un mélange de tels stéréoisomères, des composés libres et/ou de leurs sels physiologiquement acceptables, pour la fabrication d'un médicament destiné au traitement de la douleur.

10. Utilisation d'un composé selon l'une quelconque des revendications 1 à 7, sous forme d'un stéréoisomère individuel ou d'un mélange de tels stéréoisomères, des composés libres et/ou de leurs sels physiologiquement acceptables, pour la fabrication d'un médicament destiné au traitement d'états anxieux, du stress et de syndromes liés au stress, de dépressions, de l'épilepsie, de la maladie d'Alzheimer, de la démence sénile, de dysfonctionnements cognitifs généraux, de troubles de la mémoire et de l'apprentissage (en tant qu'agent nootrope), de manifestations inflammatoires, d'abus d'alcool et/ou de drogues et/ou de médicaments et/ou de la dépendance à ceux-ci, de dysfonctionnements sexuels, de maladies cardiovasculaires, de l'hypotension, de l'hypertension, de l'acouphène, du prurit, de la migraine, de troubles de l'audition, de la motilité intestinale insuffisante, de l'alimentation perturbée, de l'anorexie, de l'adiposité, de troubles locomoteurs, de la diarrhée, de la cachexie, de l'incontinence urinaire ou en tant que myorelaxant, anticonvulsivant ou anesthésique, ou à l'administration simultanée dans le traitement par un analgésique opiacé ou par un anesthésique, à la diurèse ou à l'antinatriurèse, à l'anxiolyse, à la modulation de l'activité motrice, à la modulation de l'excrétion de neurotransmetteurs et au traitement de maladies neurodégénératives qui y sont liées, au traitement de manifestations inflammatoires et/ou à la réduction du potentiel de dépendance d'opiacés.
